# EUROPEAN PATENT SPECIFICATION

(11) **EP 2 214 679 B1**
(45) Date of publication and mention of the grant of the patent: **27.03.2019**
(21) Application number: 08850126.7
(22) Date of filing: 12.11.2008
(51) Int. Cl.: A61K 9/00, A61K 9/06, A61K 47/36, A61K 47/38, A61K 45/06, A61K 31/41, A61K 31/573

(54) **CORTICOSTEROID COMPOSITIONS**
KORTIKOSTEROIDZUSAMMENSETZUNGEN
COMPOSITIONS CORTICOSTÉROÏDIENNES

(30) Priority: 13.11.2007 US 987720 P; 06.12.2007 US 12012 P; 21.12.2007 US 15998 P; 08.01.2008 US 19818 P; 07.03.2008 US 34941 P; 10.03.2008 US 35348 P; 16.05.2008 US 54103 P; 16.05.2008 US 54104 P; 16.05.2008 US 54105 P; 16.05.2008 US 54106 P; 16.05.2008 US 54107 P; 20.08.2008 US 90568 P
(43) Date of publication of application: 11.08.2010
(73) Proprietor: Meritage Pharma, Inc., Lexington, MA 02421 (US)
(72) Inventor: LICALSI, Cynthia, San Diego, California 92130 (US); PHILLIPS, Elaine, San Diego, California 92130 (US); HILL, Malcolm, San Diego, California 92130 (US); DESHMUKH, Hemant, San Diego, California 92130 (US); JOHNSON, Keith, San Diego, California 92130 (US)
(74) Representative: Goodfellow, Hugh Robin
(86) International application number: PCT/US2008/012780
(87) International publication number: WO 2009/064457

(56) References cited:
- US-A1- 2004 023 935
- US-A1- 2006 193 783
- US-A1- 2007 111 978
- US-A1- 2007 111 978
- US-B1- 6 598 603
- US-B2- 6 899 099
- S Freers: "Maltodextrin" In: "Handbook of Pharmaceutical Excipients - Fifth Edition", 1 January 2006 (2006-01-01), Pharmaceutical Press, UK / USA, XP055075444, ISBN: 978-0-85-369618-6 pages 442-444, * Monography part 7 *

## Description

### BACKGROUND OF THE INVENTION

Esophageal inflammation disorders are gaining increased recognition in both adults and children. One example is eosinophilic esophagitis (EE or EoE), which is an emerging, and fast-growing disorder characterized by high levels of eosinophils in the esophagus, as well as basal zone hyperplasia. EE (or EoE) is thought to be provoked, in at least a subset of patients, by food allergies or airborne allergen exposure (1- 5, 44). EE (or EoE) diagnosis is often associated with other hypersensitivity disorders, including asthma, rhinitis, and other food and aeroallergen inhalant sensitivities (39-40). Diagnosis is often made, e.g., in young children and depends on the finding of 15 to 20 or more to 24 or more eosinophils per high power field (eos/hpf) within esophageal mucosal biopsies (6-12).

In parallel with other atopic disorders, the incidence of EE (or EoE) appears to be increasing (15, 35). The disorder may present with reflux-like symptoms, pain and dysphagia, clinical symptoms similar to the presentation of gastroesophageal reflux disease ("GERD") (42). Symptoms of EE (or EoE) include, for example, abdominal pain, chest pain, choking, difficulty swallowing, failure to thrive, nausea, reflux not relieved by standard anti-flux therapy, skin rash or hives, vomiting, and weight loss. In one series, 15% of EE (or EoE) patients had concurrent developmental delay (45).

Although EE (or EoE) is becoming more frequently diagnosed throughout developing countries (7, 8, 13-16) many aspects of the disease remain unclear including its etiology, natural history and optimal therapy. Symptoms of EE (or EoE) often mimic those of GERD and include vomiting, dysphagia, pain and food impaction (8, 14, 17-20). However, treatment of EE (or EoE) and GERD differ and it is important to distinguish between them, particularly as untreated EE (or EoE) may be associated with esophageal narrowing in 10-30% of cases (14, 18, 20, 21). The overlap of GERD and EE (or EoE) symptoms is common; failure to respond to high PPI GERD treatment may be one diagnostic guideline for EE (or EoE) (42). The common occurrence regarding misdiagnosis of EE (or EoE) for GERD often results in delayed treatment for patients with EE (or EoE). (42).

Long term systemic steroid therapy can result in significant secondary side effects on growth and bone development. Although treatment with anti-IL-5 monoclonal antibody has been reported to be successful in EE (or EoE), this therapy is currently not approved for use in children (36).

Current treatments include elimination diets (22, 23), and elemental formulas (2, 24). Identifying true inciting food allergens can be difficult and elemental formulas are often unpalatable, thereby making dietary interventions complicated (1, 22). Improvised puff and swallow techniques may be difficult for patients, especially smaller children, and especially children with developmental delays, to perform efficiently. This may result in a less than effective dose of a topical steroid being delivered to the esophagus.
US2007/111978 discloses a method of preventing or alleviating esophageal inflammation by administering an oral composition of budesonide together with excipients.

### SUMMARY OF THE INVENTION

The invention relates to a physically and chemically stable oral pharmaceutical composition for use in treating, preventing or alleviating esophageal inflammation comprising:
a. a therapeutically effective amount of corticosteroid,
b. an antioxidant,
c. a buffer,
d. a surfactant,
e. a preservative,
f. a flavoring agent, a sweetener, or a combination thereof,
g. dextrose, and
h. a liquid vehicle,
where the oral pharmaceutical composition is suitable for single or multiple dose administration.

The matter for which protection is sought is defined by the claims. Embodiments and features which are not explicitly mentioned in the claims are illustrative and optional.

The pharmaceutical composition provided herein is suitable for single or multiple dose administration. In certain embodiments, a pharmaceutical composition described herein is in a multiple-unit container and comprising a plurality of unit doses. In some embodiments, a composition described herein remains substantially uniform after storage. In certain embodiments, a pharmaceutical composition described herein remains substantially uniform after storage for the shelf life of the formulation. In some embodiments, a pharmaceutical composition described herein has a fluid, liquid, solution, suspension, solid, semi-solid, gel, cream, ointment, spreadable, flowable, or paste-like consistency. In certain embodiments, a pharmaceutical composition described herein obtains or regains substantial uniformity upon mild or moderate agitation, swirling, gentle swirling or shaking.
In certain embodiments, after
mild or moderate agitation, swirling, gentle swirling or shaking, the pharmaceutical composition described herein remains substantially uniform for a convenient period of time, including, by way of non-limiting example, for at least 1, 2, 4, 6, 12, 18, or 24 hours, or 2, 3, 4, 5 or more days.

In certain embodiments, a pharmaceutical composition described herein is a multiple dose formulation. In certain embodiments, each dose (e.g., from a multiple unit container containing a plurality of doses of the pharmaceutical composition) of the formulation is substantially uniform with regard to one
another. In some embodiments, the first and final dose (e.g., from a multiple unit container containing a plurality of doses of the pharmaceutical composition) are substantially uniform.

In some embodiments, a pharmaceutical composition described herein comprises a corticosteroid that is readily dispersed throughout the composition upon agitation (e.g., mild or moderate). In certain embodiments, a pharmaceutical composition described herein comprises a corticosteroid that is readily dispersed throughout the composition upon mild or moderate agitation after storage. In some embodiments, a pharmaceutical composition described herein comprises a corticosteroid that is easily resuspended in the composition upon mild or moderate agitation. In certain embodiments, a pharmaceutical composition described herein comprises a corticosteroid that is easily resuspended in the composition upon mild or moderate agitation after storage. In some embodiments, a pharmaceutical composition described herein comprises a corticosteroid that is readily dispersed throughout the composition upon mild or moderate agitation. In certain embodiments, a pharmaceutical composition described herein comprises a corticosteroid that is readily dispersed throughout the composition upon mild or moderate agitation after storage. In certain embodiments, the ability of the compositions described herein are able to be easily resuspended and/or readily redispersed after storage under ambient conditions. In other embodiments, the storage is under an inert atmosphere, increased temperature and/or increased relative humidity. In certain embodiments, the ability of the compositions described herein are able to be easily resuspended and/or readily redispersed after storage for, by way of non-limiting example, 1 day, 2 days, 3 days, 1 week, 2 weeks, 3 weeks, 1 month, 3 months, 6 months, 1 year, 2 years, or the time of the shelf life.

In certain embodiments, a pharmaceutical composition described herein does not cake or aggregate during or after storage (e.g., the corticosteroid does not sediment and form a solid cake or fuse).

In some embodiments, a pharmaceutical composition described herein is a dispersion, suspension or solution. In certain embodiments, a pharmaceutical composition described herein is a solution, except that substantially all of the corticosteroid is dispersed or suspended as particles in the solution (e.g., less than about 36 µg of budesonide is in solution). In some embodiments, the at least one excipient does not enhance the solubility of the corticosteroid in the liquid vehicle.

In certain embodiments, a pharmaceutical composition described herein is a non-Newtonian fluid. In some embodiments, the non-Newtonian fluid is selected from, by way of non-limiting example, a plastic, a pseudo-plastic and a dilatant. In certain embodiments, the non-Newtonian fluid is pseudo-plastic. In some embodiments, the non-Newtonian fluid is thixotropic.

In certain embodiments, the corticosteroid is topically active (e.g., topically active on a gastrointestinal surface, such as the esophageal surface). In some embodiments, the corticosteroid is budesonide. In other embodiments, the corticosteroid is fluticasone propionate. In some embodiments, the corticosteroid is a particle (e.g., a microparticle or a nanoparticle).

In certain embodiments, the composition may contain an additional excipient selected from, by way of non-limiting example, cellulose (including derivatives thereof), one or more maltodextrin, dextrose, hydroxyethylcellulose (HEC), hydroxypropylmethyl-cellulose (HPMC), carboxymethyl-cellulose (CMC), microcrystalline cellulose (MCC), carbomer and combinations thereof. In more specific embodiments, the at least one additional excipient comprises hydroxyethylcellulose (HEC), hydroxypropylmethyl-cellulose (HPMC), carboxymethyl-cellulose (CMC), microcrystalline cellulose (MCC), carbomer and combinations thereof, and the at least one additional excipient is present in the pharmaceutical composition in an amount of about 5 mg/mL to about 100 mg/mL. In some specific embodiments, the at least one additional excipient comprises, by way of non-limiting example, maltodextrin, dextrose and combinations thereof, and the at least one additional excipient is present in an amount of about 1 mg/mL to about 1.5 g/mL.

In certain embodiments, the liquid vehicle comprises an aqueous medium.

In some embodiments, a pharmaceutical composition described herein comprises corticosteroid particles suspended in the aqueous medium. In certain embodiments, the corticosteroid particles are microparticles having a mean diameter of, by way of non-limiting example, 0.1 microns to 50 microns. In some embodiments, at least 95%, at least 97%, at least 98%, or at least 99% of the corticosteroid particles are microparticles having a diameter of less than 10 microns.

In certain embodiments, provided herein is a pharmaceutical composition wherein corticosteroid is present in an amount of 0.01 mg/mL to 1 mg/mL. In certain embodiments, the pharmaceutical composition has a total volume of 1 mL to 20 mL, about 1 mL to about 10 mL, about 1 mL to about 15 mL, or about 3 mL to about 7 mL, about 5 mL, about 10 mL, about 15 mL, or about 20 mL.

In some embodiments, one additional excipient comprises CMC, and the CMC is present in the pharmaceutical composition in an amount of 5 mg/mL to 30 mg/mL. In certain embodiments, the additional excipient comprises carbomer, and the carbomer is present in the pharmaceutical composition in an amount of about 5 mg/mL to about 100 mg/mL. In some embodiments, the additional excipient comprises HPMC, and the HPMC is present in the pharmaceutical
composition in an amount of about 5 mg/mL to about 30 mg/mL. In certain embodiments, one additional excipient comprises MCC, and the MCC is present in the pharmaceutical composition in an amount of 5 mg/mL to 30 mg/mL. In some embodiments, one additional excipient comprises a combination of CMC and MCC, and the CMC-MCC combination is present in an amount of 5 mg/mL to 40 mg/mL, and wherein the CMC/MCC mixed weight ratio is 11/89. The composition of the invention comprises dextrose. The dextrose may be present in the
pharmaceutical in an amount of about 10 mg/mL to about 1 g/mL. In some embodiments,
one additional agent comprises maltodextrin and the maltodextrin is present in the pharmaceutical composition in an amount of 10 mg/mL to 1 g/mL.

In certain embodiments, edetate is present in the pharmaceutical composition an amount of 0.05 mg/mL to 25 mg/mL. In some embodiments, citrate is present in the pharmaceutical composition in an amount of about 0.1 mg/mL to about 30 mg/mL. In certain embodiments, polysorbate 80 is present in the pharmaceutical composition in an amount of 0.05 mg/mL to about 1 mg/mL.

In further or alternative embodiments, provided herein is a pharmaceutical composition comprising:
a. budesonide in an amount of 0.02 mg/mL to 0.75 mg/mL,
b. edetate in an amount of 0.05 mg/mL to 25 mg/mL,
c. citrate in an amount of 0.1 mg/mL to 30 mg/mL,
d. polysorbate 80 in an amount of 0.05 mg/mL to 1 mg/mL,
e. a preservative,
f. a flavoring agent, a sweetener, or a combination thereof,
g. at least one additional excipient, and
h. an aqueous liquid vehicle.

Also provided herein is a pharmaceutical composition comprising:
a. budesonide in an amount of about 0.05 mg/mL to about 0.75 mg/mL,
b. edetate in an amount of about 0.05 mg/mL to about 25 mg/mL,
c. citrate in an amount of about 0.1 mg/mL to about 30 mg/mL,
d. polysorbate 80 in an amount of 0.05 mg/mL to about 1 mg/mL,
e. a preservative,
f. a flavoring agent, a sweetener, or a combination thereof,
g. at least one additional excipient, and
h. an aqueous liquid vehicle.

Also provided herein is a pharmaceutical composition comprising:
a. budesonide in an amount of about 0.1 mg/mL to about 0.75 mg/mL,
b. edetate in an amount of about 0.05 mg/mL to about 25 mg/mL,
c. citrate in an amount of about 0.1 mg/mL to about 30 mg/mL,
d. polysorbate 80 in an amount of 0.05 mg/mL to about 1 mg/mL,
e. a preservative,
f. a flavoring agent, a sweetener, or a combination thereof,
g. at least one additional excipient, and
h. an aqueous liquid vehicle.

In specific embodiments, a pharmaceutical composition provided herein has a total volume of 1 mL to 20 mL.

Also disclosed herein is a pharmaceutical composition comprising a physically and chemically stable composition comprising:
a. a therapeutically effective amount of corticosteroid,
b. a preservative,
c. a buffer,
d. a surface active agent or a surfactant,
e. an optional preservative,
f. an optional flavoring agent,
g. an optional sweetener,
h. at least one additional excipient, and
i. a liquid vehicle.

The pharmaceutical composition of the invention is suitable for single or multiple dose administration. In certain embodiments, a pharmaceutical composition described herein is in a multiple-unit container and comprising a plurality of unit doses. In some embodiments, a composition described herein remains substantially uniform after storage. In certain embodiments, a pharmaceutical composition described herein remains substantially uniform after storage for the shelf life of the formulation. In some embodiments, a pharmaceutical composition described herein has a solid, semi-solid, gel, cream, ointment, spreadable, flowable, or paste-like consistency. In certain embodiments, a pharmaceutical composition described herein obtains or regains substantial uniformity upon mild or moderate agitation, swirling, gentle swirling or shaking. In some embodiments, a pharmaceutical composition described herein obtains or regains substantial uniformity upon mild or moderate agitation, swirling, gentle swirling or shaking. In certain embodiments, after mild or moderate agitation, swirling, gentle swirling or shaking, the pharmaceutical composition described herein remains substantially uniform for a convenient period of time, including, by way of non-limiting example, for at least 1, 2, 4, 6, 12, 18, or 24 hours, or 2, 3, 4, 5 or more days.

In certain embodiments, a pharmaceutical composition described herein is a multiple dose formulation. In certain embodiments, each dose (e.g., from a multiple unit container containing a plurality of doses of the pharmaceutical composition) of the formulation is substantially uniform with regard to one another. In some embodiments, the first and final dose (e.g., from a multiple unit container containing a plurality of doses of the pharmaceutical composition) are substantially uniform.

In some embodiments, a pharmaceutical composition described herein comprises a corticosteroid that is readily dispersed throughout the composition upon agitation (e.g., mild or moderate). In certain embodiments, a pharmaceutical composition described herein comprises a corticosteroid that is readily dispersed throughout the composition upon mild or moderate agitation after storage. In some embodiments, a pharmaceutical composition described herein comprises a corticosteroid that is easily resuspended in the composition upon mild or moderate agitation. In certain embodiments, a pharmaceutical composition described herein comprises a corticosteroid that is easily resuspended in the composition upon mild or moderate agitation after storage. In some embodiments, a pharmaceutical composition described herein comprises a corticosteroid that is readily dispersed throughout the composition upon mild or moderate agitation. In certain embodiments, a pharmaceutical composition described herein comprises a corticosteroid that is readily dispersed throughout the composition upon mild or moderate agitation after storage. In certain embodiments, the ability of the compositions described herein are able to be easily resuspended and/or readily redispersed after storage under ambient conditions. In other embodiments, the storage is under an inert atmosphere, increased temperature and/or increased relative humidity. In certain embodiments, the ability of the compositions described herein are able to be easily resuspended and/or readily redispersed after storage for, by way of non-limiting example, 1 day, 2 days, 3 days, 1 week, 2 weeks, 3 weeks, 1 month, 3 months, 6 months, 1 year, 2 years, or the time of the shelf life.

In certain embodiments, a pharmaceutical composition described herein does not cake or aggregate during or after storage (e.g., the corticosteroid does not sediment and form a solid cake or fuse).

In some embodiments, a pharmaceutical composition described herein is a dispersion, suspension or solution. In certain embodiments, a pharmaceutical composition described herein is a solution, except that substantially all of the corticosteroid is dispersed or suspended as particles in the solution (e.g., less than about 36 µg of budesonide is in solution). In some embodiments, the at least one excipient does not enhance the solubility of the corticosteroid in the liquid vehicle.

In certain embodiments, a pharmaceutical composition described herein is a non-Newtonian fluid. In some embodiments, the non-Newtonian fluid is selected from, by way of non-limiting example, a plastic, a pseudo-plastic and a dilatant. In certain embodiments, the non-Newtonian fluid is pseudo-plastic. In some embodiments, the non-Newtonian fluid is thixotropic.

In certain embodiments, the corticosteroid is topically active (e.g., topically active on the esophageal surface). In some embodiments, the corticosteroid is budesonide. In other embodiments, the corticosteroid is fluticasone propionate. In some embodiments, the corticosteroid is a particle (e.g., a microparticle or a nanoparticle).

In certain embodiments, the additional excipient is selected from, by way of non-limiting example, maltodextrin, dextrose, hydroxyethylcellulose (HEC), hydroxypropylmethyl-cellulose (HPMC), carboxymethyl-cellulose (CMC), microcrystalline cellulose (MCC), carbomer and combinations thereof. In more specific embodiments, the at least one additional excipient comprises hydroxyethylcellulose (HEC), hydroxypropylmethyl-cellulose (HPMC), carboxymethyl-cellulose (CMC), microcrystalline cellulose (MCC), carbomer and combinations thereof, and the at least one additional excipient is present in the pharmaceutical composition in an amount of about 5 mg/mL to about 100 mg/mL. In some specific embodiments, the at least one additional excipient comprises, by way of non-limiting example, maltodextrin, dextrose and combinations thereof, and the at least one additional excipient is present in an amount of about 1 mg/mL to about 1.5 g/mL.

In certain embodiments, the liquid vehicle comprises an aqueous medium.

In some embodiments, a pharmaceutical composition described herein comprises corticosteroid particles suspended in the aqueous medium. In certain embodiments, the corticosteroid particles are microparticles having a mean diameter of, by way of non-limiting example, 0.1 microns to 50 microns. In some embodiments, at least 95%, at least 97%, at least 98%, or at least 99% of the corticosteroid particles are microparticles having a diameter of less than 10 microns.

In certain embodiments, provided herein is a pharmaceutical composition wherein corticosteroid is present in an amount of 0.01 mg/mL to 1 mg/mL. In certain embodiments, the pharmaceutical composition has a total volume of 1 mL to 20 mL, about 1 mL to about 10 mL, or about 3 mL to about 7 mL, about 5 mL, or about 10 mL.

In some embodiments, one additional excipient comprises CMC, and the CMC is present in the pharmaceutical composition in an amount of 5 g/mL to 30 mg/mL. In certain embodiments, the additional excipient comprises carbomer, and the carbomer is present in the pharmaceutical composition in an amount of about 5 mg/mL to about 100 mg/mL. In some embodiments, the excipient comprises HPMC, and the HPMC is present in the pharmaceutical composition in an amount of about 5 mg/mL to about 30 mg/mL. In certain embodiments, one additional excipient comprises MCC, and the MCC is present in the pharmaceutical composition in an amount of 5 mg/mL to 30 mg/mL. In some embodiments, one additional excipient
comprises a combination of CMC and MCC, and the CMC-MCC combination is present in an amount of 5 mg/mL to 40 mg/mL, and wherein the CMC/MCC mixed weight ratio is 11/89. The composition of the invention comprises dextrose which may be
present in the
pharmaceutical composition in an amount of about 10 mg/mL to about 1 g/mL. In some embodiments, one additional agent comprises maltodextrin and the maltodextrin is present in the pharmaceutical composition in an amount of 10 mg/mL to 1 g/mL.

In certain embodiments, any pharmaceutical composition described herein has a viscosity such that when a single dose of the pharmaceutical composition is orally administered to an individual, the pharmaceutical composition at least partially coats the esophagus and topically delivers a therapeutically effective amount of corticosteroid to the esophagus. In some embodiments, any pharmaceutical composition described herein has a mucoadhesive characteristic such that when a single dose of the pharmaceutical composition is orally administered to an individual, the pharmaceutical composition adheres to surface of the gastrointestinal tract (e.g., the mucosa and/or epithelium of the gastrointestinal tract or of a specific site of the gastrointestinal tract, such as the esophagus) of the individual for a time sufficient to allow topical delivery of a therapeutically effective amount of the corticosteroid to the esophagus.

The pharmaceutical composition of the invention is for use in treating, preventing or alleviating esophageal inflammation.

In certain embodiments, the corticosteroid is administered in about 0.1 mg to about 20 mg, or about 0.3 mg to about 4 mg, or about 0.25 mg to about 5 mg per day.

In certain embodiments, administration of a pharmaceutical composition described herein is to an individual that has been diagnosed with eosinophilic esophagitis, an inflammatory bowel disease involving the esophagus, Crohn's disease, celiac disease, proximal gastrointestinal pathology (e.g., in individuals suffering from hypofunctioning gallbladder), eosinophilic gastrointestinal inflammation, eosinophilic duodenitis, duodenal eosinophilia, functional dyspepsia, intermediate esophagitis, esophageal inflammation secondary to caustic/irritant ingestion, persistent/recurrent esophageal strictures of any cause and including caustic/irritant ingestion, pill-induced esophagitis, systemic diseases, congenital diseases, post-surgery inflammation, intermediate esophagitis, epithelial hyperplasia, basal cell hyperplasia, elongated papillae, dilated vessels in papillae, fungal esophagitis (e.g., Candida, turolopsis, histoplasma Aspergillus, etc.), viral esophagitis (e.g., HSV, CMV, V2V), bacterial esophagitis (e.g., tuberculosis, actinomycosis, syphlis), corrosive esophagitis, radiation esophagitis, chemotherapy esophagitis, graft vs. host disease, a skin disease with esophageal involvement (e.g., bullous pemphigoid, pemphigus vulgaris, epidermolysis bollosa, Stevens-Johnson syndrome), Behcet's disease, sarcoidosis, idiopathic esophagitis, eosinophilic gastritis, Menetrier's disease, parasitic gastritis, lymphocytic esophagitis, inflammatory bowel disease-associated esophagitis, , parasitic gastritis, or gastro enteritis. In specific embodiments, the individual has eosinophilic esophagitis. In some specific embodiments, the individual has been diagnosed with gastroesophageal reflux disease (GERD), nonerosive reflux disease (NERD), or erosive esophagitis. In some embodiments, the
inflammation of the gastrointestinal tract is inflammation of the stomach and/or the small intestines, e.g., gastro enteritis.

In certain embodiments, the individual administered a pharmaceutical composition for the treatment, prevention or alleviation of esophageal inflammation is a child or an infant. In various embodiments the child or infant is less than 16 years old, less than 12 years old, less than 8 years old, less than 6 years old, less than 4 years old or less than 2 years old.

Also disclosed herein is a kit comprising a multiple unit container and a plurality of unit doses of a pharmaceutical composition (e.g., any pharmaceutical composition described herein). Also described herein is a unit dose of a pharmaceutical composition comprising:
a. a therapeutically effective amount of corticosteroid,
b. edetate,
c. citrate,
d. polysorbate 80,
e. an optional preservative,
f. an optional flavoring agent,
g. an optional sweetener,
h. at least one additional excipient, and
i. a liquid vehicle.

In some embodiments, the at least one additional excipient comprises maltodextrin, dextrose, hydroxyethylcellulose (HEC), hydroxypropylmethyl-cellulose (HPMC), carboxymethyl-cellulose (CMC), microcrystalline cellulose (MCC), carbomer or combinations thereof.

In certain embodiments, a kit provided for herein comprises a stable pharmaceutical composition that is physically and chemically stable. In some embodiments, a kit provided for herein comprises, by way of non-limiting example, about 10 to about 60, about 14, or about 30 doses of the pharmaceutical composition. In certain embodiments, a kit provided for herein comprises, by way of non-limiting example, about 50 mL to about 500 mL, about 150 mL, about 330 mL or about 55 mL of the stable pharmaceutical composition. In some embodiments, any kit provided herein further comprises a metering device (e.g., a spoon, cup, pump, or the like) for administering the composition to an individual. In various embodiments, the metering device is incorporated into or separate from the multiple unit container.

### BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings of which:

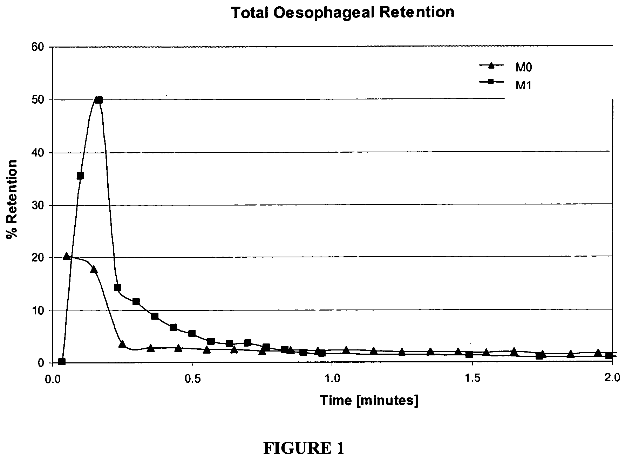

Figure 1 illustrates the percent amount of composition present in the esophagus as a function of time following oral administration (by measuring the amount of radiolabel present in the esophagus).

### DETAILED DESCRIPTION OF THE INVENTION

The composition of the invention is for use in treating, preventing or alleviating esophageal inflammation.

Also disclosed herein is a composition comprising a corticosteroid, dextrose, maltodextrin, edetate, citrate, polysorbate 80, an optional preservative, an optional flavoring agent, at least one additional excipient, and, a liquid vehicle. The composition of the invention comprises a preservative. In further or alternative embodiments, the liquid vehicle is an aqueous liquid vehicle (e.g., water) or comprises an aqueous medium. Also described herein are methods , in form of products for use,
comprising orally administering to an individual in need thereof a composition
comprising a corticosteroid, dextrose, maltodextrin, edetate, citrate, polysorbate 80, an optional preservative, an optional flavoring agent, at least one additional excipient, and, a liquid vehicle. The composition of the invention comprises a preservative and dextrose.

The composition comprises a flavoring agent, a sweetener, or a combination thereof.
In further or alternative embodiments, the liquid vehicle is an aqueous medium (e.g., water). The pharmaceutical composition is chemically and physically stable.

An individual suitable for treatment with the compositions disclosed herein may, for example, have been diagnosed with a disease or condition including, but not limited to, eosinophilic esophagitis, inflammatory bowel diseases involving the esophagus, Crohn's disease, celiac disease, proximal gastrointestinal pathology (e.g., in individuals suffering from hypofunctioning gallbladder), eosinophilic gastrointestinal inflammation, celiac disease, eosinophilic duodenitis, duodenal eosinophilia, functional dyspepsia, intermediate esophagitis, epithelial hyperplasia, basal cell hyperplasia, elongated papillae, dilated vessels in papillae, fungal esophagitis (e.g., Candida, turolopsis, histoplasma Aspergillus, etc.), viral esophagitis (e.g., HSV, CMV, V2V), bacterial esophagitis (e.g., tuberculosis, actinomycosis, syphlis), corrosive esophagitis, radiation esophagitis, chemotherapy esophagitis, graft vs. host disease, a skin disease with esophageal involvement (e.g., bullous pemphigoid, pemphigus vulgaris, epidermolysis bollosa, Stevens-Johnson syndrome), Behcet's disease, sarcoidosis, idiopathic esophagitis, eosinophilic gastritis, Menetrier's disease, parasitic gastritis, lymphocytic esophagitis, inflammatory bowel disease-associated esophagitis, , parasitic gastritis, esophageal inflammation secondary to caustic/irritant ingestion, persistent/recurrent esophageal strictures of any cause and including caustic/irritant ingestion, pill-induced
esophagitis, systemic diseases, congenital diseases, post-surgery inflammation or gastro enteritis. The composition may also be used in treating individuals diagnosed with other gastrointestinal disorders, including stomach and duodenal ulcers, hyperactive acidic discharge disorders, such as Zollinger-Ellison syndrome and laryngeal disorders. In some embodiments, the compositions or methods disclosed herein are used in methods of treating individuals diagnosed with other gastrointestinal disorders, including, by way of non-limiting example, Barrett's Esophagus, gastroesophageal reflux disease (GERD), nonerosive reflux disease (NERD), or erosive esophagitis. In some embodiments, the methods of treating, preventing or alleviating inflammation or symptoms of inflammation include methods of treating any of the gastrointestinal disorders described herein. In certain embodiments, these methods comprise orally administering to said individual a corticosteroid-containing compositions described herein.

Provided herein are methods, in form of products for use in such methods, for treating, preventing and alleviating any chronic inflammatory or malignant state that involves the gastrointestinal tract, such as the esophagus, and responds to steroid therapy. The compositions of the present invention are useful, for example, for treating, preventing and alleviating esophageal inflammation and/or symptoms and associated with eosinophilic esophagitis, inflammatory bowel diseases involving the esophagus, Crohn's disease, celiac disease, proximal gastrointestinal pathology (e.g., in individuals suffering from hypofunctioning gallbladder), eosinophilic gastrointestinal inflammation, celiac disease, eosinophilic duodenitis, duodenal eosinophilia, functional dyspepsia, intermediate esophagitis, epithelial hyperplasia, basal cell hyperplasia, elongated papillae, dilated vessels in papillae, fungal esophagitis (e.g., Candida, turolopsis, histoplasma Aspergillus, etc.), viral esophagitis (e.g., HSV, CMV, V2V), bacterial esophagitis (e.g., tuberculosis, actinomycosis, syphlis), corrosive esophagitis, radiation esophagitis, chemotherapy esophagitis, eosinophilic gastric outlet obstruction and related inflammation, graft vs. host disease, a skin disease with esophageal involvement (e.g., bullous pemphigoid, pemphigus vulgaris, epidermolysis bollosa, Stevens- Johnson syndrome), Behcet's disease, sarcoidosis, idiopathic esophagitis, eosinophilic gastritis, Menetrier's disease, parasitic gastritis, lymphocytic esophagitis, inflammatory bowel disease-associated esophagitis, , parasitic gastritis, esophageal inflammation secondary to caustic/irritant ingestion, persistent/recurrent esophageal strictures of any cause and including caustic/irritant ingestion, pill-induced esophagitis, systemic diseases, congenital diseases, Epidermolysis bullosa, post-surgery inflammation, and gastro enteritis. The present compositions are also useful for treating, preventing or alleviating esophageal inflammation associated with other diseases or conditions of the gastrointestinal tract, for example, the upper gastrointestinal tract, where it is beneficial to target a particular target site, rather than provide systemic therapy. As used herein, inflammation and/or symptoms associated with a disorder or disease disclosed herein includes inflammation and/or symptoms associated with, caused by and/or resulting from the disorder or disease. In some embodiments, provided herein is a method of reducing cytokine and/or chemokine release in the gastrointestinal tract, such as the esophagus (e.g., in the mucosa or epithelium thereof) by administering a composition described herein to the gastrointestinal tract (e.g., esophagus). In certain embodiments, provided is a method of decreasing eosinophil migration to the gastrointestinal tract (e.g., the esophagus) by administering a composition described herein to the gastrointestinal tract (e.g., the esophagus).

Also disclosed herein is a pharmaceutical
composition comprising a physically and chemically stable
composition comprising: (a) a therapeutically effective amount of corticosteroid; (b) edetate; (c) citrate; (d) polysorbate 80; (e) a preservative,
a flavoring agent, a sweetener, or a combination thereof; (f) at least one
additional excipient; and (g) a liquid vehicle. In specific embodiments, such compositions are suitable for single or multiple dose administration. Also disclosed herein is a pharmaceutical composition comprising a physically and chemically stable composition comprising: (a) a therapeutically effective amount of corticosteroid; (b) an antioxidant; (c) a buffer; (d) a surfactant; (e) a preservative, a flavoring agent, a sweetener, or a combination thereof; (f) at least one additional excipient, and (g) a liquid vehicle. The compositions of the invention are suitable for single or multiple dose administration. The compositions of the invention comprise a flavoring agent and/or a sweetener, or a combination thereof. Also disclosed herein is a pharmaceutical composition comprising a physically and chemically stable composition comprising: (a) a therapeutically effective amount of corticosteroid; (b) an optional antioxidant; (c) an optional buffer; (d) an optional surfactant; (e) an optional preservative; (f) an optional flavoring agent; (g) an optional sweetener; (h) at least one additional excipient, and (g) an optional vehicle (e.g., an aqueous vehicle).

As used herein, unless otherwise stated, the use of the terms "a", "an" and "the" include both singular and multiple embodiments. As used herein, the term "individual" includes any animal. In some embodiments, the animal is a mammal. In certain embodiments, the mammal is a human. In specific embodiments, the human is an adult. In other embodiments, the human is a child (e.g., a child under 12 or a child under 6). In certain embodiments, the human is an infant. As used herein, the phrase "method of treating" or "method for treating" can, in some embodiments, encompass methods of preventing, reducing the incidences of, providing prophylactic treatment, treating and alleviating. As used herein, the phrase "an effective amount" and "a therapeutically effective amount" is an amount sufficient to elicit a change in the symptoms of or inflammation associated with gastrointestinal disorders, including but not limited to esophageal inflammation, eosinophilic esophagitis, GERD, NERD, or erosive esophagitis. As used herein, the term "or" includes "and" and "or".

As used herein, the phrase "treating inflammatory diseases involving the esophagus" includes treating symptoms of such diseases and treating inflammation associated with the diseases.

It is to be understood that any composition disclosed herein or method comprising administration of a composition disclosed herein that comprises a salt or an acid includes the disclosure of the disassociated form of the salt or acid. For example, if dissolved, sodium carboxymethylcellulose may disassociate into its sodium cationic part or parts and the corresponding carboxymethylcellulose anionic part.

### Methods and Compositions

In certain embodiments, the corticosteroids used in the present invention include topical steroids including, for example, budesonide or fluticasone propionate. In some embodiments, corticosteroids are selected from, by way of non-limiting example, aclometasone, amcinomide, beclometasone, betamethasone, budesonide, ciclesonide, clobetasol, clobetasone, clocortolone, cloprednol, cortivazol, deflazacort, deoxycorticosterone, desonide desoximetasone, dexamethasone, diflorasone, diflucortolone, difluprednate, fluclorolone, fludrocortisone, fludroxycortide, flumetasone, flunisolide, fluocinolone acetonide, fluocinonide, fluocortin, fluocortolone, fluorometholone, fluperolone, fluticasone, fuprednidene, formocortal, halcinonide, halometasone, hydrocortisone aceponate, hydrocortisone buteprate, hydrocortisone butyrate, loteprednol, medrysone, meprednisone, methylprednisolone, methylprednisolone aceponate, mometasone furoate, paramethasone, prednicarbate, prednisone, prednisolone, prednylidene, remexolone, tixocortol, triamcinolone and ulobetasol, and combinations, pharmaceutically acceptable salts and esters thereof. In a specific embodiment, the corticosteroid is budesonide. In another embodiment, the corticosteroid is an ester of fluticasone, e.g., fluticasone propionate.

Provided herein are oral pharmaceutical compositions for use in treating, preventing or alleviating esophageal inflammation.

A corticosteroid (e.g., budesonide or fluticasone propionate) that is administered
in oral form, is delivered to the esophagus in an effective dose to reduce the inflammation of the esophagus.

In one aspect, an exemplary corticosteroid is budesonide, 16,17-(butylidenebis(oxy))-11,21-dihydroxy-, (11-β,16-α)-pregna-1,4-diene-3,20-dione, or fluticasone propionate, S-(fluoromethyl)6α,9-difluoro-11β-17-dihydroxy-16α-methyl-3-oxoandrosta-1,4-diene-17β-carbothioate, 17 propionate or (6α,11β,16α,17β)-6,9-Difluoro-11-hydroxy-16-methyl-3-oxo-17-(1-oxopropoxy)androsta-1,4-diene-17- carbothioic acid S-(fluoromethyl) ester.

In certain embodiments, the corticosteroid(s) utilized herein are utilized as particles (e.g., corticosteroid particles suspended or dispersed in an aqueous medium). In specific embodiments, the particles are microparticles. In some embodiments, the microparticles have a mean diameter of 0.1 microns to 50 microns. The microparticles may have a mean diameter of about 1 micron to about 20
microns. In certain embodiments, at least 95%, at least 98%, or at least 99% of the microparticles have a diameter of less than 10 microns.

A composition or formulation described herein may comprise less than 50% w/w, less than 40% w/w, less than 30% w/w, less than 20% w/w, less than 10% w/w, less than 8% w/w, less than 6% w/w, less than 5% w/w, less than 4% w/w, less than 3% w/w, less than 2% w/w, or about 2% w/w, less than 1% w/w, less than 0.5% w/w, less than 0.3% w/w, less than 0.2% w/w, or about 0.2% w/w of undissolved particles. In certain embodiments, a composition or formulation described herein is substantially free of non-corticosteroid particles.

In certain embodiments, pharmaceutical compositions disclosed herein and used herein comprise one or more excipients and/or one or more additional active agents. Excipients useful herein include, by way of non-limiting example, mucoadhesive agents, viscosity enhancing agents, binders, fillers (e.g., corn starch), lubricants, solvents, suspension agents, flavoring agents, coloring agents, sweeteners, preservatives, antioxidants, buffering agents, humectants, chelating agents, surfactants, and the like. As used herein, a mucoadhesive agent is an agent that adheres to a gastrointestinal surface (e.g., either or both of a gastrointestinal epithelia or mucosa).

Additional excipients are as described herein and are used in any suitable amounts, e.g., as described herein. Antioxidants, buffering agents, and surfactants are used in suitable amounts. In certain embodiments, the pharmaceutical composition provided herein is stable. In specific embodiments, the pharmaceutical composition is chemically and/or physically stable.

Also described herein are compositions or formulations comprising a corticosteroid (e.g., budesonide or fluticasone propionate), one or more excipient that increases the interaction of the composition with a surface of the gastrointestinal tract (e.g., an agent that enhances viscosity, mucoadhesive character, adsorption to a mucosal layer, and/or absorption of an active through the surface layer), optionally one or more binder, optionally one or more filler, optionally one or more lubricant, optionally one or more solvent, optionally one or more suspension agent, optionally one or more flavoring agent, optionally one or more coloring agent, optionally one or more sweetener, optionally one or more preservative, optionally one or more antioxidant, optionally one or more buffering agent, optionally one or more humectant, optionally one or more chelating agent, and optionally one or more surfactant. In certain instances, the surface of the gastrointestinal tract is a mucosa and/or epithelium of the gastrointestinal tract or of a specific site of the gastrointestinal tract, such as the esophagus. Also disclosed herein is a pharmaceutical composition comprising a corticosteroid, an antioxidant, a buffering agent, a surfactant, an optional preservative, an optional flavoring agent, at least one additional excipient, and, optionally, water.

Preservatives include, by way of non-limiting example, benzalkonium chloride, cetrimide (cetyltrimethylammonium bromide), benzoic acid, benzyl alcohol, methyl-, ethyl-,propyl-and butyl-esters of parahydroxybenzoic acid, chlorhexidine, chlorobutanol, phenylmercuric acetate, borate and nitrate, potassium sorbate, sodium benzoate, sorbic acid, thiomersal (mercurithiosalicylate), combinations thereof, or the like. Compositions and formulations described herein optionally include about 0.1% w/w to about 5% w/w, about 0.1% w/w to about 3% w/w, about 0.1% w/w to about 1% w/w, about 0.1% w/w to about 0.5% w/w, about 0.2% w/w of one or more preservative(s).

Antioxidants include, by way of non-limiting example, ascorbyl palmitate, butylated hydroxyanisole, butylated hydroxytoluene, monothioglycerol, sodium ascorbate, sodium formaldehyde sulfoxylate, sodium metabisulfite, BHT, BHA, sodium bisulfite, vitamin E or a derivative thereof, propyl gallate, edetate (EDTA) (e.g., disodium edetate), Diethylenetriaminepentaacetic acid (DTPA), Triglycollamate (NT), combinations thereof, or the like. Compositions and formulations described herein optionally include of about 0.01% w/w to about 1% w/w, about 0.01% w/w to about 0.5% w/w, about 0.01% w/w to about 0.3% w/w, or about 0.01% w/w to about 0.1% w/w one or more antioxidant(s).

Buffering agents include, by way of non-limiting example, citrate buffers (i.e., citric acid and citrate), carbonates (e.g., calcium carbonate), hydroxides (e.g., magnesium hydroxide), phosphate buffers, acetate buffers, combinations thereof, or the like.

Humectants include, by way of non-limiting example, glycerine, propylene glycol, ethylene glycol, glyceryl triacetate, polyols (e.g., sorbitol, xylitol, maltitol, polydextrose), and the like. Compositions and formulations described herein optionally include about 0.1% w/w to about 10% w/w, about 1% w/w to about 10% w/w, about 1% to about 8% w/w, or about 5% w/w of a humectant. In certain embodiments, humectants inhibit or reduce precipitation and/or crystallization of one or more component of a composition or formulation described herein (e.g., a sweetener, mucoadhesive agent or a viscosity enhancing agent).

Chelating agents include, by way of non-limiting example, edetate (EDTA) (e.g., disodium edetate), Diethylenetriaminepentaacetic acid (DTPA), Triglycollamate (NT), or the like. Compositions and formulations described herein optionally include about 0.01% w/w to about 0.5% w/w, about 0.01% w/w to about 0.3% w/w, about 0.01% w/w to about 0.1% w/w, or about 0.05% w/w of one or more chelating agent. In some embodiments, antioxidants or chelating agents (e.g., EDTA) are present in an amount of about 0.05 mg/mL to about 25 mg/mL.

In certain embodiments, sweeteners include, by way of non-limiting example, sugar, glycerin, acesulfame potassium (AceK), mono-ammonium glycyrrhizinate (e.g., Magnasweet®), sucrose, lactose, glucose, fructose, arabinose, xylose, ribose, mannose, galactose, dextrose, sorbose, sorbitol, mannitol, maltose, cellobiose, xylitol and the like. In specific embodiments, the sweetener includes glycerin, acesulfame potassium and mono-ammonium glycyrrhizinate. Sweeteners are optionally included in any suitable amount including, by way of non-limiting example, about 0.01% w/w to about 30% w/w, about 0.1% w/w to about 5% w/w, about 5% w/w to about 20% w/w, about 0.5% w/w, about 0.8% w/w, about 1% w/w, about 6% w/w, about 7% w/w, about 8% w/w, about 9% w/w, about 10% w/w, about 11% w/w, about 12% w/w, about 13% w/w, about 14% w/w, about 15% w/w, about 16% w/w, about 17% w/w, about 18% w/w or about 19% w/w. In some embodiments, flavoring agents include, by way of non-limiting example, peppermint, orange, bubble gum, wintergreen, grape and cherry. Any suitable amount of flavoring agent is optionally utilized including, e.g., about 0.01% w/w, about 0.1% w/w, about 0.2% w/w, about 0.3% w/w, about 0.4% w/w, about 0.5% w/w, about 0.6% w/w, about 0.7% w/w, about 0.8% w/w, about 0.9% w/w, about 1% w/w, up to 5% w/w, up to 10% w/w, or up to 50% w/w. In certain embodiments, a composition described herein has a reduced amount of sugar sweetener (e.g., less than 20% w/w, less than 15% w/w, less than 10% w/w, less than 9% w/w, less than 8% w/w, less than 7% w/w, less than 6% w/w, less than 5% w/w, less than 4% w/w, less than 3% w/w, or less than 2% w/w) and/or a preservative to ensure stability of the composition (e.g., to reduce microbe proliferation). In specific embodiments, glycyrrhizinate such as mono-ammonium glycyrrhizinate (e.g., Magnasweet®) is present in an amount of about 0.01% w/w to about 2.95% w/w. In certain embodiments, coloring agents include yellow agents (e.g., FD&C 5 and/or 6), red agents (e.g., FD&C Red 40, Red No. 3), blue, or the like.

Surfactants include, e.g., anionic, cationic, non-ionic, or zwitterionic surfactants, such as, by way of non-limiting example, polysorbate (e.g., polysorbate 20, polysorbate 60, polysorbate 40, polysorbate 80, polysorbate 81, polysorbate 85, polysorbate 120), bile acids or their salts (e.g., sodium taurocholates, sodium deoxytaurocholates, chenodeoxycholic acid, and ursodeoxycholic acid), nonoxynol or polyoxyethylene glycol fatty acid esters, pluronic or poloxamers such as Pluronic F68, Pluronic L44, Pluronic L101, combinations thereof, or the like. In specific embodiments, the surfactant is polysorbate 80. Compositions and formulations described herein optionally include any suitable amount of surfactant, e.g., about 0.001% w/w to about 0.5% w/w, about 0.001% w/w to about 0.3% w/w, about 0.001% w/w to about 0.1% w/w, or about 0.01% w/w of one or more surfactant.

Also described herein is a pharmaceutical composition comprising a corticosteroid, edetate, citrate, polysorbate 80, an optional preservative, an optional flavoring agent, at least one additional excipient, and, optionally, water. In more specific embodiments, the composition comprises water. In further or alternative embodiments, the at least one additional excipient is selected from cellulose (including cellulose derivatives),, dextrose, one or more maltodextrin, hydroxypropylmethyl-cellulose (HPMC), carboxymethyl-cellulose (CMC) (including, e.g., sodium carboxymethyl-cellulose (NaCMC)), microcrystalline cellulose (MCC), carbomer, hydroxyethyl cellulose (HEC) and combinations thereof. In a specific embodiment, the corticosteroid is selected from budesonide, fluticasone propionate and combinations thereof. In a more specific embodiment, the corticosteroid is budesonide. In another specific embodiment, the corticosteroid is fluticasone priopionate. In a further or additional embodiment, the at least one additional excipient comprises or is hydroxypropylmethyl-cellulose (HPMC). In a specific embodiment, the at least one additional excipient comprises or is carboxymethyl-cellulose (CMC) (including, e.g., sodium carboxymethyl-cellulose (NaCMC)). In a specific embodiment, the at least one additional excipient comprises or is microcrystalline cellulose (MCC). In a specific embodiment, the at least one additional excipient comprises or is carbomer (i.e., a high molecular weight cross-linked polyacrylic acid). In a specific embodiment, the at least one additional excipient comprises or is a combination of CMC and MCC.

In specific embodiments, an excipient that increases the interaction of the composition with a surface of the gastrointestinal tract (e.g., the mucosa and/or epithelium of the gastrointestinal tract or of a specific site of the gastrointestinal tract, such as the esophagus) utilized in any composition or formulation described herein comprises at least one maltodextrin. In certain embodiments, the excipient that increases the interaction of the composition with a surface of the gastrointestinal tract (e.g., maltodextrin) is substantially or at least partially dissolved in a liquid vehicle. In some embodiments, any composition or formulation described herein comprises a first excipient that increases the interaction of the composition with a surface of the gastrointestinal tract (e.g., maltodextrin) that is substantially or at least partially dissolved in a liquid vehicle (or substantially soluble in saliva when orally administered) and a second excipient that increases the interaction of the composition with a surface of the gastrointestinal tract that is substantially insoluble in a liquid vehicle (or in saliva when orally administered). In some embodiments, an oral pharmaceutical composition described herein comprises less than about 0.1 g or less than about 1 g of maltodextrin for every mL of liquid vehicle in the oral pharmaceutical composition. In certain instances, a
composition or formulation described herein comprises less than 2 g of maltodextrin/mL of composition, less than 1.5 g of maltodextrin/mL of composition, less than 1 g of maltodextrin/mL of composition, less than 0.5 g of maltodextrin/mL of composition, less than 0.25 g/mL of maltodextrin/mL of composition, about 0.05 g of maltodextrin/mL of composition to about 0.5 g of maltodextrin/mL of composition, about 0.05 g of maltodextrin/mL of composition to about 0.4 g of maltodextrin/mL of composition, about 0.05 g of maltodextrin/mL of composition to about 0.3 g of maltodextrin/mL of composition, about 0.1 g of maltodextrin/mL of composition to about 0.5 g of maltodextrin/mL of composition, about 0.1 g of maltodextrin/mL of composition to about 0.4 g of maltodextrin/mL of composition, about 0.1 g of maltodextrin/mL of composition to about 0.3 g of maltodextrin/mL of composition, about 0.2 g of maltodextrin/mL of composition to about 0.5 g of maltodextrin/mL of composition, about 0.2 g of maltodextrin/mL of composition to about 0.4 g of maltodextrin/mL of composition, or about 0.2 g of maltodextrin/mL of composition to about 0.3 g of maltodextrin/mL of composition. In some embodiments, any composition or formulation described herein comprises greater than about 7% w/w, greater than about 8% w/w, greater than about 9% w/w, greater than about 10% w/w, greater than about 11% w/w, greater than about 12% w/w, greater than about 13% w/w, greater than about 14% w/w, greater than about 15% w/w, greater than about 16% w/w, greater than about 17% w/w, greater than about 18% w/w, greater than about 19% w/w, greater than about 20% w/w, greater than about 21% w/w, greater than about 22% w/w, greater than about 23% w/w, greater than about 24% w/w, greater than about 25% w/w, greater than about 26% w/w, greater than about 27% w/w, greater than about 28% w/w, greater than about 29% w/w or greater than about 30% w/w of maltodextrin. In some embodiments, the maltodextrin is substantially dissolved in the liquid vehicle. In certain embodiments, the maltodextrin has a dextrose equivalents (DE) of greater than 4, greater than 5, greater than 10, greater than 11, greater than 12, greater than 13, greater than 14, greater than 15, 15, 4 to 10, 4 to 9, 4 to 8, 4 to 7 (e.g., M150), 11 to 20, 12 to 19, 13 to 18, 13 to 17 (e.g., M440), or
14 to 16. In some embodiments, a composition described herein comprises a first maltodextrin and a second maltodextrin. In specific embodiments, the first maltodextrin has a DE of about 4 to about 10, about 4 to about 9, or about 4 to about 8 and the second maltodextrin has a DE of about 10 to about 20, about 12 to about 19, or about 13 to about 18. In some embodiments, at least one maltodextrin utilized in a composition described herein has a molecular weight high enough to increase the solubility of a corticosteroid, or to increase the suspendability of a corticosteroid particle.

As used herein, "edetate" includes all compounds of Formula I wherein each R is independently selected from an H and a negative charge (e.g., as a salt or as a disassociated salt or acid). In certain embodiments, edetate is selected from, by way of non-limiting example, disodium edetate, calcium edetate, ethylenediaminetetraacetic acid and the like.

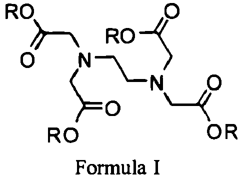

As used herein, "citrate" includes all compounds of Formula II wherein each R is independently selected from an H and a negative charge (e.g., as a salt or as a disassociated salt or acid). In certain embodiments, citrate is selected from, by way of non-limiting example, sodium citrate, citric acid and the like.

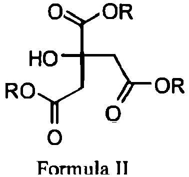

In certain embodiments, sweeteners include, by way of non-limiting example, sucrose, lactose, glucose, fructose, arabinose, xylose, ribose, mannose, galactose, dextrose, sorbose, sorbitol, mannitol, maltose, cellobiose, xylitol and the like. In some embodiments, flavoring agents include, by way of non-limiting example, peppermint, orange, bubble gum, wintergreen, grape and cherry.

Preservatives include, by way of non-limiting example, benzalkonium chloride, methylparaben (e.g., sodium methylparaben), propylparaben, potassium sorbate and sodium benzoate. In specific embodiments, the preservative is potassium sorbate.

In certain embodiments, a composition provided herein comprises or is prepared by combining the components set forth in any of Tables 1-13. In various embodiments, one or more of maltodextrin, dextrose, HEC, CMC, MCC, Carbomer and HPMC are utilized therein. The compositions are optionally prepared in any volume (e.g., any volume described herein) comprising the components in a ratio as described in of Tables 1-13.

Only the compositions comprising dextrose and a preservative form part of the invention.

**Table 1: Budesonide Composition #1**

| **Ingredient** | **Amount** |
|---|---|
| Budesonide | 1 mg to 150 mg |
| CMC, MCC, Carbomer, HPMC and/or HEC | 0.5 g to 10 g |
| Dextrose | 0 g to 100 g |
| Maltodextrin | 0 g to 100 g |
| EDTA (e.g., disodium edetate) | 5 mg to 200 mg |
| Citric Acid | 10 mg to 1 g |
| Citrate (e.g., sodium citrate) | 10 mg to 2 g |
| Polysorbate 80 (e.g., Tween 80) | 5 mg to 100 mg |
| Flavoring Agent | optional |
| Sweetener | optional |
| Preservative | optional |
| Water | q.s. to 100 mL |

**Table 2: Budesonide Composition #2**

| **Ingredient** | **Amount** |
|---|---|
| Budesonide | 1 mg to 150 mg |
| CMC, MCC, Carbomer, HPMC and/or HEC | 0 g to 10 g |
| Dextrose | 1 g to 100 g |
| Maltodextrin | 0 g to 100 g |
| EDTA (e.g., disodium edetate) | 5 mg to 200 mg |
| Citric Acid | 10 mg to 1 g |
| Citrate (e.g., sodium citrate) | 10 mg to 2 g |
| Polysorbate 80 (e.g., Tween 80) | 5 mg to 100 mg |
| Flavoring Agent | optional |
| Sweetener | optional |
| Preservative | optional |
| Water | q.s. to 100 mL |

**Table 3: Budesonide Composition #3**

| **Ingredient** | **Amount** |
|---|---|
| Budesonide | 1 mg to 150 mg |
| CMC, MCC, Carbomer, HPMC and/or HEC | 0 g to 10 g |
| Dextrose | 0 g to 100 g |
| Maltodextrin | 1 g to 100 g |
| EDTA (e.g., disodium edetate) | 5 mg to 200 mg |
| Citric Acid | 10 mg to 1 g |
| Citrate (e.g., sodium citrate) | 10 mg to 2 g |
| Polysorbate 80 (e.g., Tween 80) | 5 mg to 100 mg |
| Flavoring Agent | optional |
| Sweetener | optional |
| Preservative | optional |
| Water | q.s. to 100 mL |

**Table 4: Budesonide Composition #4**

| **Ingredient** | **Amount** |
|---|---|
| Budesonide | 0.5 mg to 2 mg |
| CMC and MCC (e.g., Avicel RC-591) | 0.01 g to 0.3 g |
| Dextrose | 0.1 gto 1 g |
| Maltodextrin | 0.5 g to 2 g |
| EDTA (e.g., disodium edetate) | 1 mg to 10 mg |
| Citric Acid | 0.1 mg to 100 mg |
| Citrate (e.g., sodium citrate) | 0.1 mg to 200 mg |
| Polysorbate 80 (e.g., Tween 80) | 0.1 mg to 10 mg |
| Cherry Flavor | 1 mg to 100 mg |
| Sweetener | 100 mg to 1 g |
| Sodium Benzoate | 1 mg to 50 mg |
| Potassium Sorbate | 1 mg to 50 mg |
| Water | q.s. to 5 mL |

**Table 5: Budesonide Composition #5**

| **Ingredient** | **Amount** |
|---|---|
| Budesonide | 0.5 mg to 2 mg |
| CMC and MCC (e.g., Avicel RC-591) | 0.02 g to 0.6 g |
| Dextrose | 0.2 g to 2 g |
| Maltodextrin | 1 g to 4 g |
| EDTA (e.g., disodium edetate) | 2 mg to 20 mg |
| Citric Acid | 0.2 mg to 200 mg |
| Citrate (e.g., sodium citrate) | 0.2 mg to 400 mg |
| Polysorbate 80 (e.g , Tween 80) | 0.2 mg to 20 mg |
| Cherry Flavor | 2 mg to 200 mg |
| Sweetener | 200 mg to 2 g |
| Sodium Benzoate | 2 mg to 100 mg |
| Potassium Sorbate | 2 mg to 100 mg |
| Water | q.s. to 10 mL |

**Table 6: Budesonide Composition #6**

| **Ingredient** | **Amount** (mg/mL) |
|---|---|
| Budesonide | 0.01 to 0.5 |
| CMC and MCC (e.g., Avicel RC-591) | 2 to 100 |
| Dextrose | 10 to 500 |
| Maltodextrin (M150) | 10 to 500 |
| EDTA (e.g., disodium edetate) | 0.01 to 10 |
| Citric acid | 0.1 to 10 |
| Citrate (e.g., sodium citrate) | 0.1 to 10 |
| Polysorbate 80 (e.g., Tween 80) | 0.01 to 1 |
| Flavoring agent (e.g., Cherry Flavor) | 0.1 to 100 |
| Glycerin | 10 to 100 |
| Acesulfame potassium | 0.1 to 40 |
| Magnasweet 110 | 0.1 to 40 |
| Sodium Benzoate | 0.1 to 10 |
| Potassium Sorbate | 0.1 to 10 |
| Water | q.s. to 1-15 mL |

**Table 7: Budesonide Composition #7**

| **Ingredient** | **Amount** (mg/mL) |
|---|---|
| Budesonide | about 0.05 to about 0.2 |
| CMC and MCC (e.g., Avicel RC-591) | 5 to 50 |
| Dextrose | 50 to 250 |
| Maltodextrin (M150) | 200 to 500 |
| EDTA (e.g., disodium edetate) | 0.1 to 1 |
| Citric acid | 0.5 to 5 |
| Citrate (e.g., sodium citrate) | 0.2 to 2 |
| Polysorbate 80 (e.g., Tween 80) | 0.01 to 0.4 |
| Flavoring agent (e.g., Cherry Flavor) | 1 to 10 |
| Glycerin | 30 to 80 |
| Acesulfame potassium | 1 to 10 |
| Magnasweet 110 | 1 to 10 |
| Sodium Benzoate | 0.5 to 4 |
| Potassium Sorbate | 0.5 to 4 |
| Water | q.s. to 1-15 mL |

**Table 8: Budesonide Composition #8**

| **Ingredient** | **Amount** (mg/mL) | **Amount % w/w** |
|---|---|---|
| Budesonide | 0.05 | 0.004 |
| Avicel RC-591 | 23.6 | 2 |
| Dextrose | 118.0 | 10 |
| Maltodextrin (M150) | 306.8 | 26 |
| Disodium edetate | 0.59 | 0.05 |
| Citric acid | 1.77 | 0.15 |
| Sodium citrate | 0.59 | 0.05 |
| Polysorbate 80 | 0.12 | 0.01 |
| Cherry Flavor | 5.9 | 0.5 |
| Glycerin | 59.0 | 5 |
| Acesulfame potassium | 5.9 | 0.5 |
| Magnasweet 110 | 5.9 | 0.5 |
| Sodium Benzoate | 2.36 | 0.2 |
| Potassium Sorbate | 2.36 | 0.2 |
| Water | q.s. to 1, 2, 3, 4, 5, 6, 7, 8, 9, 10, 11, 12,13, 14,or 15 mL | q.s. to 1, 2, 3, 4, 5, 6, 7, 8, 9, 10, 11, 12, 13, 14, or 15 mL |

**Table 9: Budesonide Composition #9**

| **Ingredient** | **Amount** (mg/mL) | **Amount % w/w** |
|---|---|---|
| Budesonide | 0.2 | 0.17 |
| Avicel RC-591 | 23.6 | 2 |
| Dextrose | 118 | 10 |
| Maltodextrin (M150) | 306.8 | 26 |
| Disodium edetate | 0.59 | 0.05 |
| Citric acid | 1.77 | 0.15 |
| Sodium citrate | 0.59 | 0.05 |
| Polysorbate 80 | 0.12 | 0.01 |
| Cherry Flavor | 5.9 | 0.5 |
| Glycerin | 59 | 5 |
| Acesulfame potassium | 5.9 | 0.5 |
| Magnasweet 110 | 5.9 | 0.5 |
| Sodium Benzoate | 2.36 | 0.2 |
| Potassium Sorbate | 2.36 | 0.2 |
| Water | q.s. to 1, 2, 3, 4, 5, 6, 7, 8, 9, 10, 11, 12, 13, 14,or 15 mL | q.s. to 1, 2, 3, 4, 5, 6, 7, 8, 9, 10, 11, 12, 13, 14,or 15 mL |

**Table 10: Fluticasone Propionate Composition #1**

| **Ingredient** | **Amount** |
|---|---|
| Fluticasone Propionate | 0.5 mg to 150 mg |
| CMC, MCC, Carbomer, HPMC and/or HEC | 0.5 g to 10 g |
| Dextrose | 0 g to 100 g |
| Maltodextrin | 0 g to 100 g |
| EDTA (e.g., disodium edetate) | 5 mg to 200 mg |
| Citric Acid | 10 mg to 1 g |
| Citrate (e.g., sodium citrate) | 10 mg to 2 g |
| Polysorbate 80 (e.g., Tween 80) | 5 mg to 100 mg |
| Flavoring Agent | optional |
| Sweetener | optional |
| Preservative | optional |
| Water | q.s. to 100 mL |

**Table 11: Fluticasone Propionate Composition #2**

| **Ingredient** | **Amount** |
|---|---|
| Fluticasone Propionate | 0.5 mg to 150 mg |
| CMC, MCC, Carbomer, HPMC and/or HEC | 0 g to 10 g |
| Dextrose | 1 g to 100 g |
| Maltodextrin | 0 g to 100 g |
| EDTA (e.g., disodium edetate) | 5 mg to 200 mg |
| Citric Acid | 10 mg to 1 g |
| Citrate (e.g., sodium citrate) | 10 mg to 2 g |
| Polysorbate 80 (e.g., Tween 80) | 5 mg to 100 mg |
| Flavoring Agent | optional |
| Sweetener | optional |
| Preservative | optional |
| Water | q.s. to 100 mL |

**Table 12: Fluticasone Propionate Composition #3**

| **Ingredient** | **Amount** |
|---|---|
| Fluticasone Propionate | 0.5 mg to 150 mg |
| CMC, MCC, Carbomer, HPMC and/or HEC | 0 g to 10 g |
| Dextrose | 0 g to 100 g |
| Maltodextrin | 1 g 100 g |
| EDTA (e.g., disodium edetate) | 5 mg to 200 mg |
| Citric Acid | 10 mg to 1 g |
| Citrate (e.g., sodium citrate) | 10 mg to 2 g |
| Polysorbate 80 (e.g., Tween 80) | 5 mg to 100 mg |
| Flavoring Agent | optional |
| Sweetener | optional |
| Preservative | optional |
| Water | q.s. to 100 mL |

**Table 13: Corticosteroid Composition**

| **Ingredient** | **Amount % w/w** |
|---|---|
| Corticosteroid | 0.001 to 1 |
| Sodium methylparaben | 0.0001 to 0.1 |
| Sorbitol | 5 to 30 |
| Sucrose | 1 to 40 |
| Corn starch | 1 to 10 |
| MCC | 0.1 to 5 |
| CMC (NaCMC) | 0.1 to 5 |
| Xanthan | 0.001 to 1 |
| Glycerin | 0.1 to 10 |
| Calcium carbonate | 0 to 30 |
| Magnesium hydroxide | 0 to 5 |
| Color (e.g., FD&C Red No. 3) | optional |
| Water | q.s. to 1, 2, 3, 4. 5, 6, 7, 8, 9, 10, 11, 12, 13, 14, or 15 mL |

The pharmaceutical composition is chemically and physically stable. In certain embodiments chemical stability is evidenced by a pharmaceutical composition that comprises at least 80%, 90%, 95%, 98%, or 99% of the initial amount or label amount of corticosteroid therein for, by way of non-limiting example, 1 week, 2 weeks, 3 weeks, 1 month, 3 months, 6 months, 1 year, 2 years, or for the duration of the shelf life. In some embodiments, physical stability is evidenced by a pharmaceutical composition that is able to substantially obtain uniformity, remain substantially uniform (e.g., for at least 1 day, 3 days, 1 week, 2 weeks, 3 weeks, 1 month, 3 months, 6 months, 1 year, 2 years, etc.), or substantially regain uniformity (e.g., via mild or moderate agitation after being undisturbed for 1 day, 3 days, 1 week, 2 weeks, 3 weeks, 1 month, 3 months, 6 months, 1 year, 2 years, etc.). In certain embodiments, physical stability is evidenced by a composition that comprises at least 80%, 90%, 95%, 98%, or 99% of the initial amount or label amount of corticosteroid and/or optional additional active agent therein for, by way of non-limiting example, 2 days, 1 week, 2 weeks, 3 weeks, 1 month, 3 months, 6 months, 1 year, 2 years, or for the duration of the shelf life. In certain embodiments, uniformity as described herein is evidenced by the uniformity of the dispersion of the corticosteroid particles throughout the pharmaceutical composition, the uniformity of the dispersed mass of
corticosteroid throughout the pharmaceutical composition, the uniformity of the concentration of one or more of the components in the composition throughout the pharmaceutical composition, and the like. In certain embodiments, mild or moderate agitation includes, by way of non-limiting example, shaking, shaking well, swirling, gentle swirling, and the like. In some embodiments, mild or moderate agitation includes agitation without a special apparatus. In some embodiments, uniformity of the pharmaceutical composition refers to dose uniformity (e.g., each dose delivered or withdrawn from the composition comprises a substantially similar amount of corticosteroid), or the concentration of corticosteroid in at least some or all of the doses from the multiple dose formulations are substantially similar. In some embodiments, substantially similar includes, e.g., within 20%, 15%, 10%, 7%, 5%, 3%, 2%, or 1%.

In some embodiments, a pharmaceutical composition described herein comprises a corticosteroid that is readily dispersed throughout the composition upon agitation (e.g., mild or moderate). In certain embodiments, a pharmaceutical composition described herein comprises a corticosteroid that is readily dispersed throughout the composition upon mild or moderate agitation after storage. In some embodiments, a pharmaceutical composition described herein comprises a corticosteroid that is easily resuspended in the composition upon mild or moderate agitation. In certain embodiments, a pharmaceutical composition described herein comprises a corticosteroid that is easily resuspended in the composition upon mild or moderate agitation after storage. In some embodiments, a pharmaceutical composition described herein comprises a corticosteroid that is readily dispersed throughout the composition upon mild or moderate agitation. In certain embodiments, a pharmaceutical composition described herein comprises a corticosteroid that is readily dispersed throughout the composition upon mild or moderate agitation after storage. In certain embodiments, the ability of the compositions described herein are able to be easily resuspended and/or readily redispersed after storage under ambient conditions. In other embodiments, the storage is under an inert atmosphere, increased temperature and/or increased relative humidity. In certain embodiments, the ability of the compositions described herein are able to be easily resuspended and/or readily redispersed after storage for, by way of non-limiting example, 1 day, 2 days, 3 days, 1 week, 2 weeks, 3 weeks, 1 month, 3 months, 6 months, 1 year, 2 years, or the time of the shelf life.

Any pharmaceutical composition described herein optionally comprises a small amount of glycine, e.g., an amount that does not result in a substantial change in viscosity of the composition.

In some embodiments, a composition described herein is retained on the esophagus, or some portion thereof, after oral administration for at least 6 seconds, for at least 12 seconds, for at least 15 seconds, for at least 30 seconds, for at least 60 seconds, for at least 90 seconds, for at least 120 seconds, for at least 3 minutes, for at least 4 minutes, for at least 5 minutes, for at least 15 minutes, or for at least 30
minutes. The composition may be retained on the esophagus after oral administration for about 15 seconds to about 120 seconds, or for about 30 to about 90 seconds.

In certain embodiments, a composition described has a viscosity sufficient to deliver an effective amount of the composition to the site of gastrointestinal inflammation, e.g., the esophagus. In some embodiments, the effective amount of the composition delivered to the esophagus is an amount sufficient to coat, or partially coat, the esophagus, and deliver the composition to the affected areas, including by way of example only, the lower esophagus, the esophageal-stomach juncture, the stomach and/or the duodenum. In certain embodiments, the viscosity of the oral dosage form is such that when administered orally, it is not so thick as to cause difficulty in swallowing, cause gagging, or be unpalatable. In certain embodiments, the viscosity of the oral dosage form is a viscosity that is sufficient to provide exposure of the corticosteroid to the esophagus for a sufficient period of time such that the symptoms of and/or inflammation associated with inflammatory diseases involving the gastrointestinal tract, including the esophagus, are reduced following administration of the corticosteroid containing oral dosage form. The corresponding SI-unit of cP (centipoise) is mPa·s.

Viscosity may be, for example, measured at room temperature, at about 20-25 degrees Celsius, or at about 37 degrees Celsius to mimic body temperature. In various embodiments of the present invention, the viscosity of the composition described herein is any viscosity suitable for delivery of the corticosteroid to the targeted and/or inflamed portion of the gastrointestinal tract. In some embodiments, the viscosity of the composition is at least 2 cP, at least 3 cP, at least 5 cP, at least 10 cP, at least 15 cP, at least 20 cP, at least 25 cP, at least 30 cP, at least 35 cP, at least 40 cP, at least 50 cP, at least 200 cP, or at least 225 cP. In some embodiments, the viscosity of the composition is at least 100 cP. In certain embodiments, the viscosity of the composition, measured at 25 degrees Celsius, is 50 cP to 250,000 cP, about 50 cP to about 70,000 cP, about 50 cP to about 25,000 cP, about 50 cP to about 10,000 cP, about 50 cP to about 3,000 cP,
or about 50 cP to about 2,000 cP. In one aspect, the viscosity of the composition, as measured at 25 degrees Celsius, is from about 25 centipoise (cP) to about 800 cP, about 50 cP to about 800, or about 300 cP to about 800 cP (e.g., measured by a Brookfield viscometer). In another aspect, the viscosity of the composition may range from about 100 cP to about 200 cP, about 200 cP to about 300 cP, about 250 cP to about 600 cP or about 400 cP to about 600 cP. In specific embodiments, the viscosity of the formulation is about 30 cP,
about 100 cP, about 200 cP, about 300 cP, about 400 cP, about 500 cP, or about 250,000 cP (e.g., as measured with a Brookfield viscometer at 25 degrees Celsius equipped with an ultra low adapter).

The viscosity of the composition may be measured at room temperature (about 25 degrees C) with a shear rate of about 13.2 sec⁻¹. In certain embodiments, provided herein is a composition having a viscosity under such conditions that is at least about 2 centipoise (cP), at least about 3 cP, at least about 5 cP, at least about 10 cP, at least about 15 cP, at least about 20 cP, at least about 25 cP, at least about 30 cP, at least about 35 cP, at least about 40 cP, at least about 50 cP, at least about 200 cP, at least about 225 cP, at least about 250 cP, at least about 300 cP, or at least about 400 cP. In some embodiments, the viscosity of the composition under such conditions is about 50 cP to about 250,000 cP, about 50 cP to about 70,000
cP, about 50 cP to about 25,000 cP, about 50 cP to about 10,000 cP, about 50 cP to about 3,000 cP, about 50 cP to about 2,000 cP, about 250 cP to about 250,000 cP, about 250 cP to about 70,000 cP, about 250 cP to about 25,000 cP, about 250 cP to about 10,000 cP, about 250 cP to about 3,000 cP, or about 250 cP to about 2,000 cP. In one aspect, the viscosity of the composition, as measured at 25 degrees Celsius, is from about
25 centipoise (cP) to about 800 cP, about 50 cP to about 800, or about 300 cP to about 800 cP (e.g.,
measured by a Brookfield viscometer). In another aspect, the viscosity of the composition under such conditions may range from about 100 cP to about 200 cP, about 200 cP to about 300 cP, about 250 cP to about 600 cP or about 400 cP to about 600 cP. In specific embodiments, the viscosity of the formulation
measured under such conditions is about 30 cP, about 40 cP, about 100 cP, about 200 cP, about 300 cP, about 400 cP, about 500 cP, or about 250,000 cP.

In some embodiments, the viscosity of the composition is measured at room temperature (about 25 degrees C) with a shear rate of about 15 sec⁻¹ (e.g., with a gap between the spindle and the sample chamber wall of about 6 mm or greater). In certain embodiments, provided herein is a composition having a viscosity under such conditions that is at least about 2 centipoise (cP), at least about 3 cP, at least about 5 cP, at least about 10 cP, at least about 15 cP, at least about 20 cP, at least about 25 cP, at least about 30 cP, at least about 50 cP, at least about 100 cP, at least about 150 centipoise (cP), at least about 160 cP, at least about 170 cP, at least about 180 cP, at least about 190 cP, or at least about 200 cP. In some embodiments, the viscosity of the composition under such conditions is about 150 cP to about 250,000 cP, 160 cP to about 250,000 cP, 170 cP to about 250,000 cP, 180 cP to about 250,000 cP, or 190 cP to about 250,000 cP.

Viscosity can also be determined by any method that will measure the resistance to shear offered by the substance or preparation. Many viscometers are available to those in the pharmaceutical field, and include those built by, for example, Brookfield.

In some embodiments, a composition or formulation described herein comprises a viscosity enhancing agent that imparts on the composition a viscosity sufficient to provide increased residence on the esophagus while also allowing migration of the active agent(s) (solute or particles) when the composition is orally administered to an individual. In other words, in some embodiments, the viscosity is high enough to increase residence time of the composition on a surface of the gastrointestinal tract (e.g., the mucosa and/or epithelium of the gastrointestinal tract or of a specific site of the gastrointestinal tract, such as the esophagus), but not so high as to prevent migration of the active agent(s) within the composition, e.g., toward the surface of the gastrointestinal tract.

An increase in the interaction of the composition with the surface of the gastrointestinal tract (e.g., esophagus) may be measured by measuring the retention time of the material along a length of a gastrointestinal surface, wherein the retention time is increased in the presence of the excipients as compared to its absence. As used herein, in certain embodiments, a gastrointestinal surface includes a gastrointestinal mucosa and/or a gastrointestinal epithelium, all of which terms are used interchangeably herein. In another embodiment, an increased interaction may be measured by the decrease in physiological manifestations or symptoms of the disease or ailment to be treated, including a decrease in total eosinophil counts in a target sample.

In certain embodiments, at least 50%, at least 30%, at least 25%, at least 20%, at least 15%, at least 10%, at least 9%, at least 8%, at least 7%, at least 6%, at least 5%, at least 4%, at least 3%, at least 2%, or at least 1% of the corticosteroid or composition described herein adheres to or resides upon a surface of the gastrointestinal tract (e.g., the esophagus), or some portion thereof, at least 5 seconds, at least 6 seconds, at least 10 seconds, at least 12 seconds, at least 15 seconds, at least 30 seconds, at least 60 seconds, at least 90 seconds, at least 120 seconds, at least 3 minutes, at least 4 minutes, at least 5 minutes, at least 15 minutes, or at least 30 minutes after oral administration (e.g., drinking or swallowing). In certain embodiments, at least 50%, at least 30%, at least 25%, at least 20%, at least 15%, at least 10%, at least 9%, at least 8%, at least 7%, at least 6%, at least 5%, at least 4%, at least 3%, at least 2%, or at least 1% of the corticosteroid or composition adheres to, resides upon, or is retained on the esophagus after oral administration for about 15 seconds to about 120 seconds, or for about 30 to about 90 seconds. In some embodiments, a portion of the composition comprises about 90% or more, about 80% or more, about 70% or more, about 60% or more, about 50% or more, about 40% or more, about 30% or more, about 20% or more, about 10% or more, or about 5% or more.

Adherence and/or absorption of a pharmaceutical composition or corticosteroid described herein to a gastrointestinal mucosal site (e.g., esophagus) may be determined in any suitable manner, e.g., by scintigraphy or by an assay. In some cases, such determinations are performed *in vivo* or *in vitro.* In certain embodiments, *in vivo* scintigraphy may include combining a pharmaceutical composition described herein with a detectable radioisotope, administering the labeled composition to a subject and detecting and/or measuring the adherence or residence of the pharmaceutical composition or corticosteroid to the gastrointestinal surface (e.g., esophagus) with a device (e.g., camera) that detects and/or measures radioactivity. In some cases, *in vivo* scintigraphy may include linking a corticosteroid described herein with a detectable radioisotope, formulating the labeled corticosteroid into a composition described herein, administering the composition to a subject and detecting and/or measuring the adherence or residence of the pharmaceutical composition or corticosteroid to the gastrointestinal surface (e.g., esophagus) with a device (e.g., camera) that detects and/or measures radioactivity. An *in vitro* assay for detecting adherence of a pharmaceutical composition or corticosteroid described herein to a gastrointestinal mucosal site (e.g., esophagus) may include applying a composition described herein to a distal portion of a strip of gastrointestinal mucosal tissue (e.g., porcine esophageal tissue) and subjecting the composition to a flow of artificial saliva in the direction of the opposite distal portion of the strip. Determination of adherence or residence of the composition and/or corticosteroid may be determined at a given time by detecting either the amount of composition and/or corticosteroid eluted or the amount of composition and/or corticosteroid remaining on the gastrointestinal surface (e.g., esophagus).

In certain embodiments, a composition described has a viscosity sufficient to deliver an effective amount of the composition to the site of gastrointestinal affliction, e.g., the esophagus. In some embodiments, the effective amount of the composition delivered to the esophagus is an amount sufficient to coat, or partially coat, a surface of the gastrointestinal tract, and deliver the composition to the affected areas, including by way of example only, the esophagus, the lower esophagus, the esophageal-stomach juncture, the stomach and/or the duodenum. In certain embodiments, the viscosity of the oral dosage form is such that when administered orally, it is not so thick as to cause difficulty in swallowing, cause gagging, and/or be unpalatable. In certain embodiments, the viscosity of the oral dosage form is a viscosity that is sufficient to provide exposure of the therapeutic agent to the esophagus for a sufficient period of time such that the disorder or symptoms of the disorder involving the gastrointestinal tract, including the esophagus, are reduced following administration of a pharmaceutical composition described herein formulated in or as an oral dosage form. In some instances wherein the pharmaceutical composition is in solid or semi-solid form, the composition described has a viscosity, when mixed with saliva or water (e.g., in certain instances wherein the composition is formulated as an effervescent tablet, sachet or home brew), the resulting composition has a viscosity sufficient to deliver an effective amount of a composition described herein to the site of gastrointestinal inflammation, e.g., the esophagus.

In certain embodiments, a pharmaceutical composition described herein is a non-Newtonian fluid or a Newtonian fluid. In some embodiments, a pharmaceutical composition described herein is a non-Newtonian fluid. In specific embodiments, the non-Newtonian fluid is a plastic, pseudo-plastic or dilatant non-Newtonian fluid. In some specific embodiments, the non-Newtonian fluid is thixotropic. In certain embodiments, the non-Newtonian fluid composition thins with shear, and thickens upon the absence of shear. Thus, in some embodiments, provided herein is a fluid pharmaceutical composition that is suitable for easy pouring following mild or moderate agitation. Furthermore, in some embodiments, provided herein is a fluid pharmaceutical composition that while being suitable for easy pouring following mild or moderate agitation becomes viscous enough upon oral administration to allow the pharmaceutical composition to at least partially coat the esophagus and topically deliver a therapeutically effective amount of corticosteroid to the esophagus. In some embodiments, the at least one additional excipient is selected from a non-Newtonian viscosity enhancing agent (i.e., an agent that provides a composition herein with a non-Newtonian character). Non-Newtonian viscosity enhancing agents include, by way of non-limiting example, acacia (e.g., used in about 5-10% w/w of a pharmaceutical composition described herein), alginic acid (e.g., about 0.5-20% w/w), carbomer, CaCMC, NaCMC, carrageenan (e.g., about 0.3-12% w/w), ceratonia (e.g., about 0.1-1% w/w), chitosin (e.g., about 0.5-2% w/w), colloidal silicon dioxide (e.g., about 2-10% w/w), ethylcellulose (e.g., about 5-25% w/w), gelatin, guar gum (e.g., about 1-2.5% w/w), HEC, hydroxyethylmethyl cellulose (e.g., about 1-5% w/w), hydroxypropyl cellulose (e.g., about 1-10% w/w), HPMC, magnesium aluminum silicate (e.g., about 2-10% w/w), one or more maltodextrin, methylcellulose (e.g., about 1-2% w/w), polyethylene glycol (e.g., about 45-60% w/w), povidone (e.g., about 10-15% w/w), saponite, sodium alginate (e.g., about 1-5% w/w), sucrose (e.g., about 50-70% w/w), tragacanth (e.g., about 0.1-2% w/w), xanthan gum (e.g., about 0.1-1% w/w), an combinations thereof.

A Newtonian fluid can be described as a fluid whose viscosity is equal to the shear stress exerted by the fluid divided by the velocity gradient perpendicular to the direction of the shear. In certain embodiments, the at least one additional excipient is selected from a Newtonian viscosity enhancing agent (i.e., an agent that provides a composition herein with a Newtonian character). Newtonian viscosity enhancing agents include, by way of non-limiting example, glycerin (e.g., about 50-80% w/w), polydextrose (e.g., about 50-70% w/w), and combinations thereof.

In certain embodiments, following administration of a composition described herein to a gastrointestinal surface (e.g., of a surface of the upper gastrointestinal tract, or of the esophagus), at least 1%, 2%, 3%, 4%, 5%, 6%, 7%, 8%, 9%, 10%, 15%, 20%, 30%, 40%, 50%, 60%, 70%, 80%, 90% or 95% by weight of the corticosteroid or composition administered adheres to and/or is absorbed at a gastrointestinal surface (e.g., of a surface of the upper gastrointestinal tract, or of the esophagus) after at least 0.08, 0.17, 0.25, 0.5, 0.75, 1, 1.5, 2, 3, 4, 5, 6, 7, 8, 9, 10, 15, 20, 25, 30, 35, 40, 45, 50, 55 or 60 minutes following application of the composition to the gastrointestinal surface (e.g., of a surface of the upper gastrointestinal tract, or of the esophagus). In specific embodiments, the gastrointestinal surface is the site of gastrointestinal inflammation. In specific embodiments, following oral administration of a composition described herein to the esophagus (e.g., following initial swallowing or drinking of the composition), at least 1%, 2%, 3%, 4%, 5%, 6%, 7%, 8%, 9%, 10%, 15%, 20%, 30%, 40%, 50%, 60%, 70%, 80%, 90% or 95% by weight of the corticosteroid or composition administered is present within the esophagus (e.g., as measured by gamma scintigraphy) after at least 5 seconds, 10 seconds, 15 seconds, 20 seconds, 25 seconds, 30 seconds, 40 seconds, 45 seconds, 50 seconds, or 1, 1.5, 2, 3, 4, 5, 6, 7, 8, 9, 10, 15, 20, 25, 30, 35, 40, 45, 50, 55 or 60 minutes following application of the composition to the esophagus. In certain instances, even small differences (e.g., increases) in adherence times (e.g., residence times) between formulations can result in therapeutically significant or clinically significant results or improvements.

In some embodiments, a pharmaceutical composition described herein is sufficiently spreadable and/or has an appropriate flow characteristic on a gastrointestinal surface (e.g., on a surface of the upper gastrointestinal tract, such as the esophagus). In certain embodiments, the spreadability and/or flow characteristic of the composition is suitable so as to allow a pharmaceutical composition or a unit dose of a pharmaceutical composition described herein to spread across and/or flow upon the gastrointestinal surface and at least partially coat the gastrointestinal surface. In some embodiments, by at least partially coating the gastrointestinal surface, topical delivery of the corticosteroid to the gastrointestinal site is achieved.

In certain embodiments, the pharmaceutical compositions provided herein are used to treat, prevent or alleviate inflammation or symptoms associated with inflammation involving the gastrointestinal tract, including the esophagus, stomach and/or digestive tract. In specific embodiments, the pharmaceutical composition is in liquid form. Liquid forms include, by way of non-limiting example, solutions, suspensions, syrups, slurries, dispersions, colloids and the like. In specific embodiments, the liquid is a suspension. In some embodiments, a pharmaceutical composition described herein is in liquid, semi-solid or solid form. In specific embodiments, a pharmaceutical composition described herein is in semi-solid form, e.g., a gel, a gel matrix, a cream, a paste, or the like. In some embodiments, semi-solid forms comprise a liquid vehicle.

The compositions of the present invention are used by individuals of any age. By "individual" is meant any animal, for example, a mammal, or, for example, a human, including, for example, patients in need of treatment. In some embodiments, the individual is a human adult. In other embodiments, the individual is a human child or infant. In certain embodiments, the human child or infant is less than 16 years old, less than 12 years old, less than 8 years old, less than 6 years old, less than 4 years old or less than 2 years old.

### Formulations

While any of the pharmaceutical compositions, methods and kits described herein are typically used in therapy for human patients, in certain embodiments, they are used in veterinary medicine to treat similar or identical diseases. In some embodiments, the compositions are used, for example, to treat mammals, including, but not limited to, primates and domesticated mammals. In some embodiments, the compositions, methods and kits are used, for example, to treat herbivores. The compositions of the present invention include geometric and optical isomers.

Pharmaceutical compositions suitable for use in the present invention include compositions wherein the active ingredient or ingredients are contained in an effective amount to achieve its intended purpose. In certain embodiments, the pharmaceutical compositions disclosed herein comprise corticosteroid in an amount sufficient to treat, prevent or alleviate esophageal inflammation.

In certain embodiments, the exact dosage of corticosteroid depends upon, by way of non-limiting example, the form in which the composition is administered, the subject to be treated, the age, body weight and/or height of the subject to be treated, and/or the preference and experience of the attending physician. Thus, in some embodiments, the dosage of corticosteroid administered may vary from those disclosed herein. In certain embodiments, the optimal concentration of the corticosteroid in the composition depends upon, by way of non-limiting example, the specific corticosteroid used, the characteristics of the patient, and/or the nature of the inflammation for which the treatment is sought. In various embodiments, these factors are determined by those of skill in the medical and pharmaceutical arts in view of the present disclosure.

Generally, a therapeutically effective dose is desired. A therapeutically effective dose refers to the amount of the corticosteroid that results in a degree of amelioration of symptoms and/or inflammation relative to the status of such symptoms and/or inflammation prior to treatment. The dosage forms and methods of applying dosage forms containing effective amounts are within the scope of the instant invention. In various embodiments, the amount of corticosteroid (e.g., budesonide or fluticasone propionate) used in a method or in a composition described herein is from about 2.5 to 400 µg/kg of body weight per day, or for example, in the range of 5 to 300 µg/kg per day, or for example in the range of 5 to 200 µg/kg per day, or for example in the range of 5 to 100 µg/kg per day, or for example in the range of 10 to 100 µg/kg per day, or for example in the range of 10-50 µg/kg per day, or for example in the range of 10-100 µg/kg/day, or for example in the range of 5-50 µg/kg/day, or in an illustrative embodiment in the range of 10-60 µg/kg/day.

In an illustrative embodiment, a dosage or amount (including a divided dose) of corticosteroid is provided in a composition of sufficient volume to allow any of the compositions disclosed herein to reach the targeted and/or inflamed portion of the gastrointestinal tract, including, e.g., the esophagus, in an effective amount. In some embodiments, the effective amount of the composition delivered to the esophagus is an amount sufficient to coat or at least partially coat the esophagus, and deliver the composition to the affected areas, including by way of example only, the lower esophagus, the esophageal-stomach juncture, the stomach and/or the duodenum. In certain embodiments, a composition described herein as a volume of, for example about 1-20 mL, or for example about 1-50 mL, or for example about 1-40 mL, or for example about 1-30 mL, or for example about 1-25 mL, or for example about 5-25 mL, or for example about 10-20 mL, or for example about 10 mL, or for example, about 15 mL, or for example, about 20 mL, or for example about 1-15 mL, or for example about 1-10 mL, or for example about 2-8 mL, or for example about 3-7 mL, or for example, about 4-6 mL, or for example, about 5 mL, or for example about 6-14 mL, or for example about 8-12 mL, or for example, about 9-11 mL, or for example, about 10 mL. In more specific embodiments, about 0.05 mg to about 20 mg, about 0.05 mg to about 10 mg, about 0.1 mg to about 10 mg, 0.25 mg to about 6 mg, about 0.25 mg, about 0.375 mg, about 0.5 mg, about 0.75 mg, about 1 mg, about 1.25 mg, about 1.5 mg, or about 2 mg of corticosteroid (e.g., budesonide) is formulated into a single or unit dose of a pharmaceutical composition described herein, the single or unit dose having a total volume of about 10-20 mL, or for example about 10 mL, or for example, about 15 mL, or for example, about 20 mL, or for example about 1-15 mL, or for example about 1-10 mL, or for example about 2-8 mL, or for example about 3-7 mL, or for example, about 4-6 mL, or for example, about 5 mL, or for example about 6-14 mL, or for example about 8-12 mL, or for example, about 9-11 mL, or for example, about 10 mL. As discussed herein, "liquid" encompasses slurries, solutions, suspensions, dispersions or any combination thereof, depending on the solubilities and amounts of the individual components and the vehicles and solvents used. In some embodiments, an appropriate palatable dosage is in a volume sufficient to coat or at least partially coat the esophagus, and in an illustrative embodiment, the volume is sufficient to coat or at least partially coat the esophagus and deliver the corticosteroid to the affected areas, including by way of example only, the lower esophagus, the esophageal-stomach juncture, the stomach and/or the duodenum. The composition may be delivered, for example, four times a day, three times a day, twice a day, once a day, every other day, three times a week, twice a week, or once a week. The dosage may, for example, be divided into multiple doses throughout the day, or be provided, for example, in four, three, two, or one dose a day. In certain instances, administration more frequent administration (e.g., b.i.d. versus once a day) provides for a shorter overall therapy or a quicker onset of symptom resolution. In one illustrative example, the dose is provided once a day.

In certain illustrative embodiments, a dose or composition described herein is administered with food. In other illustrative embodiments, a dose or composition described herein is administered without food. In other illustrative embodiments, a dose or composition described herein is administered in a fed or fasted state. In some embodiments, a dose or composition described herein is administered in the morning, in the afternoon, in the evening, at night, or a combination thereof. In some embodiments, the dose is administered at night. In another aspect, the dose is administered about 30 minutes prior to bed, with no food or water given after administration of the compositions herein. In yet another embodiment of the instant invention, the dose is administered prior to bedtime, wherein after administration of the composition, the patient or individual is in a substantially supine position for at least 30 minutes, at least 1 hour, at least 2 hours, at least 4 hours or at least 8 hours.

In some embodiments, provided herein are methods, expressed as products for use in such methods, of treating, preventing, or alleviating
inflammation or symptoms associated with inflammation of the gastrointestinal tract, e.g., the esophagus, comprising administering to an individual in need thereof a single unit dose of a pharmaceutical composition described herein from a multidose container. In specific embodiments, administering a single unit dose from a multi dose container comprises (1) shaking a multidose container, the multidose container comprising at least one unit dose of a pharmaceutical composition described herein; (2) pouring (or otherwise dispensing) a single unit dose from the multidose container into an administration device (e.g., a device suitable for administering to a human individual, such as a spoon, cup or syringe); and (3) administering the single unit dose to the individual in need thereof. In more specific embodiments, shaking of the multidose container occurs until the fluid therein has a viscosity suitable for pouring (e.g., easy pouring). In some specific illustrative embodiments, the process further comprises waiting after pouring the single unit dose and prior to administering the single unit dose to the individual in need thereof. In specific situations, the wait time is a time sufficient to allow the viscosity of composition to achieve a desired level, e.g., a viscosity to improve the coating capabilities of the composition. The wait time may be, e.g., about 3 seconds, or more; about 5 seconds, or more; about 10 seconds, or more; about 15 seconds, or more; about 20 seconds, or more; about 25 seconds, or more; about 30 seconds, or more; about 40 seconds, or more; about 45 seconds, or more; about 50 seconds, or more; or about 60 seconds, or more. The composition may be administered immediately following pouring the composition into the administration device.
The process may comprise shaking the multidose container well.

In some illustrative embodiments, initial treatment continues, for example, for about 3 days to 2 weeks for an
acute condition, or about 4 weeks to about 16 weeks for a chronic condition, or about 8 weeks to about 12 weeks for a chronic condition. In various situations, longer therapy is needed, such as, for example, therapy similar to chronic therapy for persistent asthma. In some cases, patients are, for example, be treated for up to 6 months, or up to one year. In certain cases, maintenance treatments last up to or longer than one year. In some cases, patients are treated on a maintenance basis or on an as needed basis during a problematic episode, depending on the severity of the condition. In certain other cases, patients are treated on a rotating treatment basis, where treatment is provided for a period of time and then the patient is taken off of the drug for a period before treatment resumes again. When off the drug, the patient may be given no treatment, treatment with another medication, dietary therapy, or treatment with a reduced dosage. In certain situations, patients are given treatment with a higher dose of the composition until a desired reduced disease state is achieved, and then continued on a lower dose of the composition. In certain cases, a patient combines treatment with a composition described herein with a treatment with another medication, and/or dietary therapy. In certain cases, patients are given treatment with a higher dose of the composition until a desired reduced disease state is achieved, and then continued on a lower dose of the composition.

In some embodiments, methods of treatment described herein include intermittent or continuous treatments. In certain embodiments, a method of treating gastrointestinal inflammation described herein includes prophylactic treatment of gastrointestinal inflammation (e.g., a treatment that prevents symptoms and/or inflammation from occurring). In some embodiments, a method of treating gastrointestinal inflammation described herein includes a method of prolonging and/or maintaining remission of gastrointestinal inflammation by administering or continuing to administer a pharmaceutical composition as described herein after inflammation and/or symptoms of inflammation are in remission. In specific embodiments, prophylactic and/or remissive therapies optionally comprise administration of a composition described herein comprising a reduced amount of corticosteroid compared to the amount of corticosteroid utilized when the inflammation and/or symptoms of inflammation are not in remission.

In some embodiments, provided herein is a method of diagnosing an individual with gastrointestinal inflammation (e.g., EoE) by administering a pharmaceutical composition described herein; and determining the efficacy of such a treatment. In certain instances, the individual is a patient who has gastrointestinal inflammation and/or symptoms thereof that are refractory to at least one acid inhibitor (e.g., PPI and/or H2A). In some embodiments, effective treatment of the gastrointestinal inflammation with a composition described herein is a positive indication of EoE. In certain embodiments, this method of diagnosis is used instead of an esophageal biopsy.

In some embodiments, the corticosteroid is present in a pharmaceutical composition described herein in any effective amount. In some embodiments, an effective amount is an amount sufficient to reduce inflammation or symptoms of inflammation associated with an inflammatory disease or condition of the gastrointestinal tract (e.g., the esophagus) as compared to the level of inflammation or symptoms of inflammation associated with an inflammatory disease prior to administration of the effective amount. In certain embodiments, effective amount is an amount sufficient to maintain a reduction in inflammation or symptoms of inflammation achieved in any manner including, but not limited to, by the administration of an effective amount sufficient to achieve such a reduction. In some embodiments, the effective amount is about 50 µg to about 500 mg, about 50 µg to about 200 mg, about 50 µg to about 100 mg, about 50 µg to about 50 mg, about 250 µg to about 20 mg, about 250 µg to about 15 mg, about 250 µg to about 10 mg, about 0.05 mg to about 20 mg, about 0.1 mg to about 20 mg, about 0.05 mg to about 15 mg, about 0.05 mg to about 10 mg, about 0.05 mg to about 7.5 mg, about 0.05 mg to about 5 mg, about 0.3 mg to about 4 mg, about 0.3 mg to about 2 mg, about 0.25 mg to about 3 mg, about 0.25 mg to about 2.5 mg, about 0.5 mg to about 3 mg, about 0.5 mg to about 2 mg, about 0.5 mg to about 0.1 mg, about 0.5 mg to about 5 mg, about 0.5 mg to about 4 mg, about 1 mg to about 4 mg, about 1 mg to about 3 mg, about 2 mg to about 3 mg, or about 2 mg to about 4 mg. In specific embodiments, the effective amount of corticosteroid is about 0.05 mg, about 0.1 mg., about 0.15 mg., about 0.25 mg., about 0.3 mg., about 0.35 mg, about 0.4 mg, about 0.37 mg, about 0.375 mg, about 0.7 mg, about 0.8 mg, about 0.75 mg, about 1 mg, about 1.2 mg, about 1.25 mg, about 1.3 mg, about 1.5 mg, about 2 mg, about 2.5 mg, about 3 mg, about 3.5 mg, about 4 mg, about 4.5 mg, about 5 mg, about 5.5 mg, about 6 mg, about 6.5 mg, about 7 mg, or about 7.5 mg or more. In certain embodiments, the corticosteroid is present in a pharmaceutical composition at a concentration of about 0.01 mg/mL to about 2 mg/mL of composition. In specific embodiments, the corticosteroid is present in a pharmaceutical composition at a concentration of about 0.01 mg/mL to about 1.5 mg/mL, about 0.02 mg/mL to about 1.5 mg/mL, about 0.04 mg/mL to about 1.5 mg/mL, about 0.03 mg/mL to about 1.5 mg/mL, about 0.05 mg/mL to about 1.5 mg/mL, or about 0.07 mg/mL to about 1.5 mg/mL. In more specific embodiments, the corticosteroid is present in a pharmaceutical composition at a concentration of about 0.07 mg/mL to about 1 mg/mL.

Also disclosed herein is a composition comprising a corticosteroid (e.g., budesonide or fluticasone propionate), one or more excipient that increases the interaction of the composition with a surface of the gastrointestinal tract (e.g., an agent that enhances viscosity, mucoadhesive character, adsorption to a surface of the gastrointestinal tract, and/or absorption of an active through a surface of the gastrointestinal tract), optionally one or more binder, optionally one or more filler, optionally one or more lubricant, optionally one or more solvent (or vehicle), optionally one or more suspension agent, optionally one or more flavoring agent, optionally one or more coloring agent, optionally one or more sweetener, optionally one or more preservative, optionally one or more antioxidant, optionally one or more buffering agent, optionally one or more humectant, optionally one or more chelating agent, and optionally one or more surfactant. In specific embodiments, the composition described herein is a composition comprising a
corticosteroid, dextrose, maltodextrin, edetate, citrate, polysorbate 80, an optional preservative, an optional flavoring agent, an optional sweetener, at least one additional excipient, and a liquid vehicle. The composition of the invention comprises a preservative. In further or alternative embodiments, the composition comprises a flavoring agent. In further or alternative embodiments, the liquid vehicle is an aqueous medium (e.g., water). In specific embodiments, corticosteroid particles (e.g., microparticles) are suspended in the aqueous medium.

In some embodiments, the corticosteroid is selected from, by way of non-limiting example, budesonide, fluticasone propionate and combinations thereof. In specific embodiments, corticosteroid is present in the composition in an amount of 0.01 mg/mL to 3 mg/mL, 0.01 mg/mL to 2 mg/mL, 0.01 mg/ mL to 1.5 mg/mL, 0.07 mg/mL to 1.5 mg/mL, 0.07 mg/mL to 1 mg/mL. In illustrative embodiments, budesonide is present in an amount of about 0.01 mg/mL to about 3 mg/mL, about 0.01 mg/mL to about 1.5 mg/mL, or about 0.07 mg/mL to about 1 mg/mL.
In other illustrative embodiments, fluticasone propionate is present in an amount of about 0.005 mg/mL to about 1.5 mg/mL, or about 0.01 mg/mL to about 1 mg/mL.

In some illustrative embodiments, the volume of a composition or dose of a composition described herein is an
amount sufficient to substantially coat (e.g., at least 50%, at least 60%, at least 70%, at least 80%, at least 90%, at least 95%, at least 98% or at least 99% of) the length of the esophagus of an individual to whom the composition is administered. In certain situations, the volume of a composition or a dose of a composition described herein is about 0.05 mL/cm esophageal length to about 1 mL/cm esophageal length, about 0.1 mL/cm esophageal length to about 0.8 mL/cm esophageal length, about 0.2 mL/cm esophageal length to about 0.6 mL/cm esophageal length, or about 0.3 mL/cm esophageal length to about 0.5 mL/cm esophageal length, wherein the esophageal length is the esophageal length of the individual to whom the composition is administered. The volume of a composition or dose of a composition
described herein may be
based on the esophageal length of an individual (e.g., male, female, or both) that is in the
50^{th} percentile of height for their age. Therefore, in some cases, the volume of a composition or dose of a composition described herein is about 0.05 mL/cm esophageal length to about 1 mL/cm esophageal length, about 0.1 mL/cm esophageal length to about 0.8 mL/cm esophageal length, about 0.2 mL/cm esophageal length to about 0.6 mL/cm esophageal length, about 0.3 mL/cm esophageal length to about 0.5 mL/cm esophageal length, about 0.32 mL/cm esophageal length to about 0.41 mL/cm esophageal length, or about 0.3 mL/cm esophageal length to about 0.46 mL/cm esophageal length, wherein the esophageal length is the esophageal length of an individual having a height in the 50^{th} percentile for the age of the individual to whom the composition is administered. In certain instances, esophageal length is the actual esophageal length of the individual or is calculated based on the equation: esophageal length = 1.048(cm) + (0.167*height(cm)). In certain instances, for example, the 50^{th} percentile height (CDC 2000) for male children age 2 is 87 cm, age 3 is 95 cm, age 4 is 102 cm, age 5 is 109 cm, age 6 is 115 cm, age 7 is 122 cm, age 8 is 128 cm, age 9 is 134 cm, age 10 is 139 cm, age 11 is 144 cm, age 12 is 149 cm, age 13 is 156 cm, age 14 is 164 cm, age 15 is 170 cm, age 16 is 174 cm, age 17 is 175 cm, and age 18 is 176 cm.

Furthermore, the amount of a therapeutic agent (a corticosteroid such
as budesonide) in a composition or a dose of a composition described herein may be about 0.005 mg/cm esophageal length to about 0.3 mg/cm esophageal length, about 0.008 mg/cm esophageal length to about 0.2 mg/cm esophageal length, about 0.01 mg/cm esophageal length to about 0.15 mg/cm esophageal length, or about 0.015 mg/cm esophageal length to about 0.1 mg/cm esophageal length, wherein the esophageal length is the esophageal length of the individual to whom the composition is administered. In some instances, the volume of a composition or dose of a composition described herein is based on the esophageal length of an individual (e.g., male, female, or both) that is in the 50^{th} percentile of height for their age. Therefore, in some illustrative embodiments, the amount of a therapeutic agent (e.g., a corticosteroid such as budesonide) in a composition or dose of a composition described herein is about 0.005 mg/cm esophageal length to about 0.3 mg/cm esophageal length, about 0.008 mg/cm esophageal length to about 0.2 mg/cm esophageal length, about 0.01 mg/cm esophageal length to about 0.15 mg/cm esophageal length, or about 0.015 mg/cm esophageal length to about 0.1 mg/cm esophageal length, wherein the esophageal length is the esophageal length of an individual having a height in the 50^{th} percentile for the age of the individual to whom the composition is administered.

In some embodiments,
any pharmaceutical composition or dose of a pharmaceutical composition
described herein is provided or administered in a volume sufficient to provide a bolus when orally administered to an individual. In certain embodiments, the composition has a volume that does not systemically deliver excessive amounts of the active agent. In some embodiments, the pharmaceutical composition or dose is provided in a volume sufficient to provide a bolus when administered to an individual, wherein the size of the bolus at the distal end of the esophagus (e.g., the size of the bolus prior, e.g., immediately prior, to entering or passing the lower esophageal sphincter) is less than 90%, less than 85%, less than 80%, less than 75%, less than 70%, less than 65%, less than 60%, less than 55%, less than 50%, less than 45%, less than 40%, less than 35%, less than 30%, less than 25%, less than 20%, less than 15%, less than 10% or less than 5% of size of the bolus that entered the esophagus (e.g., the size of the bolus after, e.g., immediately after, passing the upper esophageal sphincter). In some embodiments, the size of the bolus is determined as a measure of diameter or of volume. In certain embodiments, diameter of the sphincter can be determined using gamma scintigraphy techniques. In specific embodiments, the volume of the composition or dose is adjusted given the length and/or diameter of the esophagus of the individual to whom the composition or dose is administered.

In some embodiments, the at least one additional excipient comprises or is HPMC. In illustrative embodiments, HPMC is present in the composition in an amount of about 1 mg/mL to about 100 mg/mL, about 30 mg/mL to about 70 mg/mL or about 5 mg/mL to about 50 mg/mL. In some embodiments, the at least one additional excipient comprises or is CMC. In specific embodiments, CMC is present in the composition in an amount of 1 mg/mL to 100 mg/mL, 30 mg/mL to 70 mg/mL or 5 mg/mL to 50 mg/mL. In some embodiments, the at least one additional excipient comprises
or is MCC. In specific embodiments, MCC is present in the composition in an amount of 1 mg/mL to 100 mg/mL, 30 mg/mL to 70 mg/mL or 5 mg/mL to 50 mg/mL. In certain
embodiments, the at least one additional excipient comprises or is carbomer. In illustrative embodiments, carbomer is present in the composition in an amount of about 2 mg/mL to about 250 mg/mL, or about 5 mg/mL to about 100 mg/mL. In some embodiments, the at least one additional excipient comprises or is HEC. In illustrative
embodiments, HEC is present in the composition in an amount of about 1 mg/mL to about
100 mg/mL, about 30 mg/mL to about 70 mg/mL or about 5 mg/mL to about 50 mg/mL. In some embodiments, the at least one additional excipient comprises or is a combination of CMC and MCC (e.g., Avicel® RC-591). In specific embodiments, the CMC/MCC combination (e.g., Avicel® RC-591) is present in the composition in an amount of 1 mg/mL to 150 mg/mL, 1 mg/mL to 75 mg/mL, or 5 mg/mL to 40 mg/mL. In certain embodiments, the CMC/MCC mixed weight ratio is between 1/99 and 99/1, 20/80 and 5/95, 15/85 and 10/90. In a specific embodiment, the CMC is NaCMC and the CMC/MCC mixed weight ratio is 11/89.

In illustrative embodiments, dextrose is present in the composition in an amount of about 10 mg/mL to about 1 g/mL. In some embodiments, maltodextrin is present in the composition in an amount of 10 mg/mL to 1.5 g/mL.
In certain embodiments, edetate is present in a composition in an amount of 0.02 mg/mL to 5 mg/mL, 0.02 mg/mL to 2 mg/mL, 0.05 mg/mL to 2 mg/mL. In some embodiments, citrate is present in the composition in an amount of 0.1 mg/mL to 50 mg/mL, 0.02 mg/mL to 30 mg/mL. In certain embodiments, polysorbate 80 is present in the composition in an amount of 0.01 mg/mL to 10 mg/mL, 0.05 mg/mL to 1 mg/mL, or 0.05 mg/mL to 0.5 mg/mL.

In certain embodiments, amounts of component per mL refers to the amount of component in relation to the amount of total (or q.s.) volume of the composition as a whole, rather than the volume of any liquid component, such as water, alone. In specific embodiments, provided herein are compositions comprising the ingredients in about the amounts as set forth in any one of Examples 1-22, e.g., Example 19, Example 20, Example 21, or Example 22.

In certain embodiments, the compositions described herein further comprise excipients and/or auxiliaries suitable for enabling the compositions to be formulated tablets, pills, dragees, capsules, liquids, soft chews, creams, pastes, chewable tablets, gels or gel matrices, syrups, slurries, suspensions, gums, lozenges, and the like, for oral ingestion by a patient to be treated. In certain instances, oral formulations (e.g., suspensions, creams or gel matrices) are formulated such that upon oral administration, an interface layer between the oral formulation (e.g., suspension, cream or gel matrix) and a surface of the gastrointestinal tract (e.g., a mucosa or epithelium of the gastrointestinal tract) is formed. In some instances, an oral formulation (e.g., a suspension, cream or gel matrix) in contact with a surface of the gastrointestinal tract delivers a corticosteroid to the site of the gastrointestinal tract contacted with the formulation via the interface layer and as the oral formulations (e.g., suspensions, creams or gel matrices) near the interface layer is depleted of corticosteroid, a concentration gradient results. In certain instances, portions of the oral formulations (e.g., suspensions, creams or gel matrices) with high concentrations of corticosteroid relative to the portions of the oral formulations (e.g., suspensions, creams or gel matrices) proximate to the interface layer replenishes corticosteroid in the portion of the oral formulations (e.g., suspensions, creams or gel matrices) proximate to the interface layer. In certain instances, upon oral administration of an oral formulation described herein to an individual, an interface layer is formed between a mucosal membrane and a mixture of the oral formulation (e.g., chewable tablet) and saliva of the individual.

In certain embodiments, the pharmaceutical compositions described herein optionally comprise water. In certain embodiments, the pharmaceutical compositions described herein comprise water as a vehicle. In some instances, the vehicle is a combination of water and alcohol. In other instances, the vehicle is a non-aqueous liquid vehicle.

In some embodiments, a pharmaceutical composition or dosage form described herein is a suspension comprising a corticosteroid (e.g., budesonide). In some embodiments, compositions (e.g., suspensions) comprise a certain concentration of corticosteroid (e.g., budesonide) that is dissolved in the liquid medium (e.g., the solvent or liquid vehicle used, such as water, alcohol, aqueous alcohol, or the like). In certain illustrative
embodiments, the amount of corticosteroid (e.g., budesonide) dissolved in the liquid medium (e.g., in an equilibrated sample) is greater than 4 µg/mL, greater than 5 µg/mL, greater than 10 µg/mL, greater than 15 µg/mL, greater than 20 µg/mL, greater than 21 µg/mL, greater than 22 µg/mL, greater than 23 µg/mL, greater than 24 µg/mL, greater than 25 µg/mL, about 25 µg/mL, greater than 30 µg/mL, about 25 µg/mL to about 80 µg/mL, about 30 µg/mL to about 80 µg/mL, about 30 µg/mL, about 35 µg/mL, about 40 µg/mL, about 45 µg/mL, about 50 µg/mL, about 55 µg/mL, about 60 µg/mL, about 65 µg/mL, or about 70 µg/mL. In some embodiments, the corticosteroid (e.g., budesonide) is present in the composition in combination with maltodextrin. In more specific embodiments, the maltodextrin has a DE of 4-7, or 13-17. In some embodiments, an acyclic oligosaccharide (e.g., maltodextrin) provided in a composition described herein is used as a solubility enhancing agent of the corticosteroid (e.g., budesonide). As used herein, an "acyclic oligosaccharide" refers to an oligosaccharide or polysaccharide that does not form a macrocycle (e.g., a starch conversion product such as maltodextrin or an aqueous soluble corn syrup, or a glycan, such as hyaluronic acid); individual saccharide monomeric units of the oligosaccharide may be cyclic. In some embodiments, compositions described herein comprise a corticosteroid (e.g., budesonide), a carboxymethyl cellulose and/or microcrystalline cellulose (e.g., Avicel® RC-591), and a liquid medium (e.g., water). In specific embodiments, compositions described herein comprise a corticosteroid (e.g., budesonide), a carboxymethyl cellulose and/or microcrystalline cellulose (e.g., Avicel® RC-591), maltodextrin, and a liquid medium (e.g., water). In illustrative embodiments, the composition comprises greater than 4 µg/mL, greater than 5 µg/mL, greater than 10 µg/mL, greater than 15 µg/mL, greater than 20 µg/mL, or the like of budesonide dissolved in the liquid medium (e.g., water).

In certain embodiments,
a solubility inhibitor is optionally utilized in a formulation herein in order
to inhibit solvation or dissolution of corticosteroid. In some instances, inhibition of the solvation or dissolution of the corticosteroid allows the corticosteroid to retain chemical stability in the composition for an extended period of time (compared to a similar composition having a greater concentration of dissolved corticosteroid). In some embodiments, solubility inhibitors include, by way of non-limiting example, disaccharides and monosaccharides (e.g., dextrose). In certain embodiments, wherein an excipient that increases the interaction of the composition or corticosteroid with a surface of the gastrointestinal tract also increases solubility of corticosteroid in the vehicle of the composition (e.g., an aqueous medium), a solubility inhibitor is utilized to reduce or inhibit dissolution or solvation of the corticosteroid (e.g., such solvation or dissolution that is aided by the use of the excipient that increases the interaction of the composition or corticosteroid with the surface of the gastrointestinal tract).

In some embodiments, compositions (e.g., suspensions) comprise a certain concentration of budesonide that is dissolved in the liquid medium (e.g., the solvent or liquid vehicle used, such as water, alcohol, aqueous alcohol, or the like). In specific embodiments, the amount of R epimer of the dissolved budesonide (compared to the overall weight of the budesonide) is greater than 28% w/w, greater than 30% w/w, greater than 39% w/w, greater than 40%, about 39-50%, about 40-50%, less than 38% w/w, about 29%-37% w/w, less than 27% w/w, or the like. In some instances, the % epimers are obtained in a composition having an overall % R epimer (compared to overall budesonide) of about 50-55% w/w, or about 53-54% w/w. In some embodiments, maltodextrin (e.g., maltodextrin comprising a DE of about 4-7 or about 13-17) is utilized as a solubility enhancer herein. In further embodiments, maltodextrin is a solubility enhancing agent that selectively enhances the solubility of the R epimer over the S epimer (e.g., enhances the solubility of the R epimer to a greater extent than the S epimer) of budesonide. In other embodiments, maltodextrin is a solubility enhancing agent that selectively enhances the solubility of the S epimer over the R epimer (e.g., enhances the solubility of the R epimer to a greater extent than the S epimer) of budesonide. In certain instances, the R epimer of budesonide is less stable than the S epimer. In certain embodiments, compositions described herein are formulated to adjust the amount of R and/or S epimer of budesonide dissolved in the aqueous medium so as to increase or maximize stability (e.g., chemical stability) of the composition. In some instances, the concentration of corticosteroid is the concentration measured of an equilibrated sample. In certain instances, equilibration of the sample is accomplished once the concentration of the corticosteroid (e.g., budesonide) dissolved in the liquid is substantially stable, e.g., after 2 days, 3 days, 4 days, 5 days, a week, a month, or the like. In specific instances, equilibration of the sample is accomplished after 2 days.

In certain embodiments, the compositions provided herein are prepared utilizing any suitable source of active agents. In some embodiments, corticosteroid (e.g., budesonide) used in the compositions described herein are neat corticosteroid (e.g., budesonide). In some embodiments, the neat corticosteroid (e.g., budesonide) is neat, bulk corticosteroid. In certain embodiments, the neat corticosteroid (e.g., budesonide) is powder corticosteroid (e.g., budesonide). In specific embodiments, the neat corticosteroid (e.g., budesonide) is micronized corticosteroid (e.g., budesonide).

In some embodiments, the corticosteroid is administered in a commercially available formulation. In other embodiments, the corticosteroid is administered in a composition comprising a commercially available formulation of a corticosteroid and formulated as described herein. For example, in some embodiments, the corticosteroid containing composition provided herein comprises a commercially available formulation and an excipient, such as a diluents, a flavoring agent, a mucoadhesive agent, a viscosity enhancing agent, a binder, a filler, a lubricant, a solvent, a suspension agent, a coloring agent, a sweetener, a preservative, an antioxidant, a buffering agent, a humectant, a chelating agent, a surfactant, combinations thereof, or the like. In some embodiments, wherein the corticosteroid is budesonide, the commercially available formulation is Pulmicort Respules® (distributed by AstraZeneca, e.g., as set forth in NDA 20-929). In other embodiments, wherein the corticosteroid is budesonide, the commercially available formulation is Rhinocort Aqua® (distributed by AstraZeneca LP, Wilmington, DE 19850, e.g., as set forth in NDA 20-746, which is, including all supplements ). In still other embodiments, wherein the corticosteroid is budesonide, the commercially available formulation is Symbicort® (manufactured by AstraZeneca Dunkerque Production, Dunkerque, France, e.g., as set forth in NDA 21-929, which is, including all supplements). In some embodiments, wherein the corticosteroid is fluticasone, the commercially available formulation is Flonase®. In some embodiments, the ratio of commercially available formulation to the optional diluent is between about 1:0.5 and about 1:100. Diluents include any pharmaceutically acceptable oral diluent including, e.g., powder diluents (such as talc) and liquid diluents (such as water, ethanol and combinations thereof). In certain embodiments, the commercially available formulation is Entocort® (manufactured by AstraZeneca AB, S-151 85 Sodertalje, Sweden, distributed by Prometheus Laboratories Inc, San Diego, CA 92121, as set forth in NDA 21-324, which is, including all supplements). In certain embodiments, Entocort® formulations are dissolved and/or dispersed in an aqueous vehicle. In specific embodiments, the Entocort® formulation is dispersed in a liquid vehicle that has a pH sufficient to remove the enteric coating from the budesonide particles. In other embodiments, the Entocort® formulation is pre- treated with a solvent having a pH sufficient to remove the enteric coating from the budesonide particles therein, and the particles are subsequently formulated into a composition described herein.

In certain embodiments, a corticosteroid composition described herein comprises a corticosteroid, a commercially available formulation, and, optionally, one or more additional excipient. In some embodiments, a corticosteroid composition described herein comprises a corticosteroid formulated in a manner similar to a commercial formulation (e.g., lacking one or more of the active ingredients of the formulation), and, optionally, one or more additional excipient. The one or more additional excipients can be utilized to achieve a formulation as described herein. In specific embodiments, the commercially available formulation is Ultra XCID (manufactured by Matrixx Initiatives, Inc., Phoenix, AZ).

In some embodiments, the corticosteroid is budesonide and the composition comprises about 0.01 mg to about 1.0 mg of budesonide/g of composition. In some embodiments, the composition comprises about 0.1 mg to about 1.0 mg of budesonide/g of composition. In certain embodiments, the composition comprises about 0.3 mg to about 0.6 mg of budesonide/g of composition. In specific embodiments, the composition comprises about 0.4 mg or 0.44 mg of budesonide/g of composition. In certain specific embodiments, the composition comprises about 3.8 mg/8.6 g of composition. In some embodiments, the composition comprises about 0.01 mg to about 1.0 mg of budesonide/mL of composition (about 0.001 to about 0.1% w/w). In some embodiments, the composition comprises about 0.1 mg to about 1.0 mg of budesonide/mL of composition (about 0.01 to about 0.1% w/w). In certain embodiments, the composition comprises about 0.3 mg to about 0.8 mg of budesonide/mL of composition (about 0.03 to about 0.08% w/w). In specific embodiments, the composition comprises about 0.6 to about 0.7 mg of budesonide/mL of composition (about 0.06 to about 0.07% w/w). In more specific embodiments, the composition comprises about 0.63 mg of budesonide/mL of composition (about 0.063% w/w).

Also described herein is a corticosteroid containing composition comprising budesonide, a viscosity enhancing agent, a preservative, an antioxidant (including, e.g., a chelating agent), an isotonic agent, a surfactant, an aqueous vehicle, an optional pH adjusting agent and an optional sweetener. In certain embodiments, the composition is a liquid. In specific embodiments, the liquid composition is a suspension.

In certain embodiments, a viscosity enhancing agent is present in e.g. 0.005% to about 3.0% w/w of the composition. In certain embodiments, a viscosity enhancing agent may be
present in about 0.1% to about
3.0% w/w of the composition. In specific embodiments, a viscosity enhancing agent is a combination of microcrystalline cellulose and carboxymethyl cellulose (e.g., carboxymethyl cellulose sodium), such as Avicel RC-591. In more specific embodiments, the combination of MCC and CMC are present in an amount of about 0.05% to about 2.5% w/w (or about 0.5% to about 2.5% w/w) of the composition. In some embodiments, a viscosity enhancing agent is selected from, by way of non-limiting example, xanthan gum at about 0.03% to about 3% w/w (or about 0.3% to about 3% w/w), carbomer at about 0.01% to about 2% w/w (or about 0.1% to about 2% w/w), guar gum at about 0.03% to about 2% w/w (or about 0.3% to about 2% w/w), or HPMC at about 0.05% to about 3.0% w/w (or about 0.5% to about 3.0% w/w) of the composition. In some embodiments, one or more additional viscosity enhancing agents are added so as to provide a viscosity as described herein. Alternatively, the amount of an aforementioned viscosity enhancing agent present in the composition is increased so as to provide a viscosity as described herein. In some embodiments, the CMC/MCC combination (e.g., Avicel® RC-591) is present in the composition in an amount of about 1 mg/mL to about 150 mg/mL, 1 mg/mL to about 75 mg/mL, or about 5 mg/mL to about 40 mg/mL. In certain embodiments, the CMC/MCC mixed weight ratio is between about 1/99 and about 99/1, about 20/80 and about 5/95, or about 15/85 and about 10/90. In a specific embodiment, the CMC is NaCMC and the CMC/MCC mixed weight ratio is 11/89.

In some embodiments, surfactants include, by way of non-limiting example, polysorbates (e.g., polysorbate 80), poloxamers, polyoxyethylene alkyl ethers, polyoxyethylene castor oil derivatives and combinations thereof. In certain embodiments, surfactants are present in an amount of about 0.0005% to about 2% w/w (or about 0.005% to about 2% w/w) of the composition. In a specific embodiment, the surfactant is polysorbate 80. In specific embodiments, polysorbates are present in an amount of about 0.0005% to about 2% w/w (or about 0.005% to about 2% w/w) of the composition. In some embodiments, poloxamers are present in an amount of about 0.001% to about 2% w/w (or about 0.01% to about 2% w/w) of the composition, polyoxyethylene alkyl ethers are present in an amount of about 0.001 % to about 1% w/w (or about 0.01% to about 1% w/w) of the composition, and/or polyoxyethylene castor oil derivatives are present in an amount of about 0.001% to about 1% w/w (or 0.01% to about 1% w/w) of the composition.

In certain embodiments, an isotonic agent includes, by way of non-limiting example, dextrose, glycerin, mannitol, sodium chloride, potassium chloride and combinations thereof. In specific embodiments, the isotonic agent is dextrose (e.g., dextrose anhydrous). In certain embodiments, the isotonic agent is included in any suitable amount, such as, by way of non-limiting example, between about 0.5 mg and about 0.5 g per gram of composition. In specific embodiments, the isotonic agent is included in an amount of about 10 mg and about 100 mg or about 40 mg to about 60 mg per gram of composition.

In some embodiments, antioxidants include, by way of non-limiting example, disodium edetate (EDTA). In certain embodiments, the antioxidant is present in an amount of about 0.0005% to about 0.1% w/w (or about 0.005% to about 0.1% w/w) of the composition.

Preservatives include, by way of non- limiting example, benzalkonium chloride, methylparaben, propylparaben, potassium sorbate and sodium benzoate. In specific embodiments, the preservative is potassium sorbate. In some embodiments, the preservative is present in an amount of about 0.0002% to about 0.5% w/w (or about 0.002% to about 0.5% w/w) of the composition. In specific embodiments, benzalkonium chloride is present in an amount of about 0.0002% to about 0.02% w/w (or about 0.002% to about 0.02% w/w) of the composition, methylparaben is present in an amount of about 0.005% to about 0.25% w/w (or about 0.05% to about 0.25% w/w) of the composition, propylparaben is present in an amount of about 0.001% to about 0.2% w/w (or about 0.01 % to about 0.2% w/w) of the composition, potassium sorbate is present in an amount of about 0.005% to about 2.0% w/w (or about 0.05% to about 2.0% w/w) of the composition, and/or sodium benzoate is present in an amount of about 0.01% to about 0.5% w/w (or about 0.1% to about 0.5% w/w) of the composition.

Sweeteners include, by way of non-limiting example, sucralose, sucrose, lactose, glucose, fructose, arabinose, xylose, ribose, mannose, galactose, dextrose, sorbose, sorbitol, mannitol, maltose, cellobiose, xylitol, honey and the like. In general, when utilized, the sweetener is utilized in an amount sufficient to at least partially mask the taste of the composition and/or the corticosteroid (e.g., budesonide).

In some embodiments, the corticosteroid containing composition comprises micronized budesonide, microcrystalline cellulose, carboxymethyl cellulose sodium, dextrose anhydrous, polysorbate 80, disodium edetate, potassium sorbate, water, optionally hydrochloric acid and optionally one or more additional excipient. In specific embodiments, the composition has a pH of about 4.5. In a specific embodiment, at least one of the optional excipients is a sweetener, a flavoring agent, or a combination thereof.

In specific embodiments, the composition administered comprises a micronized suspension of budesonide in an aqueous medium. In some specific embodiments, microcrystalline cellulose and carboxymethyl cellulose sodium, dextrose anhydrous, polysorbate 80, disodium edetate, potassium sorbate, and purified water are contained in the aqueous medium. In more specific embodiments, hydrochloric acid is added to adjust the pH to a target of about 4.5. In certain embodiments, the compositions provided herein are prepared utilizing any suitable source of active agents. In some embodiments, corticosteroid (e.g., budesonide) used in the compositions described herein are neat corticosteroid (e.g., budesonide). In some embodiments, the neat corticosteroid (e.g., budesonide) is neat, bulk corticosteroid. In certain embodiments, the neat corticosteroid (e.g., budesonide) is powder corticosteroid (e.g., budesonide). In specific embodiments, the neat corticosteroid (e.g., budesonide) is micronized corticosteroid (e.g., budesonide). In an exemplary embodiment, the composition comprises Rhinocort Aqua® (manufactured by AstraZeneca; Rhinocort Aqua® Nasal Spray; 32 meg of budesonide per spray and 120 metered sprays after initial priming; package insert 30516-00, Rev. 01/05), an optional diluent, and an optional sweetener. In some embodiments, the diluent is any carrier suitable for oral administration, including, by way of non-limiting example, water, ethanol, and combinations thereof. In a specific embodiment, the diluent is water. In some embodiments, the composition comprises Rhinocort Aqua®, an additional viscosity enhancing agent (e.g., Splenda), an optional diluent, and an optional sweetener and/or flavoring agent. In some embodiments, a composition useful herein includes a composition comprising a composition described in U.S. 6,291,445, U.S. 6,686,346, or U.S. 6,986,904, an optional additional viscosity enhancing agent or "thickener", an optional diluent, and an optional sweetener and/or flavoring agent. In some embodiments, the composition comprises Rhinocort Aqua® and a diluent wherein the Rhinocort Aqua® and diluent are present in a ratio between about 1:0.5 and about 1:100. In more specific embodiments, the diluted Rhinocort Aqua® composition further comprises an excipient that increases the interaction of the composition with a surface of the gastrointestinal tract (e.g., a viscosity enhancing agent).

In some embodiments, the corticosteroid is selected from, by way of non-limiting example, fluticasone propionate. In specific embodiments, corticosteroid is present in the composition in an amount of about 0.01 mg/mL to about 3 mg/mL, about 0.01 mg/mL to about 2 mg/mL, about 0.01 mg/mL to about 1.5 mg/mL, about 0.07 mg/mL to about 1.5 mg/mL, or about 0.07 mg/mL to about 1 mg/mL. In more specific embodiments, fluticasone propionate is present in an amount of about 0.01 mg/mL to about 3 mg/mL, about 0.01 mg/mL to about 1.5 mg/mL, or about 0.07 mg/mL to about 1 mg/mL. In other specific embodiments, fluticasone propionate is present in an amount of about 0.005 mg/mL to about 1.5 mg/mL, or about 0.01 mg/mL to about 1 mg/mL. In some embodiments, the corticosteroid is fluticasone propionate and the composition comprises about 0.01 mg to about 1.0 mg of fluticasone propionate/g of composition. In some embodiments, the composition comprises about 0.1 mg to about 1.0 mg of fluticasone propionate/g of composition. In certain embodiments, the composition comprises about 0.3 mg to about 0.6 mg of fluticasone propionate/g of composition. In specific embodiments, the composition comprises about 0.5 mg of fluticasone propionate/g of composition.

In certain embodiments, the present invention provides for an oral liquid formulation comprising corticosteroid particles. In certain embodiments, the particles are, e.g., microparticles or nanoparticles. In some embodiments, the particles are selected so as to increase or maximize the corticosteroid surface area per unit volume of the pharmaceutical formulation when compared to an otherwise identical pharmaceutical formulation comprising larger particles. In certain embodiments, a particle is selected so as to increase surface area of the corticosteroid per unit volume of pharmaceutical formulation, increased dissolution rate of the corticosteroid and increase the absorption in the gastrointestinal tissue of the corticosteroid.

In one embodiment, the corticosteroid containing composition comprises fluticasone propionate, a preservative, an isotonic agent, a surfactant, an aqueous vehicle, an excipient, an optional pH adjusting agent (e.g., hydrochloric acid and/or sodium hydroxide) and an optional sweetener and/or fragrance. In certain embodiments, the excipient is microcrystalline cellulose (MCC). In other embodiments, the excipient is carboxymethyl cellulose (CMC). In some embodiments, the corticosteroid containing composition contains two or more excipients. For example, in some embodiments, the two or more excipients are a combination of microcrystalline cellulose (MCC) and carboxymethyl cellulose (CMC). In some embodiments, the CMC/MCC combination (e.g., Avicel® RC-591) is present in the composition in an amount of about 1 mg/mL to about 150 mg/mL, 1 mg/mL to about 75 mg/mL, or about 5 mg/mL to about 40 mg/mL. In certain embodiments, the CMC/MCC mixed weight ratio is between about 1/99 and about 99/1, about 20/80 and about 5/95, or about 15/85 and about 10/90. In a specific embodiment, the CMC is NaCMC and the CMC/MCC mixed weight ratio is about 11/89. In some embodiments, the excipient is an excipient that increases the interaction of the composition with a surface of the gastrointestinal tract (e.g., the mucosa and/or epithelium of the gastrointestinal tract or of a specific site of the gastrointestinal tract, such as the esophagus) (e.g., a viscosity enhancing agent). In certain embodiments, the composition is a liquid. In specific embodiments, the liquid composition is a suspension.

In certain embodiments, the excipient that increases the interaction of the composition with a surface of the gastrointestinal tract (e.g., the mucosa and/or epithelium of the gastrointestinal tract or of a specific site of the gastrointestinal tract, such as the esophagus) is a viscosity enhancing agent. In some embodiments, the viscosity enhancing agent is present in about 0.01% to about 10% w/w of the composition. In certain embodiments, a viscosity enhancing agent is present in about 0.01% to about 5.0% w/w, about 0.01% to about 3.0% w/w, or about 0.1% to about 5.0% w/w, about 0.1% to about 3.0% w/w of the composition. In specific embodiments, a viscosity enhancing agent is a combination of microcrystalline cellulose and carboxymethyl cellulose (e.g., carboxymethyl cellulose sodium). In another specific embodiment, the viscosity enhancing agent is carboxymethyl cellulose. In some embodiments, one or more additional excipient that increases the interaction of the composition with a surface of the gastrointestinal tract (e.g., one or more viscosity enhancing agent) is added so as to provide a viscosity as described herein. Alternatively, the amount of the excipient that increases the interaction of the composition with a surface of the gastrointestinal tract (e.g., the mucosa and/or epithelium of the gastrointestinal tract or of a specific site of the gastrointestinal tract, such as the esophagus) present in the composition is increased so as to provide a viscosity as described herein.

In some embodiments, surfactants include, by way of non-limiting example, polysorbates (e.g., polysorbate 80), poloxamers, polyoxyethylene alkyl ethers, polyoxyethylene castor oil derivatives and combinations thereof. In certain embodiments, surfactants are present in an amount of about 0.0001% to about 5% w/w, about 0.0001% to about 2% w/w or about 0.001% to about 2% w/w of the composition. In a specific embodiment, the surfactant is polysorbate 80.

In certain embodiments, an isotonic agent includes, by way of non-limiting example, dextrose, glycerin, mannitol, sodium chloride, potassium chloride and combinations thereof. In specific embodiments, the isotonic agent is dextrose (e.g., dextrose anhydrous). In certain embodiments, the isotonic agent is included in any suitable amount, such as, by way of non-limiting example, between about 0.5 mg and about 0.5 g per gram of composition. In specific embodiments, the isotonic agent is included in an amount of about 10 mg and about 100 mg or about 40 mg to about 60 mg per gram of composition.

Preservatives include, by way of non-limiting example, benzalkonium chloride, methylparaben, propylparaben, potassium sorbate, phenylethyl alcohol and sodium benzoate. In specific embodiments, the preservative is potassium sorbate. In some embodiments, the preservative is present in an amount of about 0.0002% to about 0.5% w/w (or about 0.002% to about 0.5% w/w) of the composition. In specific embodiments, benzalkonium chloride is present in an amount of about 0.0002% to about 0.03% w/w, about 0.002% to about 0.03% w/w, or about 0.02% w/w of the composition. In some embodiments, phenylethyl alcohol is present in an amount of about 0.005% to about 0.4% w/w, about 0.05 to about 0.3% w/w, or about 0.25% w/w of the composition. In specific embodiments, both benzalkonium chloride and phenylethyl alcohol are present.

Sweeteners include, by way of non-limiting example, sucralose, sucrose, lactose, glucose, fructose, arabinose, xylose, ribose, mannose, galactose, dextrose, sorbose, sorbitol, mannitol, maltose, cellobiose, xylitol, honey and the like. In general, when utilized, the sweetener is utilized in an amount sufficient to at least partially mask the taste of the composition and/or the corticosteroid (e.g., fluticasone propionate).

Fragrances include, by way of non-limiting example, phenylethyl alcohol. The fragrance can be included in any suitable amount including, by way of non-limiting example, about 0.005% to about 0.4% w/w, about 0.05 to about 0.3% w/w, or about 0.25% w/w of the composition.

In some embodiments, the corticosteroid containing composition comprises microfine fluticasone propionate, microcrystalline cellulose, carboxymethyl cellulose sodium, dextrose anhydrous, polysorbate 80, potassium sorbate, water, optionally hydrochloric acid and optionally one or more additional excipient. In specific embodiments, the composition has a pH of about 5 to about 7. In a specific embodiment, at least one of the optional excipients is a sweetener, a flavoring agent, or a combination thereof.

In specific embodiments, the composition administered comprises a suspension of microfine fluticasone propionate in an aqueous medium. In some specific embodiments, microcrystalline cellulose and carboxymethyl cellulose sodium, dextrose anhydrous, polysorbate 80, phenylethyl alcohol, benzalkonium chloride, and water are contained in the aqueous medium. In more specific embodiments the composition is adjusted to a target pH of about 5 to about 7. In certain embodiments, the compositions provided herein are prepared utilizing any suitable source of active agents, in some embodiments, corticosteroid (e.g., fluticasone propionate) used in the compositions described herein are neat corticosteroid (e.g., fluticasone propionate). In some embodiments, the neat corticosteroid (e.g., fluticasone propionate) is neat, bulk corticosteroid. In certain embodiments, the neat corticosteroid (e.g., fluticasone propionate) is powder corticosteroid (e.g., fluticasone propionate). In specific embodiments, the neat corticosteroid (e.g., fluticasone propionate) is micronized corticosteroid (e.g., fluticasone propionate). In an exemplary embodiment, the composition comprises Flonase® (manufactured by GlaxoSmithKline; Flonase® Nasal Spray; 50 mcg of fluticasone propionate per spray and 120 metered sprays after initial priming; Prescribing Information dated August 2007, © 2007, FLN: 1PI), an optional diluent, and an optional sweetener. In some embodiments, the diluent is any carrier suitable for oral administration, including, by way of non-limiting example, water, ethanol, and combinations thereof. In a specific embodiment, the diluent is water. In some embodiments, the composition comprises Flonase®, an additional viscosity enhancing agent (e.g., Splenda®), an optional diluent, and an optional sweetener and/or flavoring agent. In some embodiments, the composition comprises Flonase® and a diluent wherein the Flonase® and diluent are present in a ratio between about 1 :0.5 and about 1 : 100. In more specific embodiments, the diluted Flonase® composition further comprises an excipient that increases the interaction of the composition with a surface of the gastrointestinal tract (e.g., a viscosity enhancing agent).

In specific, non-limiting embodiments provided herein is a composition, or a method of administering a composition, comprising the components as set forth in either of Tables 14 or 15.

**Table 14**

| **Ingredient** | **Amount (% w/w)** |
|---|---|
| Fluticasone propionate, microfine | 0.05 |
| Dextrose | 5.0 |
| Polysorbate 80 | 0.02 |
| MCC and CMC | 1.0-2.0 |
| Benzalkonium chloride | 0.02 |
| Phenylethyl alcohol | 0.25 |
| Water | to 100 |

**Table 15**

| Ingredient | Amount (% w/w) |
|---|---|
| Fluticasone propionate, microfine | 0.01-0.1 |
| Dextrose | 4.0-6.0 |
| Polysorbate 80 | 0.001-5 |
| MCC and CMC | 0.1-10 |
| Benzalkonium chloride | 0.002-0.5 |
| Phenylethyl alcohol | 0.05-0.4 |
| Water | to 100 |

### Diseases

In some embodiments, provided herein are compositions for use in
treating, preventing, or alleviating
inflammation or symptoms associated with inflammation of the gastrointestinal tract, e.g., the esophagus. In specific embodiments, the method provided herein is a method of reducing or alleviating symptoms of inflammation of the gastrointestinal tract. In more specific embodiments, the inflammation of the gastrointestinal tract is eosinophilic esophagitis (EE or EoE). In some embodiments, the method provided herein is a method of treating inflammation associated with eosinophilic esophagitis (EE or EoE). In certain embodiments, the method provided herein is a method of treating dysphagia associated with eosinophilic esophagitis (EE or EoE). In some embodiments, the method provided herein is a method of treating inflammation and dysphagia associated with eosinophilic esophagitis (EE or EoE). In certain embodiments, provided herein are methods of treating diseases or conditions of the gastrointestinal tract (e.g., a disease or condition of the upper gastrointestinal tract, including a disease or condition of the esophagus), by administering a composition described herein.

In some embodiments, administration of the composition described herein treats, prevents, or alleviates inflammation or symptoms associated with the inflammatory disease or condition. Diseases or conditions of the gastrointestinal tract include, by way of non-limiting example, any chronic inflammatory or malignant state that involves the gastrointestinal tract (e.g., the upper gastrointestinal tract, esophagus, stomach and/or digestive tract) and responds to steroid therapy. In certain instances, the diseases or conditions treated by the compositions described herein include diseases or conditions of the upper gastrointestinal tract (including pre-colonic disease and disorders), the esophagus, the stomach, and/or the digestive tract. The composition of the present invention is useful, for example, for treating, preventing and alleviating esophageal inflammation associated with or symptoms of eosinophilic esophagitis, inflammatory bowel diseases involving the esophagus, Crohn's disease, celiac disease, proximal gastrointestinal pathology (e.g., in individuals suffering from hypofunctioning gallbladder), eosinophilic gastrointestinal inflammation, celiac disease, eosinophilic duodenitis, duodenal eosinophilia, functional dyspepsia, intermediate esophagitis, epithelial hyperplasia, basal cell hyperplasia, elongated papillae, dilated vessels in papillae, fungal esophagitis (e.g., Candida, turolopsis, histoplasma Aspergillus, etc.), viral esophagitis (e.g., HSV, CMV, V2V), bacterial esophagitis (e.g., tuberculosis, actinomycosis, syphlis), corrosive esophagitis, radiation esophagitis, chemotherapy esophagitis, graft vs. host disease, a skin disease with esophageal involvement (e.g., bullous pemphigoid, pemphigus vulgaris, epidermolysis bollosa, Stevens-Johnson syndrome), Behcet's disease, sarcoidosis, idiopathic esophagitis, eosinophilic gastritis, Menetrier's disease, parasitic gastritis, lymphocytic esophagitis, inflammatory bowel disease-associated esophagitis, , parasitic gastritis, acute esophageal inflammation secondary to caustic/irritant ingestion, persistent/recurrent esophageal strictures secondary to caustic/irritant, conditions due to ingestion, systemic diseases, congenital diseases, post-surgery inflammation, and gastro enteritis. The composition of the present invention is also useful, for example, for treating, preventing and alleviating esophageal inflammation associated with or symptoms of gastroesophageal reflux disease (GERD), nonerosive reflux disease (NERD), Barrett's Esophagus, and/or erosive esophagitis.

It will be appreciated that reference herein to treatment extends to prophylaxis as well as the treatment of inflammation or other symptoms.

In certain embodiments, provided herein is a composition for use in treating, preventing or alleviating inflammation of the gastrointestinal tract, including, by way of non-limiting example, the esophagus, stomach and/or digestive tract, in an individual comprising orally administering to said individual any of the compositions described herein. In certain embodiments, the oral dosage form comprises a liquid vehicle and is formulated as, e.g., a slurry, suspension, syrup, dispersion, solution, etc.

The patient is administered a corticosteroid such as, for example, budesonide or fluticasone propionate.

In some embodiments, the inflammation treated by the methods and compositions described herein is associated with eosinophilic inflammation and/or neutrophilic inflammation. In some embodiments, individuals (e.g., patients) to be treated with compositions described herein include those that have been diagnosed with eosinophilic esophagitis, an inflammatory bowel disease involving the esophagus, Crohn's disease, celiac disease, proximal gastrointestinal pathology (e.g., in individuals suffering from hypofunctioning gallbladder), eosinophilic gastrointestinal inflammation, celiac disease, eosinophilic duodenitis, duodenal eosinophilia, functional dyspepsia, intermediate esophagitis, epithelial hyperplasia, basal cell hyperplasia, elongated papillae, dilated vessels in papillae, fungal esophagitis (e.g., Candida, turolopsis, histoplasma Aspergillus, etc.), viral esophagitis (e.g., HSV, CMV, V2V), bacterial esophagitis
(e.g., tuberculosis, actinomycosis, syphlis), corrosive esophagitis, radiation esophagitis, chemotherapy esophagitis, graft vs. host disease, a skin disease with esophageal involvement (e.g., bullous pemphigoid, pemphigus vulgaris, epidermolysis bollosa, Stevens-Johnson syndrome), Behçet's disease, sarcoidosis, idiopathic esophagitis, eosinophilic gastritis, Menetrier's disease, parasitic gastritis, lymphocytic esophagitis, inflammatory bowel disease-associated esophagitis, , parasitic gastritis, esophageal inflammation secondary to caustic/irritant ingestion, persistent/recurrent esophageal strictures of any cause and including caustic/irritant ingestion, pill-induced esophagitis, systemic diseases, congenital diseases, post-surgery inflammation, or gastro enteritis. In one non-limiting example, the patient has eosinophilic esophagitis. In some embodiments, individuals (e.g., patients) to be treated with the compositions described herein include those that have been diagnosed with gastroesophageal reflux disease (GERD), nonerosive reflux disease (NERD), Barrett's Esophagus, and/or erosive esophagitis. In some embodiments, the patient is an adult. In other embodiments, the patient is a child or infant. In various aspects, a patient is a child or infant less than 16 years old, less than 12 years old, less than 8 years old, less than 6 years old, less than 4 years old or less than 2 years old.

In some embodiments, a composition is in a unit dose formulation for oral administration of a patient. In some embodiments, a unit dose of the corticosteroid is administered from a metered dose device. In some embodiments, the metered dose device delivers a metered unit dose of a composition described herein to the mouth or throat of an individual in need thereof. In certain embodiments, the metered dose device is a metered inhaler, which is utilized to administer a metered unit dose to the mouth or throat of an individual (the individual swallows rather than inhales the metered unit dose). In certain embodiments, a metered dose device dispenses a metered unit dose of a composition described herein into a receptacle (e.g., a cup), which is then utilized to orally administer the metered unit dose to the mouth or throat.

In some embodiments, provided herein is a multiple unit container comprising about 2 to about 180, about 10 to about 60, about 14, or about 30 unit doses of any pharmaceutical composition described herein. In more specific embodiments, each dose comprises about 1 mL to about 25 mL, about 1 mL to about 20 mL, about 7 mL to about 25 mL, about 10 to about 20 mL, about 15 mL, about 20 mL, about 3 to about 7 mL, about 5 mL, about 8 mL to about 12 mL, or about 10 mL. In still more specific embodiments, each dose comprises about 0.1 to about 20 mg, about 0.1 to about 10 mg, about 0.1 to about 7.5 mg, about 0.1 to about 5 mg, about 0.3 to about 4 mg, about 0.25 to about 2.5 mg, about 0.3 mg to about 2 mg, about 0.5 mg to about 1 mg, about 0.7 mg to about 1.5 mg, about 0.375 mg, about 0.75 mg, about 1 mg, about 1.25 mg, about 1.5 mg or about 2 mg of corticosteroid. In certain embodiments, provided herein is a multiple unit container comprising about 10 mL to about 1500 mL, about 50 mL to about 600 mL, about 150 mL, about 300 mL, about 600 mL, or about 1,200 mL of any pharmaceutical composition described herein. In specific embodiments, the multidose container comprises about 330 mL or about 55 mL of a composition described herein. In some embodiments, a kit provided herein comprises any multidose container as described herein, a pharmaceutical composition as described herein (e.g., in a volume described), and a delivery or metered device (e.g., a syringe, a cup, a spoon, or the like). In specific embodiments, the delivery device is incorporated into the container (e.g., an nebulizer, a aerosolizer, a pump, or the like). In certain
embodiments, the pharmaceutical composition contained within any of the multiple unit containers described herein is physically and chemically stable.

In certain aspects, about 0.1 mg to about 20 mg, about 0.25 mg to about 20 mg, about 0.25 mg to about 15 mg, about 0.25 mg to about 10 mg, or about 0.25 mg to about 5 mg (e.g., about 0.1 to about 5 mg, about 0.3 mg to about 4 mg, about 0.25 to about 2.5 mg, about 0.3 mg to about 2 mg, about 0.5 mg to about 1 mg, about 0.7 mg to about 1.5 mg, about 0.375 mg, about 0.75 mg, about 1 mg, about 1.25 mg, about 1.5 mg or about 2 mg) corticosteroid per day is administered to a patient. In some embodiments, the corticosteroid is present in a unit dose in an amount of between about 0.25 mg and about 5 mg. In some embodiments, the amount of corticosteroid administered daily or in a unit dose is between about 0.1 mg and about 20 mg. In some embodiments, the amount of corticosteroid administered daily or in a unit dose is between about 0.3 mg and about 4 mg. In certain embodiments, the amount of corticosteroid administered daily or in a unit dose is between about 0.5 mg and about 3 mg. In other embodiments, the amount of corticosteroid present in a unit dose or administered daily is between about 1 and about 3 mg, or between about 1 and about 2 mg, or between about 2 and about 3 mg.

In some embodiments, the dose or volume of a composition administered herein is adjusted based on the efficacy of treatment. In certain embodiments, a diagnosis of eosinophilic esophagitis (EE or EoE) is achieved by administering a composition described herein and determining the efficacy of the treatment. In certain embodiments, a composition described herein and separately determined to be effective in treating eosinophilic esophagitis (EE or EoE) is utilized. Efficacy of treatment can be determined in any suitable manner including, e.g., symptom score assessment, gastrointestinoscopy (e.g., esophagogastroduodenoscopy), gastrointestinal (e.g., esophageal) biopsy, histological evaluation, or a combination thereof. Processes of diagnosing eosinophilic esophagitis (EE or EoE) and/or determining efficacy of treatment include any suitable process including, by way of non-limiting example, processes as set forth in Aceves et al, J Allergy Clin Immunol, Feb. 2008; abstract 270, or Aceves et al., Am J Gastroenterol., Oct. 2007, 102(10):2271-9.

In some embodiments, a process for determining efficacy of a treatment (e.g., for eosinophilic esophagitis) described herein is a clinical symptom score assessment comprising (i) administering a composition described herein to an individual diagnosed with or suspected of having eosinophilic esophagitis; and (ii) evaluating one or more symptom of the individual. In certain embodiments, prior to administering the composition, the process comprises evaluating the one or more symptom of the individual. Symptoms that are optionally scored include, by way of non-limiting example, nausea, vomiting, pain, and heartburn. Total score or change in score is optionally utilized to diagnose a disorder and/or determine efficacy of treatment.

In certain embodiments, a process for determining efficacy of a treatment described herein comprises (i) administering a composition described herein to an individual diagnosed with or suspected of suffering from inflammation of the gastrointestinal tract (e.g., eosinophilic esophagitis); (ii) endoscoping the gastrointestinal surface of the individual; (iii) biopsying the gastrointestinal surface tissue; and (iv) evaluating the biopsied tissue and optionally determining an endoscopy score of the tissues biopsied. In specific embodiments, the process further comprises comparing the evaluated biopsied tissue and/or the
endoscopy score obtained prior to administration of the composition to the biopsied tissue and/or endoscopy score subsequent to administration of the composition.

In some embodiments, provided herein is a process of diagnosing an individual with gastrointestinal inflammation by (i) detecting and/or measuring symptoms of the individual prior to administering to the individual a composition described herein; (ii) administering to the individual any composition described herein; (iii) detecting and/or measuring symptoms of the individual following administration of the composition; and (iv) comparing the symptoms measured or detected prior to and following administration of a composition described herein. If the symptoms exhibited by the individual are reduced (e.g., by a statistically significant or clinically relevant amount), a positive diagnosis occurs. In specific embodiments, the process of diagnosing an individual with gastrointestinal inflammation is diagnosing an individual with eosinophilic esophagitis.

Provided in certain embodiments here is a method of treating, preventing or alleviating inflammation of the gastrointestinal tract comprising orally administering to an individual in need thereof a composition comprising a corticosteroid, a preservative, an antioxidant, an isotonic agent, a surfactant, and an excipient that increases the interaction of the composition with a mucosal layer. In some embodiments, the corticosteroid is, by way of non-limiting example, budesonide. In specific embodiments, the corticosteroid is present in the composition in an amount of about 0.01 mg to about 1.0 mg of corticosteroid per gram of composition. In some embodiments, the corticosteroid is present in the composition in an amount of about 0.01 mg to about 1.0 mg of corticosteroid per mL of composition.

In some embodiments, the preservative is, by way of non-limiting example, potassium sorbate. In specific embodiments, the preservative is present in the composition in an amount of about 0.0002% to about 0.5% w/w of the composition. In some embodiments, the antioxidant is, by way of non-limiting example, disodium edetate. In specific embodiments, the antioxidant is present in the composition in an amount of about 0.0005% to about 0.1% w/w of the composition. In certain embodiments, the isotonic agent is, by way of non-limiting example, dextrose. In some embodiments, the surfactant is, by way of non-limiting example, polysorbate 80. In specific embodiments, the surfactant is present in the composition in an amount of about 0.0005% to about 2% w/w of the composition. In certain embodiments, the excipient that increases the interaction of the composition with a mucosal layer is a viscosity enhancing agent. In specific embodiments, the viscosity enhancing agent is selected from, by way of non-limiting example, microcrystalline cellulose, carboxymethyl cellulose sodium and a combination thereof. In more specific embodiments, the viscosity enhancing agent is a combination of microcrystalline cellulose and carboxymethyl cellulose sodium. In some embodiments, the viscosity enhancing agent is present in the composition in about 0.01% to about 3.0% w/w of the composition. In certain embodiments, the viscosity enhancing agent is Avicel® RC-591.

In some embodiments, the composition has a first and a second excipient. In specific embodiments, the second excipient is selected from an excipient that increases the interaction of the composition with a mucosal layer, a binder, a filler, a disintegrant, a diluent, a carrier, a vehicle and combinations thereof. In other embodiments, the second excipient that increases the interaction of the composition with a mucosal layer is a second viscosity enhancing agent. In some embodiments, the second excipient is a vehicle (including a diluent) and is present in the composition in an amount of about 50% to about 99.5% w/w of the composition. In specific embodiments, the vehicle is selected from, by way of non-limiting example, a liquid vehicle, a solid vehicle and combinations thereof. In some embodiments, the vehicle is a solid vehicle and is selected from, by way of non-limiting example, talc, bentonite, kaolin calcium carbonate, and combinations thereof. In certain embodiments, the vehicle is a liquid vehicle and is selected from, by way of non-limiting example, water, ethanol, an organic solvent, an oil (e.g., corn oil) and combinations thereof. In specific embodiments, the corticosteroid containing composition is formulated as a micronized suspension of the corticosteroid in an aqueous vehicle.

In some embodiments, the corticosteroid containing composition has a target pH of about 4.5. In certain embodiments, the target pH of the composition is attained by adding a pH adjusting agent to the composition. In specific embodiments, the pH adjusting agent is, by way of non-limiting example, hydrochloric acid or sodium hydroxide.

In certain embodiments, the corticosteroid containing composition also contains, by way of non- limiting example, a sweetener, a flavoring agent or a combination thereof.

Also described herein is a corticosteroid containing composition containing budesonide as the corticosteroid, potassium sorbate as the preservative, disodium edetate as the antioxidant, dextrose as the isotonic agent, polysorbate 80 as the surfactant, a combination of microcrystalline cellulose and carboxymethyl cellulose sodium as the excipient that increases the interaction of the composition with a mucosal layer, and hydrochloric acid as the pH adjusting agent. In more specific embodiments, the corticosteroid containing composition contains about 0.01 mg to about 1.0 mg of budesonide per mL of composition, about 0.0002% to about 0.5% w/w of potassium sorbate, about 0.0005% to about 0.1% w/w of disodium edetate, about 0.0005% to about 2% w/w of polysorbate 80, and about 0.01% to about 3.0% w/w of a combination of microcrystalline cellulose and carboxymethyl cellulose sodium. In still more specific embodiments, the composition is further comprises an aqueous vehicle. In some embodiments, the corticosteroid containing composition is formulated as a micronized suspension of the budesonide in the aqueous vehicle. In yet more specific embodiments, the composition has a pH of about 4.5. In some embodiments, the composition comprises a second excipient that increases the interaction of the composition with a mucosal layer.

Also described herein is a method of treating, preventing or alleviating inflammation of the gastrointestinal tract comprising orally administering to an individual in need thereof a composition comprising a corticosteroid, a preservative, an isotonic agent, a surfactant, and an excipient.

In certain embodiments, the corticosteroid is, by way of non-limiting example, fluticasone propionate. In some embodiments, the corticosteroid is present in the composition in an amount of about 0.01 mg to about 1.0 mg of corticosteroid per gram of composition.

In some embodiments, the preservative is, by way of non-limiting example, benzalkonium chloride. In certain embodiments, the preservative is present in the composition in an amount of about 0.0002% to about 0.03% w/w of the composition.

In some embodiments, the isotonic agent is, by way of non-limiting example, dextrose. In certain embodiments, the surfactant is, by way of non-limiting example, polysorbate 80. In some embodiments, the excipient is an excipient that increases the interaction of the composition with a surface of the gastrointestinal tract (e.g., the mucosa and/or epithelium of the gastrointestinal tract or of a specific site of the gastrointestinal tract, such as the esophagus). In certain embodiments, the excipient that increases the interaction of the composition with a surface of the gastrointestinal tract (e.g., the mucosa and/or epithelium of the gastrointestinal tract or of a specific site of the gastrointestinal tract, such as the esophagus) is a viscosity enhancing agent. In some embodiments, the viscosity enhancing agent is selected from, by way of non-limiting example, microcrystalline cellulose, carboxymethyl cellulose sodium and a combination thereof. In certain specific embodiments, the viscosity enhancing agent is a combination of microcrystalline cellulose and carboxymethyl cellulose sodium. In other specific embodiments, the viscosity enhancing agent is carboxymethyl cellulose sodium. In some embodiments, a corticosteroid containing composition of the present invention comprises a second excipient. In certain embodiments, the second excipient is selected from, by way of non-limiting example, an excipient that increases the interaction of the composition with a surface of the gastrointestinal tract (e.g., the mucosa and/or epithelium of the gastrointestinal tract or of a specific site of the gastrointestinal tract, such as the esophagus), a binder, a filler, a disintegrant, a diluent, a carrier a vehicle and combinations thereof. In certain specific embodiments, the second excipient that increases the interaction of the composition with a surface of the gastrointestinal tract (e.g., the mucosa and/or epithelium of the gastrointestinal tract or of a specific site of the gastrointestinal tract, such as the esophagus) is a second viscosity enhancing agent.

In some embodiments, the composition further comprises a fragrance. In specific embodiments, the fragrance is, by way of non-limiting example, phenylethyl alcohol. In some embodiments, the fragrance is present in the composition in an amount of about 0.0005 to about 0.4% w/w of the composition.

In certain embodiments, the vehicle is present in the composition in an amount of about 50% to about 99.5% w/w of the composition. In some embodiments, the vehicle is selected from a liquid vehicle, a solid vehicle and combinations thereof. In certain embodiments, the solid vehicle is selected from, by way of non-limiting example, talc, bentonite, kaolin calcium carbonate, and combinations thereof. In some embodiments, the liquid vehicle is selected from, by way of non-limiting example, water, ethanol, an organic solvent, an oil and combinations thereof.

In some embodiments, the composition is formulated as a suspension of microfine corticosteroid particles suspended in an aqueous vehicle. In certain embodiments, the composition has a pH of about 5 to about 7.

In some embodiments, the composition further comprising a sweetener, a flavoring agent or a combination thereof.

In specific embodiments, the corticosteroid is microfine fluticasone propionate, the preservative is benzalkonium chloride, the isotonic agent is dextrose, the surfactant is polysorbate 80, the fragrance is phenylethyl alcohol, and the excipient that increases the interaction of the composition with a surface of the gastrointestinal tract (e.g., the mucosa and/or epithelium of the gastrointestinal tract or of a specific site of the gastrointestinal tract, such as the esophagus) is a combination of microcrystalline cellulose and carboxymethyl cellulose sodium. In some specific embodiments, the corticosteroid is microfine fluticasone propionate, the preservative is a combination of benzalkonium chloride and phenylethyl alcohol, the isotonic agent is dextrose, the surfactant is polysorbate 80, and the excipient that increases the interaction of the composition with a surface of the gastrointestinal tract (e.g., the mucosa and/or epithelium of the gastrointestinal tract or of a specific site of the gastrointestinal tract, such as the esophagus) is a combination of microcrystalline cellulose and carboxymethyl cellulose sodium. In more specific embodiments, the composition further comprises an aqueous vehicle. In even more specific embodiments, the composition is formulated as a suspension of microfine corticosteroid particles suspended in the aqueous vehicle. In some specific embodiments, the composition has a pH of about 5 to about 7. In certain embodiments, the composition further comprises a second excipient that increases the interaction of the composition with a surface of the gastrointestinal tract (e.g., the mucosa and/or epithelium of the gastrointestinal tract or of a specific site of the gastrointestinal tract, such as the esophagus).

In some embodiments, the benzalkonium chloride is present in the composition in an amount of about 0.0002% to about 0.03% w/w of the composition and the phenylethyl alcohol is present in the composition in an amount of about 0.0005 to about 0.4% w/w of the composition.

As discussed herein, compositions and formulations described comprise at least one corticosteroid (e.g., budesonide or fluticasone propionate). In some embodiments, a composition or formulation described herein further comprises at least one additional active agent. In specific embodiments, a composition or formulation described herein comprises a therapeutically effective amount of a corticosteroid and a therapeutically effective amount of at least one additional active agent. In some embodiments, the at least one additional active agent is an agent that treats, prevents, or alleviates the symptoms of and/or inflammation associated with inflammatory diseases involving the gastrointestinal tract (e.g., esophagus). It is to be understood that in certain instances, when the corticosteroid is combined with an additional active agent, the therapeutically effective amount of the corticosteroid is less than it when the additional active agent is absent.

Furthermore, provided herein are methods of preventing or alleviating gastrointestinal (e.g., esophageal) inflammation in an individual comprising orally administering to the individual a corticosteroid in association or combination with at least one additional active agent. In certain embodiments, the corticosteroid and the at least one additional active agent is in a single dosage form. In other embodiments, the corticosteroid and the at least one additional active agent are in separate dosage forms and are administered in any manner, including, by way of non-limiting example, simultaneously, sequentially, or at different times. For example, in certain embodiments, several doses of a corticosteroid composition are administered over a period of time, after which administration of the corticosteroid composition is discontinued and administration of at least one additional active agent is administered at least once.

In some embodiments, the at least one additional active agent utilized in a composition, formulation or method described herein is an agent that treats, prevents, or alleviates the symptoms of and/or inflammation associated with inflammatory diseases involving the gastrointestinal tract (e.g., esophagus). In more specific embodiments, the at least one additional active agent is not a second corticosteroid. In certain embodiments, the at least one additional active agent is an acid inhibitor (e.g., an H2 antagonist and/or a PPI). In certain embodiments, the at least one additional active agent is, by way of non-limiting example, a proton pump inhibitor (PPI), a histamine (e.g., HI, H2, and/or H3) receptor ligand (e.g., antagonist), a transient lower esophageal sphincter relaxation (TLESR)-reducing agent, a prokinetic serotonergic agent, a potassium-competitive acid blocker (P-CAB), a mucosal protectant, an anti-gastrin agent, a leukotriene antagonist, a mast cell inhibitor, a mast cell stabilizer, an immunomodulator, a biologic, an anti-asthmatic agent, a non-steroidal anti-inflammatory drug (NSAID), a chemotherapeutic, mGluR₅ antagonists, acetylcholine modulator, 5HT₄ receptor agonist, 5HT₃ receptor antagonist, 5HT₁ receptor antagonist, antibiotics, or a combination thereof. In certain embodiments, the at least one additional active agent is an antacid (e.g., calcium carbonate and/or magnesium hydroxide).

PPIs useful herein include, by way of non-limiting example, omeprazole, hydroxyomeprazole, esomeprazole, tenatoprazole, lansoprazole, pantoprazole, rabeprazole, dontoprazole, habeprazole, perprazole, ransoprazole, pariprazole, leminoprazole, S-tenatoprazole-Na, and dexlansoprazole.

In some embodiments, the at least one additional active agent is an H2 receptor ligand (e.g., H2 receptor antagonist). In certain embodiments, H2 receptor antagonists useful herein include, by way of non-limiting example, cimetidine, rantitidine, famotidine and nizatidine. In some embodiments, the therapeutic agent is a H3 receptor ligand (e.g., agonist or antagonist). In certain embodiments, suitable H3 receptor agonists include, by way of non-limiting example, (R)-α-methyl-histamine. In some embodiments, the therapeutic agent is a H1 receptor ligand (e.g., antagonist).

In certain embodiments, the at least one additional active agent is a TLESR-reducing agent. Suitable TLESR-reducing agents include, by way of non-limiting example, GABA_{B} agonists (e.g., baclofen), cholecystokinin (CCK-A or CCK-1) antagonists, anticholinergic agents, NO synthase inhibitors, and combinations thereof.

In some embodiments, the at least one additional active agent is a prokinetic serotonergic agent. In certain embodiments, suitable prokinetic serotonergic agents include, by way of non-limiting example, 5-HT₄ receptor agonists (e.g., selective 5-HT₄ receptor agonists). 5-HT₄ receptor agonists include, by way of non-limiting example, cisapride, mosapride, tegaserod, and ATI-7505.

In some embodiments, the at least one additional active agent is a potassium competitive acid blocker (P-CAB). In certain embodiments, suitable P-CAB include, by way of non-limiting example, soraprazan (BY359), revaprazan (YH1885), AZD0865, CS-526 and combinations thereof.

In certain embodiments, the at least one additional active agent is a mucosal protectant. In some embodiments, suitable mucosal protectants include, by way of non-limiting example, sucralfate. In some embodiments, mucosal protectants include one or more of prostaglandin E₂ (PGE₂), epidermal growth factor (EGF) and/or transforming growth factor-a (TGF- α), or analogs thereof. In a specific embodiment, the mucosal protectant comprises the PGE₂ analog trimoprostil.

In certain embodiments, the at least one additional active agent is an anti-gastrin agent. In some embodiments, suitable anti-gastrin agents include, by way of non-limiting example, cholecystokinin (CCK-B or CCK-2) antagonists. Cholecystokinin (CCK-B or CCK-2) antagonists include, by way of non-limiting example, Z-360.

In some embodiments, the therapeutic agent is a wound healing agent, an agent that promotes cell survival, an agent that promotes cell proliferation, an antifungal agent, an antibacterial agent, an antibiotic, or a combination thereof.

In further embodiments, the at least one additional active agent is a promotility agent. In some embodiments, promotility agents include, by way of non-limiting embodiments, metoclopramide, cisapride, bethanechol, or the like.

In some embodiments, the at least one additional active agent is a chemotherapeutic agent. In some embodiments, chemotherapeutic agents include, by way of non-limiting example, 5-fluorouracil, cisplatin, docetaxel, irinotecan, mitomycin, paclitaxel, vindesine, vinorelbine, and the like.

In certain embodiments, the at least one additional active agent is a mast cell inhibitor. In some embodiments, suitable mast cell inhibitors include, by way of non-limiting example, cromolyn, cromolyn sodium, nedocromil, and the like. In certain embodiments, the therapeutic agent is a leukotriene antagonist. In some embodiments, suitable leukotriene antagonists include, by way of non-limiting example, montelukast, zafirlukast, zileuton, and the like. In some embodiments, mast cell inhibitors described herein (e.g., montelukast) are present in a composition or dose of a composition described herein in an amount sufficient to provide to an individual about 0.1 to about 20 mg/day, about 0.3 to about 4 mg/day, about 10 mg/day to about 500 mg/day, about 20 mg/day to about 40 mg/day, about 20 mg/day to about 100 mg/day, or any other therapeutically effective amount.

In some embodiments, the at least one additional active agent is a non-steroidal anti-inflammatory drug (NSAID). In specific embodiments, the NSAID is ketoprofen. In various other embodiments, the therapeutic agent is an anti-inflammatory agent, e.g., one as set forth in WO 2008/070129.

In some embodiments, the at least one additional active agent is an immunomodulator or immunosuppressant. In specific embodiments, the immunomodulator is 6-mercaptopurine (6MP), azathioprine, suplatast tosylate, mycophenolate mofetil, lactobacillus, calcineurin inhibitors (e.g., tacrolimus, cyclosporine, or the like), or the like.

In certain embodiments, the at least one additional active agent is a biologic. In specific embodiments, the biologic is an anti IL5, an anti TNF (e.g., TNF-α), an IFN (e.g., IFN- α), an anti-eotaxin-1 antibody, an anti IL-3, an anti IgE, omalizumab, reslizumab, mepolizumab, daclizumab, SCH55700, or the like.

In some embodiments, chemotherapeutic agents include, by way of non-limiting example, imatinib, imatinib mesylate, dasatinib, AMN 107, cladribine, or the like.

In some embodiments, the at least one additional active agent is an antispasmodic, an agent for treating chest pain (e.g., nitrates, nitroglycerine, or the like), a drug normally administered sublingually (e.g., vitamins, minerals, or the like), mepoliz, esomepraz, STI571, imatinib, an anti-CD25 (e.g., daclizumab), tyrosine kinase inhibitors, somatostatin, somatostatin analogs, an anti-CCR-3, an anti-TIM-3, ketotifen, a platelet derived growth factor receptor (PDGFR) inhibitor, or the like.

Citation of the above patents, patent applications, publications and
documents is not an admission that any of the foregoing is pertinent prior art, nor does it constitute any admission as to the contents or date of these publications or documents.

Unless defined otherwise, all technical and scientific terms used herein have the same meanings as commonly understood by one of ordinary skill in the art to which this invention belongs. Although any methods and systems similar or equivalent to those described herein can be used in the practice or testing of the present invention, the methods, devices, and materials are now described. Nothing herein is to be construed as an admission that the invention is not entitled to antedate such disclosure by virtue of prior invention.

Modifications may be made to the foregoing without departing from the basic aspects of the invention. Although the invention has been described in substantial detail with reference to one or more specific embodiments, those of ordinary skill in the art will recognize that changes may be made to the embodiments specifically disclosed in this application, and yet these modifications and improvements are within the scope of the invention. The invention illustratively described herein suitably may be practiced in the absence of any element(s) not specifically disclosed herein. Thus, for example, in each instance herein any of the terms "comprising", "consisting essentially of, and "consisting of may be replaced with either of the other two terms. Thus, the terms and expressions which have been employed are used as terms of description and not of limitation, equivalents of the features shown and described, or portions thereof, are not excluded, and it is recognized that various modifications are possible within the scope of the invention.

While certain embodiments have been shown and described herein, it will be apparent to those skilled in the art that such embodiments are provided by way of example only. Numerous variations, changes, and substitutions will now occur to those skilled in the art and are considered to be within the scope of the disclosure herein. It should be understood that various alternatives to the embodiments of the invention described herein may be employed in practicing the invention. It is intended that the following claims define the scope of the invention and that methods and structures within the scope of these claims and their equivalents be covered thereby.

### Examples (the following compositions comprising dextrose and a preservative form part of the invention)

### Example 1: Budesonide Formulation

An exemplary composition described herein is prepared by combining the following:

| **Ingredient** | **Amount** |
|---|---|
| Budesonide | 7.5 mg to 100 mg |
| Avicel (RC-591) | 0.5 g to 4 g |
| Disodium Edetate | 5 mg to 200 mg |
| Citric Acid | 10 mg to 1 g |
| Sodium Citrate | 10 mg to 2 g |
| Tween 80 | 5 mg to 100 mg |
| Flavoring Agent | optional |
| Sweetener | optional |
| Preservative | optional |
| Water | q.s. to 100 mL |

The composition is divided into a unit dose of about 1 mL to about 10 mL (e.g., about 5 mL) and administered orally to an individual to treat, prevent or alleviate inflammation or symptoms of inflammation of the gastrointestinal tract (e.g., the esophagus).

### Example 2: Budesonide Formulation

An exemplary composition described herein is prepared by combining the following:

| **Ingredient** | **Amount** |
|---|---|
| Budesonide | 7.5 mg to 100 mg |
| CMC | 0.5 g to 3 g |
| Disodium Edetate | 5 mg to 200 mg |
| Citric Acid | 10 mg to 1 g |
| Sodium Citrate | 10 mg to 2 g |
| Tween 80 | 5 mg to 100 mg |
| Flavoring Agent | optional |
| Sweetener | optional |
| Preservative | optional |
| Water | q.s. to 100 mL |

The composition is divided into a unit dose of about 1 mL to about 10 mL (e.g., about 5 mL) and administered orally to an individual to treat, prevent or alleviate inflammation or symptoms of inflammation of the gastrointestinal tract (e.g., the esophagus).

### Example 3: Budesonide Formulation

An exemplary composition described herein is prepared by combining the following:

| **Ingredient** | **Amount** |
|---|---|
| Budesonide | 7.5 mg to 100 mg |
| Carbomer | 0.5 g to 10 g |
| Disodium Edetate | 5 mg to 200 mg |
| Citric Acid | 10 mg to 1 g |
| Sodium Citrate | 10 mg to 2 g |
| Tween 80 | 5 mg to 100 mg |
| Flavoring Agent | optional |
| Sweetener | optional |
| Preservative | optional |
| Water | q.s. to 100 mL |

The composition is divided into a unit dose of about 1 mL to about 10 mL (e.g., about 5 mL) and administered orally to an individual to treat, prevent or alleviate inflammation or symptoms of inflammation of the gastrointestinal tract (e.g., the esophagus).

### Example 4: Budesonide Formulation

An exemplary composition described herein is prepared by combining the following:

| **Ingredient** | **Amount** |
|---|---|
| Budesonide | 7.5 mg to 100 mg |
| HPMC | 0.5 g to 3 g |
| Disodium Edetate | 5 mg to 200 mg |
| Citric Acid | 10 mg to 1 g |
| Sodium Citrate | 10 mg to 2 g |
| Tween 80 | 5 mg to 100 mg |
| Flavoring Agent | optional |
| Sweetener | optional |
| Preservative | optional |
| Water | q.s. to 100 mL |

The composition is divided into a unit dose of about 1 mL to about 10 mL (e.g., about 5 mL) and administered orally to an individual to treat, prevent or alleviate inflammation or symptoms of inflammation of the gastrointestinal tract (e.g., the esophagus).

### Example 5: Budesonide Formulation

An exemplary composition described herein is prepared by combining the following:

| **Ingredient** | **Amount** |
|---|---|
| Budesonide | 7.5 mg to 100 mg |
| MCC | 0.5 g to 3 g |
| Disodium Edetate | 5 mg to 200 mg |
| Citric Acid | 10 mg to 1 g |
| Sodium Citrate | 10 mg to 2 g |
| Tween 80 | 5 mg to 100 mg |
| Flavoring Agent | optional |
| Sweetener | optional |
| Preservative | optional |
| Water | q.s. to 100 mL |

The composition is divided into a unit dose of about 1 mL to about 10 mL (e.g., about 5 mL) and administered orally to an individual to treat, prevent or alleviate inflammation or symptoms of inflammation of the gastrointestinal tract (e.g., the esophagus).

### Example 6: Budesonide Formulation

An exemplary composition described herein is prepared by combining the following:

| **Ingredient** | **Amount** |
|---|---|
| Budesonide | 7.5 mg to 100 mg |
| Dextrose | 1 g to 100 g |
| Disodium Edetate | 5 mg to 200 mg |
| Citric Acid | 10 mg to 1 g |
| Sodium Citrate | 10 mg to 2 g |
| Tween 80 | 5 mg to 100 mg |
| Flavoring Agent | optional |
| Sweetener | optional |
| Preservative | optional |
| Water | q.s. to 100 mL |

The composition is divided into a unit dose of about 1 mL to about 10 mL (e.g., about 5 mL) and administered orally to an individual to treat, prevent or alleviate inflammation or symptoms of inflammation of the gastrointestinal tract (e.g., the esophagus).

### Example 7: Budesonide Formulation

An exemplary composition described herein is prepared by combining the following:

| **Ingredient** | **Amount** |
|---|---|
| Budesonide | 7.5 mg to 100 mg |
| Maltodextrin | 1 g to 100 g |
| Disodium Edetate | 5 mg to 200 mg |
| Citric Acid | 10 mg to 1 g |
| Sodium Citrate | 10 mg to 2 g |
| Tween 80 | 5 mg to 100 mg |
| Flavoring Agent | optional |
| Sweetener | optional |
| Preservative | optional |
| Water | q.s. to 100 mL |

The composition is divided into a unit dose of about 1 mL to about 10 mL (e.g., about 5 mL) and administered orally to an individual to treat, prevent or alleviate inflammation or symptoms of inflammation of the gastrointestinal tract (e.g., the esophagus).

### Example 8: Budesonide Formulation

An exemplary composition described herein is prepared by combining the following:

| **Ingredient** | **Amount** |
|---|---|
| Budesonide | 7.5 mg to 100 mg |
| Dextrose | 1 g to 100 g |
| Maltodextrin | 1 g to 100 g |
| Disodium Edetate | 5 mg to 200 m |
| Citric Acid | 10 mg to 1 g |
| Sodium Citrate | 10 mg to 2 g |
| Tween 80 | 5 mg to 100 mg |
| Flavoring Agent | optional |
| Sweetener | optional |
| Preservative | optional |
| Water | q.s. to 100 mL |

The composition is divided into a unit dose of about 1 mL to about 10 mL (e.g., about 5 mL) and administered orally to an individual to treat, prevent or alleviate inflammation or symptoms of inflammation of the gastrointestinal tract (e.g., the esophagus).

### Example 9: Budesonide Formulation

An exemplary composition described herein is prepared by combining the following:

| **Ingredient** | **Amount** |
|---|---|
| Budesonide | 7.5 mg to 100 mg |
| HEC | 0.5 g to 5 g |
| Disodium Edetate | 5 mg to 200 mg |
| Citric Acid | 10 mg to 1 g |
| Sodium Citrate | 10 mg to 2 g |
| Tween 80 | 5 mg to 100 mg |
| Flavoring Agent | optional |
| Sweetener | optional |
| Preservative | optional |
| Water | q.s. to 100 mL |

The composition is divided into a unit dose of about 1 mL to about 10 mL (e.g., about 5 mL) and administered orally to an individual to treat, prevent or alleviate inflammation or symptoms of inflammation of the gastrointestinal tract (e.g., the esophagus).

### Example 10: Fluticasone Propionate Formulation

An exemplary composition described herein is prepared by combining the following:

| **Ingredient** | **Amount** |
|---|---|
| Fluticasone Propionate | 1 mg to 100 mg |
| Avicel (RC-591) | 0.5 g to 4 g |
| Disodium Edetate | 5 mg to 200 mg |
| Citric Acid | 10 mg to 1 g |
| Sodium Citrate | 10 mg to 2 g |
| Tween 80 | 5 m to 100 mg |
| Flavoring Agent | optional |
| Sweetener | optional |
| Preservative | optional |
| Water | q.s. to 100 mL |

The composition is divided into a unit dose of about 1 mL to about 10 mL (e.g., about 5 mL) and administered orally to an individual to treat, prevent or alleviate inflammation or symptoms of inflammation of the gastrointestinal tract (e.g., the esophagus).

### Example 11: Fluticasone Propionate Formulation

An exemplary composition described herein is prepared by combining the following:

| **Ingredient** | **Amount** |
|---|---|
| Fluticasone Propionate | 1 mg to 100 mg |
| CMC | 0.5 g to 3 g |
| Disodium Edetate | 5 mg to 200 mg |
| Citric Acid | 10 mg to 1 g |
| Sodium Citrate | 10 mg to 2 g |
| Tween 80 | 5 mg to 100 mg |
| Flavoring Agent | optional |
| Sweetener | optional |
| Preservative | optional |
| Water | q.s. to 100 mL |

The composition is divided into a unit dose of about 1 mL to about 10 mL (e.g., about 5 mL) and administered orally to an individual to treat, prevent or alleviate inflammation or symptoms of inflammation of the gastrointestinal tract (e.g., the esophagus).

### Example 12: Fluticasone Propionate Formulation

An exemplary composition described herein is prepared by combining the following:

| **Ingredient** | **Amount** |
|---|---|
| Fluticasone Propionate | 1 mg to 100 mg |
| Carbomer | 0.5 g to 10 g |
| Disodium Edetate | 5 mg to 200 mg |
| Citric Acid | 10 mg to 1 g |
| Sodium Citrate | 10 mg to 2 g |
| Tween 80 | 5 mg to 100 mg |
| Flavoring Agent | optional |
| Sweetener | optional |
| Preservative | optional |
| Water | q.s. to 100 mL |

The composition is divided into a unit dose of about 1 mL to about 10 mL (e.g., about 5 mL) and administered orally to an individual to treat, prevent or alleviate inflammation or symptoms of inflammation of the gastrointestinal tract (e.g., the esophagus).

### Example 13: Fluticasone Propionate Formulation

An exemplary composition described herein is prepared by combining the following:

| **Ingredient** | **Amount** |
|---|---|
| Fluticasone Propionate | 1 mg to 100 mg |
| HPMC | 0.5 g to 3 g |
| Disodium Edetate | 5 mg to 200 mg |
| Citric Acid | 10 mg to 1 g |
| Sodium Citrate | 10 mg to 2 g |
| Tween 80 | 5 mg to 100 mg |
| Flavoring Agent | optional |
| Sweetener | optional |
| Preservative | optional |
| Water | q.s. to 100 mL |

The composition is divided into a unit dose of about 1 mL to about 10 mL (e.g., about 5 mL) and administered orally to an individual to treat, prevent or alleviate inflammation or symptoms of inflammation of the gastrointestinal tract (e.g., the esophagus).

### Example 14: Fluticasone Propionate Formulation

An exemplary composition described herein is prepared by combining the following:

| **Ingredient** | **Amount** |
|---|---|
| Fluticasone Propionate | 1 mg to 100 mg |
| MCC | 0.5 g to 3 g |
| Disodium Edetate | 5 mg to 200 mg |
| Citric Acid | 10 mg to 1 g |
| Sodium Citrate | 10 mg to 2 g |
| Tween 80 | 5 mg to 100 mg |
| Flavoring Agent | optional |
| Sweetener | optional |
| Preservative | optional |
| Water | q.s. to 100 mL |

The composition is divided into a unit dose of about 1 mL to about 10 mL (e.g., about 5 mL) and administered orally to an individual to treat, prevent or alleviate inflammation or symptoms of inflammation of the gastrointestinal tract (e.g., the esophagus).

### Example 15: Fluticasone Propionate Formulation

An exemplary composition described herein is prepared by combining the following:

| **Ingredient** | **Amount** |
|---|---|
| Fluticasone Propionate | 1 mg to 100 mg |
| Maltodextrin | 1 g to 100 g |
| Disodium Edetate | 5 mg to 200 mg |
| Citric Acid | 10 mg to 1 g |
| Sodium Citrate | 10 mg to 2 g |
| Tween 80 | 5 mg to 100 mg |
| Flavoring Agent | optional |
| Sweetener | optional |
| Preservative | optional |
| Water | q.s. to 100 mL |

The composition is divided into a unit dose of about 1 mL to about 10 mL (e.g., about 5 mL) and administered orally to an individual to treat, prevent or alleviate inflammation or symptoms of inflammation of the gastrointestinal tract (e.g., the esophagus).

### Example 16: Fluticasone Propionate Formulation

An exemplary composition described herein is prepared by combining the following:

| **Ingredient** | **Amount** |
|---|---|
| Fluticasone Propionate | 1 mg to 100 mg |
| Dextrose | 1 g to 100 g |
| Disodium Edetate | 5 mg to 200 mg |
| Citric Acid | 10 mg to 1 g |
| Sodium Citrate | 10 mg to 2 g |
| Tween 80 | 5 mg to 100 mg |
| Flavoring Agent | optional |
| Sweetener | optional |
| Preservative | optional |
| Water | q.s. to 100 mL |

The composition is divided into a unit dose of about 1 mL to about 10 mL (e.g., about 5 mL) and administered orally to an individual to treat, prevent or alleviate inflammation or symptoms of inflammation of the gastrointestinal tract (e.g., the esophagus).

### Example 17: Fluticasone Propionate Formulation

An exemplary composition described herein is prepared by combining the following:

| **Ingredient** | **Amount** |
|---|---|
| Fluticasone Propionate | 1 mg to 100 mg |
| Dextrose | 1 g to 100 g |
| Maltodextrin | 1 g to 100 g |
| Disodium Edetate | 5 mg to 200 mg |
| Citric Acid | 10 mg to 1 g |
| Sodium Citrate | 10 mg to 2 g |
| Tween 80 | 5 mg to 100 mg |
| Flavoring Agent | optional |
| Sweetener | optional |
| Preservative | optional |
| Water | q.s. to 100 mL |

The composition is divided into a unit dose of about 1 mL to about 10 mL (e.g., about 5 mL) and administered orally to an individual to treat, prevent or alleviate inflammation or symptoms of inflammation of the gastrointestinal tract (e.g., the esophagus).

### Example 18: Fluticasone Propionate Formulation

An exemplary composition described herein is prepared by combining the following:

| **Ingredient** | **Amount** |
|---|---|
| Fluticasone Propionate | 1 mg to 100 mg |
| HEC | 0.5 g to 5 g |
| Disodium Edetate | 5 mg to 200 mg |
| Citric Acid | 10 mg to 1 g |
| Sodium Citrate | 10 mg to 2 g |
| Tween 80 | 5 mg to 100 mg |
| Flavoring Agent | optional |
| Sweetener | optional |
| Preservative | optional |
| Water | q.s. to 100 mL |

The composition is divided into a unit dose of about 1 mL to about 10 mL (e.g., about 5 mL) and administered orally to an individual to treat, prevent or alleviate inflammation or symptoms of inflammation of the gastrointestinal tract (e.g., the esophagus).

### Example 18: Fluticasone Propionate Formulation

An exemplary composition described herein is prepared by combining the following:

| **Ingredient** | **Amount** |
|---|---|
| Fluticasone Propionate | 1 mg to 100 mg |
| HEC | 0.5 g to 5 g |
| Disodium Edetate | 5 mg to 200 mg |
| Citric Acid | 10 mg to 1 g |
| Sodium Citrate | 10 mg to 2 g |
| Tween 80 | 5 mg to 100 mg |
| Flavoring Agent | optional |
| Sweetener | optional |
| Preservative | optional |
| Water | q.s. to 100 mL |

The composition is divided into a unit dose of about 1 mL to about 10 mL (e.g., about 5 mL) and administered orally to an individual to treat, prevent or alleviate inflammation or symptoms of inflammation of the gastrointestinal tract (e.g., the esophagus).

### Example 19: Budesonide Formulation

An exemplary composition described herein is prepared by combining the following:

| **Ingredient** | **Amount** |
|---|---|
| Budesonide | 1 mg |
| Avicel | 100 mg |
| Dextrose | 0.5 g |
| Maltodextrin (M150) | 1.3 g |
| EDTA | 2.5 mg |
| Tween 80 | 0.5 mg |
| Cherry Flavor | 25 mg |
| Glycerin | 250 mg |
| AceK | 37.5 mg |
| Magnasweet | 25 mg |
| Sodium Benzoate | 10 mg |
| Potassium Sorbate | 10 mg |
| Aqueous Citric Acid Buffer | q.s. to 5 mL |

The composition is administered orally to an individual to treat, prevent or alleviate inflammation or symptoms of inflammation of the gastrointestinal tract (e.g., the esophagus).

### Example 20: Budesonide Formulation

An exemplary composition described herein is prepared by combining the following:

| **Ingredient** | **Amount** |
|---|---|
| Budesonide | 1 mg |
| Avicel | 200 mg |
| Dextrose | 1 g |
| Maltodextrin (M150) | 2.6 g |
| EDTA | 5 mg |
| Tween 80 | 1 mg |
| Cherry Flavor | 50 mg |
| Glycerin | 500 mg |
| AceK | 75 mg |
| Magnasweet | 50mg |
| Sodium Benzoate | 10 mg |
| Potassium Sorbate | 10 mg |
| Aqueous Citric Acid Buffer | q.s. to 10 mL |

The composition is administered orally to an individual to treat, prevent or alleviate inflammation or symptoms of inflammation of the gastrointestinal tract (e.g., the esophagus).

### Example 21: Budesonide Formulation

An exemplary composition described herein is prepared by combining the following:

| **Ingredient** | **Amount** (mg/mL) |
|---|---|
| Budesonide | 0.05 |
| Avicel RC-591 | 23.6 |
| Dextrose | 118 |
| Maltodextrin (M150) | 306.8 |
| Disodium edetate | 0.59 |
| Citric acid | 1.77 |
| Sodium citrate | 0.59 |
| Polysorbate 80 | 0.12 |
| Cherry Flavor | 5.9 |
| Glycerin | 59 |
| Acesulfame potassium | 5.9 |
| Magnasweet 110 | 5.9 |
| Sodium Benzoate | 2.36 |
| Potassium Sorbate | 2.36 |
| Water | q.s. to 1, 2, 3, 4, 5, 6, 7, 8, 9, 10, 11, 12, 13, 14,or 15 mL |

The composition is administered orally to an individual to treat, prevent or alleviate inflammation or symptoms of inflammation of the gastrointestinal tract (e.g., the esophagus).

### Example 22: Budesonide Formulation

An exemplary composition described herein is prepared by combining the following:

| **Ingredient** | **Amount (mg/mL)** |
|---|---|
| Budesonide | 0.2 |
| Avicel RC-591 | 23.6 |
| Dextrose | 118 |
| Maltodextrin (M150) | 306.8 |
| Disodium edetate | 0.59 |
| Citric acid | 1.77 |
| Sodium citrate | 0.59 |
| Polysorbate 80 | 0.12 |
| Cherry Flavor | 5.9 |
| Glycerin | 59 |
| Acesulfame potassium | 5.9 |
| Magnasweet 110 | 5.9 |
| Sodium Benzoate | 2.36 |
| Potassium Sorbate | 2.36 |
| Water | q.s. to 1, 2, 3, 4, 5, 6, 7, 8, 9, 10, 11, 12, 13, 14,or 15 mL |

The composition is administered orally to an individual to treat, prevent or alleviate inflammation or symptoms of inflammation of the gastrointestinal tract (e.g., the esophagus).

### Example 23:

In certain instances, the formulations described in the examples set forth herein are scaled to an amount sufficient to provide any desired amount, e.g., about 150 mL or 300 mL of total composition volume, either by scaling up a composition described in the examples herein, e.g., to about 150 mL, or by scaling to a larger volume and dividing the scaled composition into smaller portions, e.g., portions comprising about 150 mL. The portions, e.g., those comprising about 150 mL or 300 mL, may then be placed into a multi dose container. A plurality of doses may then be dispensed and administered to an individual to treat, prevent or alleviate inflammation or symptoms of inflammation of the gastrointestinal tract (e.g., the esophagus).

### Example 24

Budesonide solubility of certain formulations is described. To determine the solubility of budesonide in the liquid medium of a formulation, particulates of the formulation are filtered. Samples are centrifuged at 12,000 rpm and 20 °C using Whatman centrifuge filters, 0.45 µm (Nylon). Filtration time vary depending on sample, but are generally between 5 minutes and 1 hour. Prior to centrifugation, samples are equilibrated, if necessary, to achieve a substantially equilibrated concentration of budesonide. Determined is the concentration of budesonide in the liquid medium and the % (w/w) of the budesonide dissolved that is the R epimer. Formulation A is prepared by combining two Pulmicort Respules® (0.5 mg budesonide in 2 mL each) with 10 g of Splenda® (distributed by McNeil Nutritionals, LLC Fort Washington, PA 19034-2299; about 1 g per packet) and equilibrating for about 48 hours. Formulation B is prepared by combining two Pulmicort Respules® (0.5 mg budesonide in 2 mL each) with about 9 g of maltodextrin having a DE of about 17 and a trace amount of sucralose, and equilibrating for about 48 hours. For Formulations C-I, excess budesonide particles were combined with water, and optionally maltodextrin, and equilibrated over 5 days. Formulation C is saturated budesonide in water. Formulation D is an aqueous composition comprising 2.5% (w/w) maltodextrin (M440, DE 4-7). Formulation E is an aqueous composition comprising 5% (w/w) maltodextrin (M440, DE 4-7). Formulation F is an aqueous composition comprising 10% (w/w) maltodextrin (M440, DE 4-7). Formulation G is an aqueous composition comprising 7.5% (w/w) of maltodextrin (M150, DE 13-17). Formulation H is an aqueous composition comprising 15% (w/w) of maltodextrin (M150, DE 13-17). Formulation I is an aqueous composition comprising 30% (w/w) of maltodextrin (M150, DE 13-17). Formulation J is the aqueous composition of Example 22, comprising 26% (w/w) of maltodextrin (M150, DE 13-17). Formulation K is an aqueous composition comprising 20% w/w of dextrose, equilibrated for 48 hours. Formulation L is an aqueous composition comprising 40% w/w of dextrose, equilibrated for 48 hours. Formulation M is prepared by combining excess budesonide and GUM® Rincinol® P.R.N.™ and filtering. Results are set forth in Table 10.

**Table 10: Budesonide Solubility**

| **Formulation** | **Solubility (µg/mL)** | **% R epimer (w/w)** | **Filtration time (min)** |
|---|---|---|---|
| A | 20 | 37 | 5 |
| B | 70 | 48 | 5 |
| C | 19 | 28 | 5 |
| D | 23 | 33 | 5 |
| E | 34 | 26 | 5 |
| F | 50 | 25 | 5 |
| G | 31 | 32 | 5 |
| H | 45 | 31 | 5 |
| I | 67 | 43 | 5 |
| J | 25 | | 5 |
| K | 16 | 24 | 5 |
| L | 11 | 24 | 5 |
| M | 34 | 29 | 5 |
| Rhinocort Aqua® | 4.0 | 27.9 | 30 |
| Pulmicort Respules® | 19.0 | 27.1 | 5 |

For Formulation A, the unequilibrated concentration of budesonide was 28 µg/mL, 38.7% w/w of the dissolved budesonide being the R epimer. For Formulation B, the unequilibrated concentration of budesonide was 48 µg/mL, 45.3% w/w of the dissolved budesonide being the R epimer.

### Example 25:

This example details the efficacy and safety of once daily and twice daily use of budesonide formulations of Examples 21 and 22 in 5 mL, 7 mL, 10 mL, 12 mL, 15 mL, and 17.5 mL doses in inducing and maintaining remission of disease activity in children with EE. A number of children (e.g., 20 per budesonide dose frequency, amount, and volume) are evaluated to determine the highest eosinophil count (eos/hpf) and the mean highest eosinophil count for the group. Evaluation of the highest eosinophil count (eos/hpf) and the mean highest eosinophil count for the group is also determined following therapy. Symptom scores and mean symptom scores are also determined before and after therapy.

In some instances, individuals who received previous therapy with proton pump inhibitor, elimination diet based upon skin or blood allergy testing, or elimination diet or refused elimination diet, but continued to have ≧ 24 eos/hpf on esophageal biopsy are included in the review. Patients are defined as having food or aeroallergen sensitization if RAST and/or skin prick testing are positive. No changes are made to longstanding therapy used for treating chronic conditions such as asthma or eczema and none of the children receive concurrent immune-modulatory treatment.

Endoscopy is performed using the Olympus P160 endoscope (by RD) and pan-esophageal, gastric and duodenal biopsies are taken. Eosinophilic esophagitis (EE or EoE) is diagnosed when ≧24 eos/hpf are found in at least one of the esophageal sites biopsied. Two mucosal biopsies re taken from the proximal esophagus (3 cm below the crycopharyngeus muscle), distal esophagus (3 cm above the gastroesophageal junction (GEJ), and mid-esophagus (midpoint between the crycopharyngeus muscle and the GEJ). Biopsies are processed routinely and evaluated by a pediatric pathologist (RN). The highest number of eosinophils per x400 high power field are counted. Basal zone hyperplasia (BZH) is reported when basal zone cells extend towards the luminal surface of the epithelium (>25% of epithelial thickness).

Follow-up endoscopy with biopsies are taken after 3-4 months treatment. Counting the highest number of eos/hpf within biopsies determined the response to therapy and patients are categorized into responders (0-7 eos/hpf), partial-responders (8-23 eos/hpf) and non-responders (≧24 eos/hpf).

An EE (EoE)Endoscopy Score is devised to compare findings before and after treatment. It is calculated from procedure reports and photographs. Four categories, (1) pallor and diminished vascular markings; (2) furrowing with "thickened" mucosa; (3) white mucosal plaques; (4) concentric rings or strictures. For each category, one point is allocated if 1 or 2 esophageal sites are involved, and two points for pan-esophageal involvement. The maximum score is 8.

Patients receive a formulation described herein for between 0.25 and 2 mg daily and are instructed not to ingest any solids or liquids for 30 minutes afterwards. No dietary changes are made in patients already on dietary restrictions.

A modified symptom score based on children with acid-peptic disease is used routinely in the EE (EoE) clinic. The symptom categories include (1) heartburn or regurgitation; (2) abdominal pain or unexplained irritability in younger children; (3) nausea or vomiting; (4) anorexia or early satiety; (5) dysphagia or odynophagia, (6) nocturnal wakening with symptoms; (7) gastrointestinal bleeding (previous 4 months). Each category scored 0-2 points with a maximum of 14 points. Zero points are awarded if the symptom is absent; one point if the symptom is mild, did not interfere with daily activities; 2 points if the symptoms are severe enough to interrupt daily activities. Previous GI bleeding is considered mild (1 point) if there is no associated hemodynamic compromise or anemia, and severe (2 points) if bleeds are multiple, caused anemia, or required blood transfusion.

All statistical analysis is carried out using NCSS Statistical Softward Package. Two-tailed p values are calculated using paired t-tests to compare the means of patient values for eos/hpf, EE (EoE) Endoscopy Scores and Symptom Scores before and after budesonide therapy. Two-tailed unpaired t-tests are utilized in order to compare variables grouped by responders versus non-responders. Spearman's correlation coefficients are generated using GraphPad Prism software. Results with p values <0.05 are considered statistically significant. Both mean and median statistics re generated, both are equivalent and mean statistics are presented.

Subjects. Chart reviews are undertaken on a number of children. All children have >24 eos/hpf on repeat esophageal biopsy before starting therapy.

Treatment. Patients received the described formation for a designated amount of time (e.g., 1 week, 2 weeks, 1 month, 2 months, 3 months, 4 months, 6 months, or the like) before repeat endoscopy. Various patients received budesonide in amounts ranging from 0.25 to 2 mg/day.

Histology. Before treatment the mean highest eosinophil count is measured for all patients, including distal, mid and proximal esophageal sites. All sites are likewise evaluated aver the designated amount of time, and again if desired.

Upper Gastrointestinal Endoscopy. Before treatment, the mean EE (EoE) Endoscopy Score for all patients is determined. Following treatment the mean EE (EoE) Endoscopy Score is repeated. Decreases in endoscopy scores (e.g., of >95%, >90%, >85%, >75%, >50%, >25%, or the like) in an individual indicate successful treatment.

Symptom Score. Before treatment the mean symptom score for all patients is determined. It is again determined following treatment. Decreases in symptom scores (e.g., of >95%, >90%, >85%, >75%, >50%, >25%, or the like) in an individual indicate successful treatment (alone or in combination with the above referenced decreases in endoscopy scores).

Adults: these parameters are repeated in adults to determine efficacy and safety therein.

### Example 26:

This example illustrates the increased interaction between a composition described herein and the esophagus when compared to a radiolabeled oral composition made by combining Pulmicort Respules® (4 mL) with ^{99m}Tc pertechnetate, and diluting with saline to about 7-8 mL (M0). The M0 composition has a viscosity of about 1 cP at 13.2 sec⁻¹. Also administered to a population of healthy individuals was a radiolabeled budesonide composition (Rhinocort Aqua®, M1), which has a viscosity of about 39 at 13.2 sec⁻¹. Increased interaction of the budesonide composition was determined by measuring the amount of radiolabel present in the esophagus following oral administration of the M1 budesonide composition. Figure 1 illustrates the percent amount of composition present in the esophagus as a function of time following oral administration (by measuring the amount of radiolabel present in the esophagus).

The area under the curve (AUCr) of the percent of the dose administered as a function of time (%dose·time(min)) was determined from the time of 50% swallow (i.e., 50% of the administered dose had passed from the mouth), until esophageal activity had peaked and fallen to 10% of the peak value. The area under the curve from t=0 min to t = 1 min (AUC₀₋₁); and from t= 0 min to t = 2 min (AUC₀₋₂) was also determined. These results (including the ratio of the non-viscous sample to the viscous sample) are set forth below:

| **Formulation** | **AUCr** | | **AUC₀₋₁** | | **AUC₀₋₂** | |
|---|---|---|---|---|---|---|
| | geometric mean | ratio | geometric mean | ratio | geometric mean | ratio |
| M0 | 3.95 | | 5.51 | | 6.93 | |
| M1 | 9.39 | 0.42 | 11.07 | 0.5 | 14.16 | 0.49 |

### Example 27:

This example details the efficacy and safety of once daily and twice daily use of a formulation or composition described herein in inducing and maintaining remission of disease activity in individuals (children and/or adults) with GERD. Doses of 0-1 mg, 1-2 mg, 2-3 mg, 3-4 mg, 4-5 mg, and 5-6 mg per daily dose are administered once a day, b.i.d. or t.i.d. in volumes of 3, 5, 7, 10, 12, 15, or 17.5 mL. A number of individuals (e.g., 20 per budesonide dose frequency, amount, and volume) are evaluated to determine the symptoms prior to therapy, during therapy and following therapy. Administration is conducted for 7 days, 14 days, and 28 days. Primary Outcome Measures include complete resolution of heartburn and regurgitation (e.g., no more than one day with either mild heartburn or regurgitation over the seven days prior to the assessment time-point). Secondary Outcome Measures include: Number of days with heartburn (daytime and night-time); Number of days with regurgitation (daytime and night-time); Number of heartburn and regurgitation-free days (24hrs); Composite score of heartburn and regurgitation frequency and severity; Time to resolution of symptoms of heartburn/regurgitation; Severity of additional GERD symptoms; Quality of Life (assessed using PAGI-QOL to PGIC (Patient Global Impression of Change); Complete resolution of heartburn; Complete resolution of regurgitation; Average severity of heartburn (daytime and night-time); Average severity of regurgitation (daytime and night-time). These symptoms are scored (e.g., assigning a 3 to the most severe symptoms and a 0 to a lack of symptoms) and utilized to determine the efficacy of the treatment.

### References

1. Liacouras C A, Ruchelli E. Eosinophilic esophagitis. Cuff. Opin. Pediatr. 2004; 16:560-6.
2. Kelly K J, Lazenby A J, Rowe P C, et al. Eosinophilic esophagitis attributed to gastroesophageal reflux: improvement with an amino acid-based formula. Gastroenterology 1995; 109: 1503-12.
3. Fogg M I, Ruchelli E, Spergel J M. Pollen and eosinophilic esophagitis. J. Allergy Clin. Immunol. 2003; 112:796-7.
4. Mishra A, Hogan S P, Brandt E B, Rothenberg M E. An etiological role for aeroallergens and eosinophils in experimental esophagitis. J. Clin. Invest. 2001; 107:83-90.
5. Spergel J M, Beausoleil J L, Mascarenhas M, Liacouras C A. The use of skin prick tests and patch tests to identify causative foods in eosinophilic esophagitis. J. Allergy Clin. Immunol. 2002; 109:363-8.
6. Ruchelli E, Wenner W, Voytek T, et al. Severity of esophageal eosinophilia predicts response to conventional gastroesophageal reflux therapy. Pediatr. Dev. Pathol. 1999; 2:15-8.
7. Steiner S J, Gupta S K, Croffie J M, Fitzgerald J F. Correlation between number of eosinophils and reflux index on same day esophageal biopsy and 24 hour esophageal pH monitoring. Am. J. Gastroenterol. 2004; 99:801-5.
8. Orenstein S R, Shalaby T M, Di Lorenzo C, et al. The spectrum of pediatric eosinophilic esophagitis beyond infancy: a clinical series of 30 children. Am. J. Gastroenterol. 2000; 95:1422-30.
9. Rothenberg M E, Mishra A, Collins M H, Putnam P E. Pathogenesis and clinical features of eosinophilic esophagitis. J. Allergy Clin. Immunol. 2001; 108:891-4.
10. Ravelli A M, Villanacci V, Ruzzenenti N, et al. Dilated Intercellular Spaces: A Major Morphological Feature of Esophagitis. J. Pediatr. Gastroenterol. Nutr. 2006; 42:510-515.
11. Steiner S J, Kernek K M, Fitzgerald J F. Severity of Basal Cell Hyperplasia Differs in Reflux Versus Eosinophilic Esophagitis. J. Pediatr. Gastroenterol. Nutr. 2006; 42:506-509.
12. Mueller S, Aigner T, Neureiter D, Stolte M. Eosinophil infiltration and degranulation in oesophageal mucosa from adult patients with eosinophilic oesophagitis: a retrospective comparative study on pathologic biopsy. J. Clin. Pathol. 2006; 59:1175-80.
13. Croese J, Fairley S K, Masson J W, et al. Clinical and endoscopic features of eosinophilic esophagitis in adults. Gastrointest. Endosc. 2003; 58:516-22.
14. Aceves S, Newbury, RO, Dohil R, Schwimmer J, Bastian J. Distinguishing Eosinophilic Esophagitis in pediatric patients: clinical, endoscopic, and histologic features of an emerging disorder. Journal of Clinical Gastroenterology 2006; 41(3):252-6.
15. Straumann A, Simon H U. Eosinophilic esophagitis: escalating epidemiology? J. Allergy Clin. Immunol. 2005; 115:418-9.
16. Cherian S, Smith N M, Forbes D A. Rapidly increasing prevalence of eosinophilic oesophagitis in Western Australia. Arch. Dis. Child 2006; 91:1000-4.
17. Sant'Anna A M, Rolland S, Fournet J C, et al. Eosinophilic Esophagitis in Children: Symptoms, Histology and pH Probe Results. J. Pediatr. Gastroenterol. Nutr. 2004; 39:373-377.
18. Potter J W, Saeian K, Staff D, et al. Eosinophilic esophagitis in adults: an emerging problem with unique esophageal features. Gastrointest. Endosc. 2004; 59:355-61.
19. Parfitt J R, Gregor J C, Suskin N G, et al. Eosinophilic esophagitis in adults: distinguishing features from gastroesophageal reflux disease: a study of 41 patients. Mod. Pathol. 2006; 19:90-6.
20. Desai T K, Stecevic V, Chang C H, et al. Association of eosinophilic inflammation with esophageal food impaction in adults. Gastrointest. Endosc. 2005; 61:795-801.
21. Straumann A, Spichtin H P, Grize L, et al. Natural history of primary eosinophilic esophagitis: a follow-up of 30 adult patients for up to 11.5 years. Gastroenterology 2003; 125:1660-9.
22. Spergel J M, Andrews T, Brown-Whitehorn T F, et al. Treatment of eosinophilic esophagitis with specific food elimination diet directed by a combination of skin prick and patch tests. Ann. Allergy Asthma Immunol. 2005; 95:336-43.
23. Kagalwalla A F, Sentongo T A, Ritz S, et al. Effect of six-food elimination diet on clinical and histologic outcomes in eosinophilic esophagitis. Clin. Gastroenterol. Hepatol. 2006; 4:1097-102.
24. Markowitz J E, Spergel J M, Ruchelli E, Liacouras C A. Elemental diet is an effective treatment for eosinophilic esophagitis in children and adolescents. Am. J. Gastroenterol. 2003; 98:777-82.
25. Liacouras C A, Wenner W J, Brown K, Ruchelli E. Primary eosinophilic esophagitis in children: successful treatment with oral corticosteroids. J. Pediatr. Gastroenterol. Nutr. 1998; 26:380-5.
26. Teitelbaum J E, Fox V L, Twarog F J, et al. Eosinophilic esophagitis in children: immunopathological analysis and response to fluticasone propionate. Gastroenterology 2002; 122:1216-25.
27. Faubion W A, Jr., Perrault J, Burgart L J, et al. Treatment of eosinophilic esophagitis with inhaled corticosteroids. J. Pediatr. Gastroenterol. Nutr. 1998; 27:90-3.
28. Aceves S S, Dohil R, Newbury R O, Bastian J F. Topical viscous budesonide suspension for treatment of eosinophilic esophagitis. J. Allergy Clin. Immunol. 2005; 116:705-6.
29. Noel R J, Putnam P E, Collins M H, et al. Clinical and immunopathologic effects of swallowed fluticasone for eosinophilic esophagitis. Clin. Gastroenterol. Hepatol. 2004; 2:568-75.
30. Remedios M, Campbell C, Jones D M, Kerlin P. Eosinophilic esophagitis in adults: clinical, endoscopic, histologic findings, and response to treatment with fluticasone propionate. Gastrointest. Endosc. 2006; 63:3-12.
31. Dohil R, Newbury R O, Sellers Z M, et al. The evaluation and treatment of gastrointestinal disease in children with cystinosis receiving cysteamine. J. Pediatr. 2003; 14:224-30.
32. Cheung K M, Oliver M R, Cameron D J, et al. Esophageal eosinophilia in children with dysphagia. J. Pediatr. Gastroenterol. Nutr. 2003;37:498-503.
33. Fox V L, Nurko S, Furuta G T. Eosinophilic esophagitis: it's not just kid's stuff. Gastrointest. Endosc. 2002; 56:260-70
34. Budin C, Villard-Truc F, Rivet C, et al. [Eosinophilic esophagitis: 3 case reports]. Gastroenterol. Clin. Biol. 2005; 29:73-5.
35. Noel R J, Putnam P E, Rothenberg M E. Eosinophilic esophagitis. N. Engl. J. Med. 2004; 351:940-1.
36. Guajardo J R, Plotnick L M, Fende J M, et al. Eosinophil-associated gastrointestinal disorders: a world-wide-web based registry. J. Pediatr. 2002; 141:576-81.
37. Liacouras C A, Spergel J M, Ruchelli E, et al. Eosinophilic esophagitis: a 10-year experience in 381 children. Clin. Gastroenterol. Hepatol. 2005; 3:1198-206.
38. Liacouras C A. Eosinophilic esophagitis: treatment in 2005. Curr. Opin. Gastroenterol. 2006; 22:147-152.
39. Spergel JM. Eosinophilic esophagitis in adults and children: evidence for a food allergy component in many patients. Curr. Opin. Allergy Clin. Immunol. 2007; 7:274-8.
40. Plaza-Martin, AM, Jimenez-Feijoo R, Andaluz C, Giner-Munoz MT, Martin-Mateos MA, Piquer-Gibert M, Sierra-Martinez JI. Polysensitization to aeroallergens and food in eosinophilic esophagitis in a pediatric population. Alergol. Immunopathol. 2007; 35:35-7.
41. Nicolazzo, JA, Reed, BL, Finnin, BC. Buccal penetration enhancers - how do they really work? J. Controlled Release 2005; 105:1-15.
42. Furuta, GT, Liacouras, CA, Collins, MH, Sandeep, KG, Justinich, C, Putnam, PE, Bonis, P, Hassall, E, Straumann, A, Rothenberg, ME. Eosiniophilic esophagitis in children and adults: A systematic review and consensus recommendations for diagnosis and treatment. Gastroenterology 2007; 133:1342-1363.
43. Aceves, SS, Bastian JF, Newbury, RO, Dohil, R. Oral viscous budesonide: A potential new therapy for eosinophilic esophagitis. Amer. Journal of Gastroenterology 2007; 102:1-9.
44. Rothenberg M E. Eosinophilic gastrointestinal disorders. J. Allergy Clin. Immunol. 2004; 113:11-28.
45. Garrett J K, Jameson S C, Thomson B, Collins M H, Wagoner L E, Freese, D K, et al. Anti-interleukin-5 (mepolizumab) therapy for hypereosinophilic syndromes. J. Allergy Clin. Immunol. 2004; 113:115-9.

## Claims

1. A physically and chemically stable oral pharmaceutical composition for use in treating, preventing or alleviating esophageal inflammation comprising:
a. a therapeutically effective amount of corticosteroid,
b. an antioxidant,
c. a buffer,
d. a surfactant,
e. a preservative,
f. a flavoring agent, a sweetener, or a combination thereof;
g. dextrose, and
h. a liquid vehicle,
wherein the oral pharmaceutical composition is suitable for single or multiple dose administration.

2. A composition for use of claim 1 comprising:
a. disodium edetate as an antioxidant,
b. sodium citrate as a buffer,
c. polysorbate 80 as a surfactant, and
d. water as a liquid vehicle.

3. A composition for use of either of claims 1 or 2, wherein the pharmaceutical composition is for use in treating or preventing esophageal inflammation or symptoms associated therewith.

4. A composition for use of claim 3, wherein the esophageal inflammation is associated with eosinophilic esophagitis or gastroesophageal reflux disease (GERD).

5. A composition for use of either of claims 1 or 2, wherein the composition remains substantially uniform after storage for at least one day.

6. A composition for use of claim 1, wherein the composition is a multiple dose formulation and wherein the composition is present in a multiple-dose container comprising a plurality of doses.

7. A composition for use of claim 1, wherein the corticosteroid is a topically active corticosteroid.

8. A composition for use of claim 7, wherein the topically active corticosteroid is budesonide, fluticasone, mometasone furoate, ciclesonide, triamcinolone, beclomethasone, or a pharmaceutically acceptable ester thereof, or a combination thereof.

9. A composition for use of claim 1, further comprising one or more maltodextrin, hydroxyethylcellulose (HEC), hydroxypropylmethyl-cellulose (HPMC), carboxymethyl-cellulose (CMC), microcrystalline cellulose (MCC), carbomer or combinations thereof.

10. A composition for use of claim 1, wherein the composition is a suspension of corticosteroids and wherein the composition is substantially free of non-corticosteroid particles.

11. A composition for use of claim 1, wherein the composition is a suspension of corticosteroids particles and wherein the composition comprises less than 5% w/w of undissolved particles.

12. A composition for use of claim 1, wherein the pharmaceutical composition is a non-Newtonian fluid.

13. A composition for use of claim 12, wherein the non-Newtonian fluid is selected from the group consisting of plastic, pseudoplastic and dilatant.

14. A composition for use of claim 1, having a gel, cream, ointment, spreadable, flowable, or paste-like consistency.

15. A composition for use of claim 13, wherein the non-Newtonian fluid is pseudoplastic and thixotropic.

16. A composition for use of claim 1, wherein the liquid vehicle comprises an aqueous medium.

17. A composition for use of claim 16, wherein the pharmaceutical composition comprises corticosteroid particles suspended in the aqueous medium.

18. A composition for use of claim 17, wherein the corticosteroid particles are microparticles having a mean diameter of 0.1 microns to 50 microns.

19. A composition for use of claim 17, wherein at least 95% of the corticosteroid particles are microparticles having a diameter of less than 10 microns.

20. A composition for use of claim 16, the corticosteroid is present in an amount of 0.01 mg to 1 mg per mL.

21. A composition for use of claim 16, wherein the pharmaceutical composition has a volume of 1 mL to 20 mL.

22. A composition for use of claim 21, wherein the pharmaceutical composition comprises 0.1 mg to 20 mg of corticosteroid.

23. A composition for use of claim 16, wherein the composition comprises an additional excipient which comprises CMC, and wherein the CMC is present in an amount of 1 mg to 30 mg per mL of composition.

24. A composition for use of claim 16, wherein the composition comprises an additional excipient which comprises MCC, and wherein the MCC is present in an amount of 5 mg to 30 mg per mL of composition.

25. A composition for use of claim 16, wherein the composition comprises an additional excipient which comprises a combination of CMC and MCC, wherein the CMC-MCC combination is present in an amount of 10 mg per mL to 40 mg per mL of composition, and wherein the CMC/ MCC mixed weight ratio is 11/89.

26. A composition for use of claim 1, wherein the composition comprises an additional excipient which comprises maltodextrin, and wherein the maltodextrin is present in an amount of greater than 8% w/w.

27. A composition for use of claim 26, wherein the maltodextrin has a dextrose equivalents (DE) of 13 to 18.

28. A composition for use of claim 26, wherein the maltodextrin has a dextrose equivalents (DE) of greater than 5.

29. A composition for use of claim 16, wherein the antioxidant is disodium edetate and edetate is present in an amount of 0.05 mg to 25 mg per mL of composition.

30. A composition for use of claim 1, having a viscosity of at least 100 mPa.s measured at 20 to 25 degrees Celsius with a shear rate of 15 sec⁻¹.

31. A composition for use of claim 1, wherein the stable pharmaceutical composition is chemically stable, comprising at least 90% of the initial amount of topically active corticosteroid after being stored for 3 weeks.

32. A composition for use of claim 2 comprising:
a. budesonide in an amount of 0.02 mg to 0.75 mg per mL of composition,
b. disodium edetate in an amount of 0.05 mg to 25 mg per mL of composition,
c. sodium citrate in an amount of 0.1 mg to 30 mg per mL of composition,
d. polysorbate 80 in an amount of 0.05 mg to 1 mg per mL of composition, and
e. an aqueous liquid vehicle.

33. A kit comprising a multiple unit container and a plurality of doses of a stable oral pharmaceutical composition for use of claim 1.

34. A kit of claim 33, wherein the kit comprises 2 to 60 doses of the pharmaceutical composition.

35. A kit of claim 33, wherein the kit further comprises a metering device for administering the composition to an individual.

36. The composition for use of claim 1, wherein the composition comprises an additional excipient which is a viscosity enhancing agent and wherein at least 10 w% of the composition is present within the esophagus at least 15 seconds after oral administration.

37. A composition for use of claim 36, wherein the composition is formulated as a tablet, pill, dragee, capsule, soft chew, cream, paste, chewable tablet, gel, gel matrix, gums, effervescent tablet, or lozenge.

## Patentansprüche

1. Physikalisch und chemisch stabile orale pharmazeutische Zusammensetzung zur Verwendung in der Behandlung, Prävention oder Linderung einer Speiseröhrenentzündung, umfassend:
a. eine therapeutisch wirksame Menge eines Kortikosteroids,
b. ein Antioxidans,
c. einen Puffer,
d. ein Tensid,
e. ein Konservierungsmittel,
f. einen Aromastoff, ein Süßungsmittel oder eine Kombination davon;
g. Dextrose, und
h. eine Trägerflüssigkeit,
wobei die orale pharmazeutische Zusammensetzung zur Verabreichung von Einzel- oder Mehrfachdosen geeignet ist.

2. Zusammensetzung zur Verwendung nach Anspruch 1, umfassend:
a. Dinatriumedetat als Antioxidans,
b. Natriumzitrat als Puffer,
c. Polysorbat 80 als Tensid, und
d. Wasser als Trägerflüssigkeit.

3. Zusammensetzung zur Verwendung nach Anspruch 1 oder 2, wobei die pharmazeutische Zusammensetzung zur Verwendung in der Behandlung oder Prävention von Speiseröhrenentzündung oder den damit verbundenen Symptomen bestimmt ist.

4. Zusammensetzung zur Verwendung nach Anspruch 3, wobei die Speiseröhrenentzündung mit eosinophiler Ösophagitis oder gastroösophagealer Refluxkrankheit (GERD) assoziiert ist.

5. Zusammensetzung zur Verwendung nach Anspruch 1 oder 2, wobei die Zusammensetzung nach Lagerung für mindestens einen Tag im Wesentlichen gleichförmig bleibt.

6. Zusammensetzung zur Verwendung nach Anspruch 1, wobei die Zusammensetzung eine Mehrfachdosis-Formulierung ist und wobei die Zusammensetzung in einem Mehrfachdosisbehälter, der eine Vielzahl von Dosen umfasst, enthalten ist.

7. Zusammensetzung zur Verwendung nach Anspruch 1, wobei das Kortikosteroid ein topisch wirksames Kortikosteroid ist.

8. Zusammensetzung zur Verwendung nach Anspruch 7, wobei das topisch wirksame Kortikosteroid Budesonid, Fluticason, Mometasonfuroat, Ciclesonid, Triamcinolon, Beclomethason oder ein pharmazeutisch annehmbarer Ester davon oder eine Kombination davon ist.

9. Zusammensetzung zur Verwendung nach Anspruch 1, ferner umfassend ein oder mehrere von Maltodextrin, Hydroxyethylcellulose (HEC), Hydroxypropylmethylcellulose (HPMC), Carboxymethylcellulose (CMC), mikrokristalline Cellulose (MCC), Carbomer oder Kombinationen davon.

10. Zusammensetzung zur Verwendung nach Anspruch 1, wobei die Zusammensetzung eine Suspension von Kortikosteroiden ist und wobei die Zusammensetzung im Wesentlichen frei von Nicht-Kortikosteroid-Partikeln ist.

11. Zusammensetzung zur Verwendung nach Anspruch 1, wobei die Zusammensetzung eine Suspension von Kortikosteroid-Partikeln ist und wobei die Zusammensetzung weniger als 5% w/w ungelöste Partikel umfasst.

12. Zusammensetzung zur Verwendung nach Anspruch 1, wobei die pharmazeutische Zusammensetzung eine nichtnewtonsche Flüssigkeit ist.

13. Zusammensetzung zur Verwendung nach Anspruch 12, wobei die nichtnewtonsche Flüssigkeit aus der aus Plastik, Pseudoplastik und Dilatationsmittel bestehenden Gruppe ausgewählt ist.

14. Zusammensetzung zur Verwendung nach Anspruch 1, die eine gelförmige, cremige, salbenförmige, verteilbare, fließfähige oder pastenförmige Konsistenz aufweist.

15. Zusammensetzung zur Verwendung nach Anspruch 13, wobei die nichtnewtonsche Flüssigkeit pseudoplastisch und thixotrop ist.

16. Zusammensetzung zur Verwendung nach Anspruch 1, wobei die Trägerflüssigkeit ein wässriges Medium umfasst.

17. Zusammensetzung zur Verwendung nach Anspruch 16, wobei die pharmazeutische Zusammensetzung Kortikosteroid-Partikel, die im wässrigen Medium suspendiert sind, umfasst.

18. Zusammensetzung zur Verwendung nach Anspruch 17, wobei die Kortikosteroid-Partikel Mikropartikel mit einem mittleren Durchmesser von 0,1 Mikron bis 50 Mikron sind.

19. Zusammensetzung zur Verwendung nach Anspruch 17, wobei mindestens 95% der Kortikosteroid-Partikel Mikropartikel mit einem Durchmesser von weniger als 10 Mikron sind.

20. Zusammensetzung zur Verwendung nach Anspruch 16, wobei das Kortikosteroid in einer Menge von 0,01 mg bis 1 mg pro ml vorliegt.

21. Zusammensetzung zur Verwendung nach Anspruch 16, wobei die pharmazeutische Zusammensetzung ein Volumen von 1 ml bis 20 ml aufweist.

22. Zusammensetzung zur Verwendung nach Anspruch 21, wobei die pharmazeutische Zusammensetzung 0,1 mg bis 20 mg Kortikosteroid umfasst.

23. Zusammensetzung zur Verwendung nach Anspruch 16, wobei die Zusammensetzung einen zusätzlichen, CMC umfassenden Hilfsstoff umfasst, und wobei CMC in einer Menge von 1 mg bis 30 mg pro ml Zusammensetzung vorliegt.

24. Zusammensetzung zur Verwendung nach Anspruch 16, wobei die Zusammensetzung einen zusätzlichen, MCC umfassenden Hilfsstoff umfasst, und wobei MCC in einer Menge von 5 mg bis 30 mg pro ml Zusammensetzung vorliegt.

25. Zusammensetzung zur Verwendung nach Anspruch 16, wobei die Zusammensetzung einen zusätzlichen Hilfsstoff umfasst, der eine Kombination von CMC und MCC umfasst, wobei die CMC-MCC-Kombination in einer Menge von 10 mg pro ml bis 40 mg pro ml Zusammensetzung vorliegt, und wobei das gemischte Gewichtsverhältnis von CMC/MCC 11/89 beträgt.

26. Zusammensetzung zur Verwendung nach Anspruch 1, wobei die Zusammensetzung einen zusätzlichen, Maltodextrin umfassenden Hilfsstoff umfasst, und wobei Maltodextrin in einer Menge von mehr als 8% w/w vorliegt.

27. Zusammensetzung zur Verwendung nach Anspruch 26, wobei Maltodextrin Dextrose-Äquivalente (DE) von 13 bis 18 aufweist.

28. Zusammensetzung zur Verwendung nach Anspruch 26, wobei Maltodextrin Dextrose-Äquivalente (DE) von mehr als 5 aufweist.

29. Zusammensetzung zur Verwendung nach Anspruch 16, wobei das Antioxidans Dinatriumedetat ist und Edetat in einer Menge von 0,05 mg bis 25 mg pro ml Zusammensetzung vorliegt.

30. Zusammensetzung zur Verwendung nach Anspruch 1, die eine Viskosität von mindestens 100 mPa.s, gemessen bei 20 bis 25 Grad Celsius, mit einer Scherrate von 15 sec⁻¹ aufweist.

31. Zusammensetzung zur Verwendung nach Anspruch 1, wobei die stabile pharmazeutische Zusammensetzung chemisch stabil ist und mindestens 90% der Anfangsmenge des topisch wirksamen Kortikosteroids nach einer 3-wöchigen Lagerung umfasst.

32. Zusammensetzung zur Verwendung nach Anspruch 2, umfassend:
a. Budesonid in einer Menge von 0,02 mg bis 0,75 mg pro ml Zusammensetzung,
b. Dinatriumedetat in einer Menge von 0,05 mg bis 25 mg pro ml Zusammensetzung,
c. Natriumcitrat in einer Menge von 0,1 mg bis 30 mg pro ml Zusammensetzung,
d. Polysorbat 80 in einer Menge von 0,05 mg bis 1 mg pro ml Zusammensetzung, und
e. eine wässrige Trägerflüssigkeit.

33. Kit, umfassend einen Mehrfachdosisbehälter und eine Vielzahl von Dosen einer stabilen oralen pharmazeutischen Zusammensetzung zur Verwendung nach Anspruch 1.

34. Kit nach Anspruch 33, wobei das Kit 2 bis 60 Dosen der pharmazeutischen Zusammensetzung umfasst.

35. Kit nach Anspruch 33, wobei das Kit ferner eine Dosiervorrichtung zur Verabreichung der Zusammensetzung an ein Individuum umfasst.

36. Zusammensetzung zur Verwendung nach Anspruch 1, wobei die Zusammensetzung einen zusätzlichen Hilfsstoff umfasst, der ein viskositätsverbesserndes Mittel ist und wobei mindestens 10 Gew.-% der Zusammensetzung mindestens 15 Sekunden nach oraler Verabreichung in der Speiseröhre vorliegt.

37. Zusammensetzung zur Verwendung nach Anspruch 36, wobei die Zusammensetzung als Tablette, Pille, Dragee, Kapsel, weiches Kaudragee, Creme, Paste, Kautablette, Gel, Gelmatrix, Gummi, Brausetablette oder Lutschtablette formuliert ist.

## Revendications

1. Composition pharmaceutique orale physiquement et chimiquement stable pour utilisation dans le traitement, la prévention ou le soulagement d'une inflammation oesophagienne comprenant :
a. une quantité thérapeutiquement active de corticostéroïde,
b. un antioxydant,
c. un tampon,
d. un tensioactif,
e. un conservateur,
f. un arôme, un agent sucrant, ou l'une de leurs combinaisons ;
g. du dextrose, et
h. un véhicule liquide,
où la composition pharmaceutique orale est adaptée à une administration à dose unique ou à doses multiples.

2. Composition pour utilisation selon la revendication 1 comprenant :
a. de l'édétate de disodium en tant qu'antioxydant,
b. de citrate de sodium en tant que tampon,
c. du polysorbate 80 en tant que tensioactif, et
d. de l'eau en tant que véhicule liquide.

3. Composition pour utilisation selon l'une ou l'autre des revendications 1 ou 2, où la composition pharmaceutique est destinée à une utilisation dans le traitement ou la prévention d'une inflammation oesophagienne ou des symptômes qui y sont associés.

4. Composition pour utilisation selon la revendication 3, où l'inflammation oesophagienne est associée à une oesophagite à éosinophiles ou à une maladie de reflux gastro-oesophagien (RGO).

5. Composition pour utilisation selon l'une ou l'autre des revendications 1 ou 2, où la composition reste substantiellement uniforme après un stockage pendant au moins un jour.

6. Composition pour utilisation selon la revendication 1, où la composition est une formule à doses multiples et où la composition est présente dans un récipient à doses multiples comprenant une multitude de doses.

7. Composition pour utilisation selon la revendication 1, où le corticostéroïde est un corticostéroïde à activité topique.

8. Composition pour utilisation selon la revendication 7, où le corticostéroïde à activité topique est l'un des suivants : budésonide, fluticasone, mométasone furoate, ciclésonide, triamcinolone, béclométhasone, ou un ester pharmaceutiquement acceptable de celui-ci, ou l'une de leurs combinaisons.

9. Composition pour utilisation selon la revendication 1, comprenant en outre un ou plusieurs des éléments du groupe constitué par les suivants : maltodextrine, hydroxyéthylcellulose (HEC), hydroxypropylméthyl-cellulose (HPMC), carboxyméthyl-cellulose (CMC), cellulose microcristalline (MCC), carbomère ou leurs combinaisons.

10. Composition pour utilisation selon la revendication 1, où la composition est une suspension de corticostéroïdes et où la composition est substantiellement exempte de particules autres que le corticostéroïde.

11. Composition pour utilisation selon la revendication 1, où la composition est une suspension de particules de corticostéroïdes et où la composition comprend moins de 5 % en masse de particules non dissoutes.

12. Composition pour utilisation selon la revendication 1, où la composition pharmaceutique est un fluide non newtonien.

13. Composition pour utilisation selon la revendication 12, où le fluide non newtonien est choisi dans le groupe constitué par un fluide plastique, pseudoplastique et dilatant.

14. Composition pour utilisation selon la revendication 1, ayant une consistance de gel, de crème, d'onguent, étalable, fluide ou pâteuse.

15. Composition pour utilisation selon la revendication 13, où le fluide non newtonien est pseudoplastique et thixotrope.

16. Composition pour utilisation selon la revendication 1, où le véhicule liquide comprend un milieu aqueux.

17. Composition pour utilisation selon la revendication 16, où la composition pharmaceutique comprend des particules de corticostéroïde suspendues dans le milieu aqueux.

18. Composition pour utilisation selon la revendication 17, où les particules de corticostéroïde sont des microparticules ayant un diamètre moyen de 0,1 micron à 50 microns.

19. Composition pour utilisation selon la revendication 17, où au moins 95 % des particules de corticostéroïde sont des microparticules ayant un diamètre de moins de 10 microns.

20. Composition pour utilisation selon la revendication 16, où le corticostéroïde est présent à une teneur de 0,01 mg à 1 mg par mL.

21. Composition pour utilisation selon la revendication 16, où la composition pharmaceutique présente un volume de 1 mL à 20 mL.

22. Composition pour utilisation selon la revendication 21, où la composition pharmaceutique comprend 0,1 mg à 20 mg de corticostéroïde.

23. Composition pour utilisation selon la revendication 16, où la composition comprend un excipient supplémentaire qui comprend de la CMC, et où la CMC est présente à une teneur de 1 mg à 30 mg par mL de composition.

24. Composition pour utilisation selon la revendication 16, où la composition comprend un excipient supplémentaire qui comprend de la MCC, et où la MCC est présente à une teneur de 5 mg à 30 mg par mL de composition.

25. Composition pour utilisation selon la revendication 16, où la composition comprend un excipient supplémentaire qui comprend une combinaison de CMC et de MCC, où la combinaison CMC-MCC est présente à une teneur de 10 mg par mL à 40 mg par mL de composition, et où le rapport massique mixte CMC/MCC est de 11/89.

26. Composition pour utilisation selon la revendication 1, où la composition comprend un excipient supplémentaire qui comprend de la maltodextrine, et où la maltodextrine est présente à une teneur supérieure à 8 % en masse.

27. Composition pour utilisation selon la revendication 26, où la maltodextrine présente un équivalent dextrose (ED) de 13 à 18.

28. Composition pour utilisation selon la revendication 26, où la maltodextrine présente un équivalent dextrose (ED) supérieur à 5.

29. Composition pour utilisation selon la revendication 16, où l'antioxydant est l'édétate de disodium et l'édétate est présent à une teneur de 0,05 mg à 25 mg par mL de composition.

30. Composition pour utilisation selon la revendication 1, ayant une viscosité d'au moins 100 mPa.s mesurée entre 20 et 25 degrés Celsius à une vitesse de cisaillement de 15 s⁻¹.

31. Composition pour utilisation selon la revendication 1, où la composition pharmaceutique stable est chimiquement stable, comprenant au moins 90 % de la quantité initiale de corticostéroïde à activité topique après avoir été stockée pendant 3 semaines.

32. Composition pour utilisation selon la revendication 2 comprenant :
a. du budésonide à une teneur de 0,02 mg à 0,75 mg par mL de composition,
b. de l'édétate de disodium à une teneur de 0,05 mg à 25 mg par mL de composition,
c. du citrate de sodium à une teneur de 0,1 mg à 30 mg par mL de composition,
d. du polysorbate 80 à une teneur de 0,05 mg à 1 mg par mL de composition, et
e. un véhicule liquide aqueux.

33. Kit comprenant un récipient à unités multiples et une multitude de doses d'une composition pharmaceutique orale stable pour utilisation selon la revendication 1.

34. Kit selon la revendication 33, où le kit comprend 2 à 60 doses de la composition pharmaceutique.

35. Kit selon la revendication 33, où le kit comprend en outre un dispositif de mesure pour l'administration de la composition à un individu.

36. Composition pour utilisation selon la revendication 1, où la composition comprend un excipient supplémentaire qui est un agent améliorant la viscosité et où au moins 10 % en masse de la composition sont présents dans l'oesophage au moins 15 secondes après administration orale.

37. Composition pour utilisation selon la revendication 36, où la composition est formulée sous forme de comprimé, de pilule, de dragée, de capsule, de pâte à mâcher, de crème, de pâte, de comprimé à mâcher, de gel, de matrice de gel, de gommes, de comprimé effervescent ou de tablette.
